(19) Europäisches Patentamt
European Patent Office
Office européen des brevets

(11) EP 2 556 078 B1

(12) **EUROPÄISCHE PATENTSCHRIFT**

(45) Veröffentlichungstag und Bekanntmachung des Hinweises auf die Patenterteilung:
**24.09.2014 Patentblatt 2014/39**

(21) Anmeldenummer: **11722991.4**

(22) Anmeldetag: **31.03.2011**

(51) Int Cl.:
***C07F 11/00*** *(2006.01)* ***C08F 4/72*** *(2006.01)*
***B01J 31/22*** *(2006.01)* ***C07C 6/02*** *(2006.01)*

(86) Internationale Anmeldenummer:
**PCT/DE2011/000348**

(87) Internationale Veröffentlichungsnummer:
**WO 2011/120508 (06.10.2011 Gazette 2011/40)**

# (54) KATALYSATOREN FÜR DIE ALKINMETATHESE

CATALYSTS FOR ALKYNE METATHESIS

CATALYSEUR POUR METATHESE D'ALKYNE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **10.06.2010 DE 102010023326**
**03.04.2010 DE 102010013813**

(43) Veröffentlichungstag der Anmeldung:
**13.02.2013 Patentblatt 2013/07**

(73) Patentinhaber: **Studiengesellschaft Kohle MbH**
**45470 Mülheim an der Ruhr (DE)**

(72) Erfinder:
- **FÜRSTNER, Alois**
  **45478 Mülheim an der Ruhr (DE)**
- **HEPPEKAUSEN, Johannes**
  **45470 Mülheim an der Ruhr (DE)**
- **HICKMANN, Volker**
  **67125 Dannstadt (DE)**
- **STADE, Robert**
  **45470 Mülheim an der Ruhr (DE)**

(74) Vertreter: **Ricker, Mathias**
**Wallinger Ricker Schlotter Tostmann**
**Patent- und Rechtsanwälte**
**Zweibrückenstrasse 5-7**
**80331 München (DE)**

(56) Entgegenhaltungen:
- **TSA Y-C ET AL: "FACILE SYNTHESIS OF TRIALKOXYMOLYBDENUM(VI) ALKYLIDYNE COMPLEXES FOR ALKYNE METATHESIS", ORGANOMETALLICS, ACS, WASHINGTON, DC, US, Bd. 19, Nr. 25, 11. Dezember 2000 (2000-12-11), Seiten 5260-5262, XP000976163, ISSN: 0276-7333, DOI: DOI:10.1021/OM000644F**
- **JOHANNES HEPPEKAUSEN ET AL: "Practical New Silyloxy-Based Alkyne Metathesis Catalysts with Optimized Activity and Selectivity Profiles", JOURNAL OF THE AMERICAN CHEMICAL SOCIETY, Bd. 132, Nr. 32, 18. August 2010 (2010-08-18), Seiten 11045-11057, XP55002082, ISSN: 0002-7863, DOI: 10.1021/ja104800w**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 556 078 B1

**Beschreibung**

Die vorliegende Erfindung betrifft metallorganische W- und Mo-Komplexe, ein Verfahren zur ihrer Herstellung und ihre Verwendung als Präkatalysatoren und Katalysatoren für die Alkinmetathese.

Unter Alkinmetathese versteht man die wechselseitige Umalkylidinierung von Alkinen gemäß Schema 1. Alkinmetathesen können sowohl intermolekular als auch intramolekular durchgeführt werden (Fürstner, A.; Davies, P. W. Chem. Commun. 2005, 2307; Zhang, W.; Moore, J. S. Adv. Synth. Catal. 2007, 349, 93; Schrock, R. R.; Czekelius, C. Adv. Synth. Catal. 2007, 349, 55).

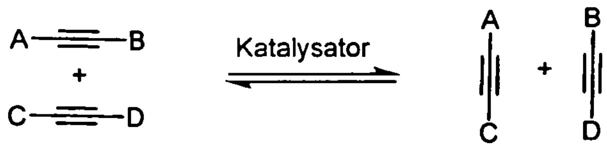


Schema 1. Prinzip der Alkinmetathese

Alkinmetathese-Reaktionen werden von Metallverbindungen katalysiert, die im Reaktionsgemisch in homogener oder in heterogener Form vorliegen können. Am häufigsten wurden in der Literatur Metallverbindungen des Wolfram, Molybdän und Rhenium verwendet. Als eigentlich katalytisch aktive Spezies gelten sogenannte Schrock-Alkylidin Komplexe (Schrock, R. R. Chem. Rev. 2002, 102, 145; Schrock, R. R. Angew. Chem., Int. Ed. 2006, 45, 3748), die in situ oder ex situ dargestellt werden können. Die bisher eingesetzten Schrock-Alkylidin Komplexe reagieren jedoch in der Regel sowohl als Feststoffe als auch in Lösung rasch mit Sauerstoff und/oder Feuchtigkeit unter Bildung katalytisch inaktiver Spezies, und müssen daher unter rigorosem Luft- und Feuchtigkeitsausschluss gehandhabt werden. Repräsentative Beispiele finden sich in Schema 2.

Im Gegensatz dazu lassen sich Alkylidin-Komplexe ("Fischer-Carbine", siehe: Fischer, E. O. Adv. Organomet. Chem. 1976, 14, 1), die am Metallzentrum einen oder mehrere Kohlenmonoxid Liganden (CO) aufweisen, durch Sauerstoff-, Stickstoff- oder Phosphorhaltige Liganden stabilisieren. Die so erhaltenen Fischer-Carbine sind zum Teil sogar luftstabil (Mayr, A. et al. Organometallics 1985, 4, 608; Hazra, D. et al., J. Organomet. Chem. 2003, 671, 52), zeigen aber keine katalytische Aktivität in der Alkinmetathese.

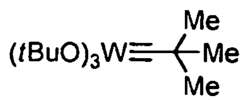

1

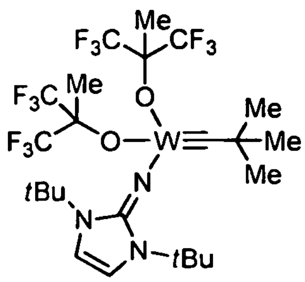

2

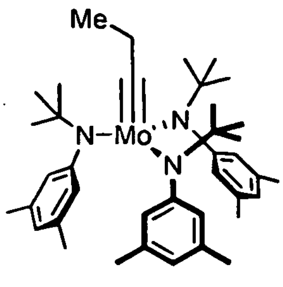

3

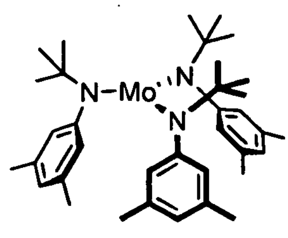

4

Schema 2. Repräsentative Beispiele für Präkatalysatoren und Schrock-Alkylidinkomplexe für die Alkinmetathese.

Obwohl sich katalytisch aktive Schrock-Alkylidinkomplexe im Reaktionsgemisch auch in situ aus geeigneten Präkatalysatoren erzeugen lassen, müssen auch im Fall dieser indirekten Methoden meist ähnlich rigorose Bedingungen bezüglich des Ausschlusses von Luft und Feuchtigkeit angewendet werden. Dies gilt etwa für die Verwendung von Molybdäntrisamido Komplexe vom Typ **4,** aus denen durch Reaktion mit Halogenverbindungen in situ katalytisch aktive Molybdänalkylidine gebildet werden (Fürstner, A. et al. J. Am. Chem. Soc. 1999, 121, 9453; Zhang, W. et al. J. Am. Chem. Soc. 2004, 126, 329). Der Präkatalysator **4** reagiert nicht nur mit Luft und Wasser, sondern aktiviert unter anderem auch molekularen Stickstoff ($N_2$), sodass selbst die üblichen Arbeitstechniken unter Verwendung von Stickstoff als Schutzgas nicht angewendet werden können, sondern auf teures Argon als Schutzgas zurückgegriffen werden muss. Die Verwendung von **4** als Präkatalysator hat jedoch zahlreiche Anwendungen der Alkinmetathese auf hochfunktionalisierte Substrate und Naturstoffe ermöglicht, für die andere Alkinmetathesekatalysatoren nicht geeignet waren (Fürstner,

A. et al. Chem. Eur. J. 2001, 7, 5299; Fürstner, A. et al. W. Chem. Commun. 2005, 2307).

Benutzerfreundlichere Rezepturen zur in situ Darstellung von Katalysatoren für die Alkinmetathese benutzen Mischungen von Molybdän- oder Wolframverbindungen in Kombination mit diversen Additiva. Typische Methoden verwenden unter anderem Kombinationen von $Mo(CO)_6$/Phenol oder substituierten Phenolen, sowie $MoO_2(acac)_2/Et_3Al$, $MoO_3/SiO_2$, $WO_3/SiO_2$ (Übersicht: Mortreux, A. et al. J. Mol. Catal. 2006, 254, 96). Diese Verfahren tolerieren jedoch meist nur wenige funktionelle Gruppen. Auch müssen die Alkinmetathesen in der Regel bei hohen Temperaturen durchgeführt werden, was Anwendungen auf hochfunktionalisierte und thermisch labile Substrate ausschließt.

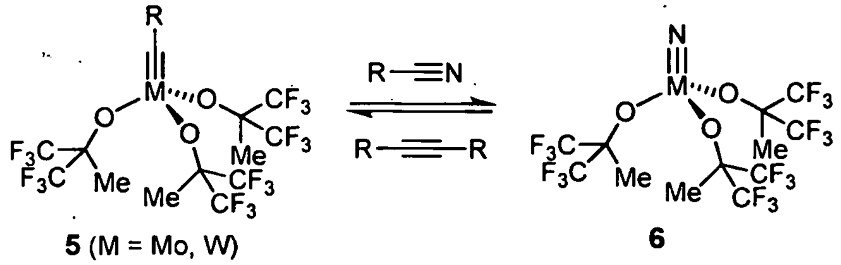

**5** (M = Mo, W) **6**

Schema 3. Darstellung von Metall-Alkylidinkomplexen aus Metallnitrid Komplexen; R = Alkyl, Aryl.

Alternativ können katalytisch aktive Schrock-Alkylidinkomplexe in situ durch Umsetzung von Metallnitrid Komplexen als Präkatalysatoren mit Alkinen dargestellt werden (Schema 3). Dies erfordert jedoch spezielle Alkoxid Liganden in der Koordinationssphäre des Präkatalysators, wobei sich hoch fluorierte oder perfluorierte, meist verzweigte Alkoxide (z. B. Perfluoro-tert-butanolat, Hexafluoro-tert-butanolat) als besonders geeignet erwiesen haben (Geyer, A. M. et al. J. Am. Chem. Soc. 2008, 130, 8984; Gdula, R. L. et al. J. Am. Chem. Soc. 2006, 128, 9614).

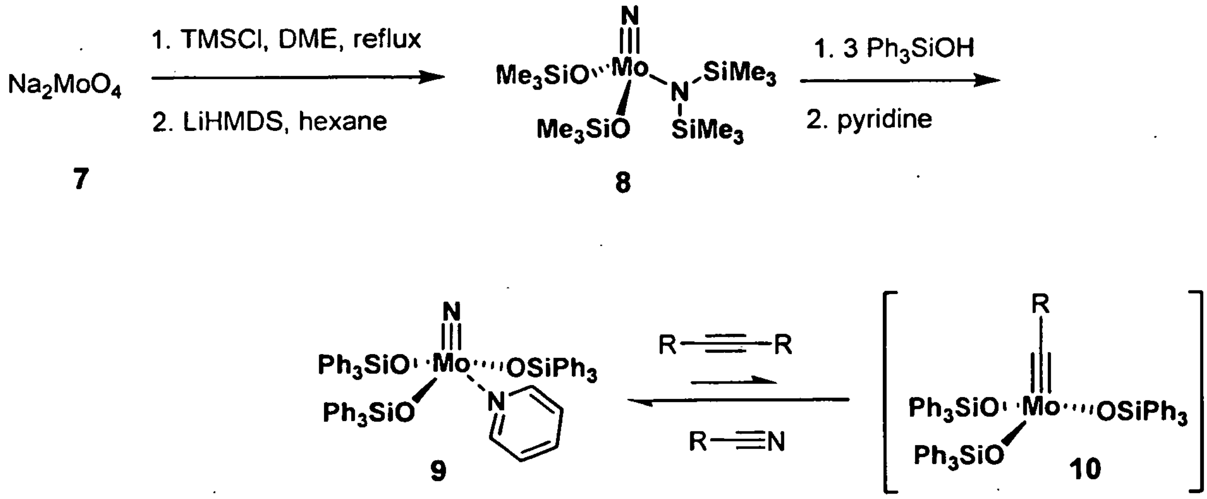


**7** **8** **9** **10**

Schema 4. Verwendung von Metallnitrid Komplexen mit Silanolat Liganden als Präkatalysatoren; R = Alkyl, Aryl.

Bekannt ist ferner, dass auch Metallnitrid Komplexe des Wolfram oder Molybdäns mit Silanolat Liganden geeignet sind, um aktive Katalysatoren für die Alkinmetathese herzustellen; dabei wird die in situ Bildung von Alkylidinkomplexen des Typs **10** postuliert, ohne dass dieser Aspekt durch Isolierung oder Charakterisierung der aktiven Spezies eindeutig geklärt werden konnte (Fürstner A. et al., J. Am. Chem. Soc. 2009, 131, 9468; Heilmann, E., Dissertation, Technische Universität Dortmund, 2008; Bindl, M., Technische Universität Dortmund, 2009). Wird ein solcher Präkatalysator mit einem zusätzlichen Pyridinliganden stabilisiert, wie am Beispiel von Verbindung **9** gezeigt (Schema 4), so kann dieser für kurze Zeit an der Luft gehandhabt werden. Eine Aufbewahrung unter Luft führt jedoch in weniger als 30 Minuten zur Zersetzung und zum vollständigen Verlust der katalytischen Aktivität.

Überraschenderweise hat sich herausgestellt, dass Nitridokomplexe des Molbydäns und Wolframs, die anstelle eines einfachen Pyridin-Liganden einen verbrückten Liganden, wie zum Beispiel 2,2'-Bipyridin, 1,10-Phenanthrolin oder

deren Derivate tragen, im Gegensatz zu Verbindung **9** über Monate unzersetzt an der Luft gelagert werden können, ohne dass Maßnahmen zum Ausschluss von Feuchtigkeit getroffen werden müssen. Obwohl solche Komplexe selbst keine oder nur geringe katalytische Aktivität in der Alkinmetathese von 1-Phenyl-1-propin aufweisen unter Bedingungen, bei denen etwa der Pyridinkomplex 9 zu hohen Ausbeuten an Tolan führt (Toluol, 80°C), fanden wir jedoch, dass sich aus Nitridakomplexen des Molbydans und Wolframs mit stabilisierendem 2,2'-Bipyridin- oder 1,10-Phenanthrolin-Liganden durch Zugabe geeigneter Metallsalze als Additiva aktive Katalysatoren für die Alkinmetathese erzeugen lassen. Ebenso lassen sich durch 2,2'-Bipyridin- oder 1,10-Phenanthrolin-Liganden auch Schrock-Alkylidinkomplexe stabilisieren. Diese Addukte weisen ebenfalls keine oder nur geringe Aktivität als Katalysatoren für die Alkinmetathese auf. Allerdings konnte gezeigt werden, dass auch aus diesen Addukten durch Zugabe geeigneter Metallsalze als Additiva aktive Katalysatoren für die Alkinmetathese freigesetzt werden können.

Gegenstand der vorliegenden Erfindung sind demnach metallorganische Verbindungen der allgemeinen Formel **I**,

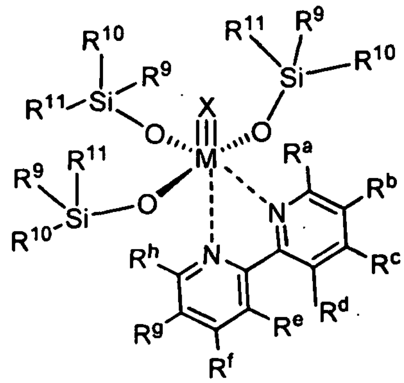

**(I)**

worin

M = Mo, W,
die Substituenten $R^a$, $R^b$, $R^e$, $R^d$, $R^e$, $R^f$, $R^g$, $R^h$ gleich oder verschieden sein können und unabhängig voneinander ausgewählt werden können aus: H, $C_1$-$C_{12}$-Alkyl, $C_3$-$C_{12}$-Cycloalkyl, 6- bis 18-gliedriges Aryl, 5- bis 10-gliedriges Heteroaryl, $C_1$-$C_{12}$-Alkyloxy, $C_3$-$C_{12}$-Cycloalkyloxy, $C_6$-$C_{20}$-Aryloxy, Di-$C_1$-$C_4$-alkylamino. Halogen, Nitro, Cyano, Trifluormethyl, - $COOR^{12}$, worin $R^{12}$ = $C_1$-$C_{12}$ Alkyl, $C_3$-$C_{12}$ Cycloalkyl, 6- bis 18-gliedriges Aryl, 5- bis 10-gliedriges Heteroaryl sein kann,
und die Substituenten $R^a$ und $R^b$ oder $R^b$ und $R^c$ oder $R^c$ und $R^d$ sowie $R^h$ und $R^g$ oder $R^g$ und $R^f$ oder $R^f$ und $R^e$ auch unter Bildung eines gesättigten, ungesättigten oder aromatischen Ringes miteinander verbunden sein können, und/oder die Reste $R^d$ und $R^e$ unter Bildung eines 5- bis 8-gliedrigen gesättigten, ungesättigten oder aromatischen Ringes miteinander verbunden sein können,
die Substituenten $R^9$, $R^{10}$, $R^{11}$ gleich oder verschieden sein können und unabhängig voneinander ausgewählt werden können aus folgenden Gruppen: $C_1$-$C_{12}$-Alkyl, $C_3$-$C_{12}$-Cycloalkyl, 6- bis 18-gliedriges Aryl, 5- bis 10-gliedriges Heteroaryl, mit der Maßgabe, dass mindestens einer dieser Substituenten ein 6- bis 18-gliedriger Aryl- oder 5- bis 10-gliedriger Heteroaryl Rest ist, worin dieser Aryl- oder Heteroaryl Rest am Silicium seinerseits bis zu fünf identische oder verschiedene Substituenten aufweisen kann, aus der Liste: H, $C_1$-$C_{12}$-Alkyl, $C_1$-$C_{12}$-Alkoxy, Di-$C_1$-$C_4$-Alkylamino, $C_3$-$C_{12}$-Cycloalkyl, $C_3$-$C_{12}$ Cycloalkyloxy, 6- bis 18-gliedriges Aryl, 5- bis 10-gliedriges Heteroaryl, Halogen, und
X = N, $CR^{13}$, worin $R^{13}$ = $C_1$-$C_{12}$-Alkyl, $C_3$-$C_{12}$-Cycloalkyl, 6- bis 18-gliedriges Aryl, 5- bis 10-gliedriges Heteroaryl sein kann.

Die Reste $R^d$ und $R^e$ können auch unter Bildung eines 5 bis 8 gliedrigen gesättigten, ungesättigten oder aromatischen Ringes miteinander verbunden sein und somit eine Brücke bilden, die $CR^{41}R^{42}$, $CR^{43}=CR^{44}$, $CR^{45}R^{46}-CR^{47}R^{48}$, $CR^{49}R^{50}-CR^{51}R^{52}-CR^{53}R^{54}$, $CR^{55}R^{56}=C^{57}R^{58}-CR^{59}R^{60}$ $CR^{61}R^{62}-CR^{63}R^{65}-CR^{65}R^{66}-CR^{67}CR^{68}$, $CR^{69}=CR^{70}-CR^{71}R^{72}-CR^{73}CR^{74}$ oder $CR^{75}R^{76}-CR^{77}=CR^{78}-CR^{79}CR^{80}$ sein kann, in welchen $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$, $R^{75}$, $R^{76}$, $R^{77}$, $R^{78}$, $R^{79}$ und $R^{80}$ jeweils unabhängig voneinander ausgewählt werden können und die gleiche Bedeutung haben können wie $R^a$.

Stehen die Reste $R^d$ und $R^e$ für eine nicht-verbrückende Gruppe, handelt es sich bei den Liganden um ein 2,2'-Bipyridin Derivat (2,2'-Dipyridyl Derivat).

Bevorzugte Verbindungen der Formel I werden durch die allgemeinen Formel **11** wiedergegeben

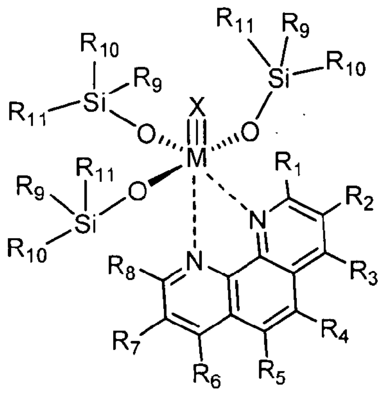

**11**

worin

die Substituenten $R^1$, $R^2$, $R^3$, $R^4$, $R^5$, $R^6$, $R^7$, $R^8$ gleich oder verschieden sein können und unabhängig voneinander ausgewählt werden können aus: H, $C_1$-$C_{12}$-Alkyl, $C_3$-$C_{12}$-Cycloalkyl, 6- bis 18-gliedriges Aryl, 5- bis 10-gliedriges Heteroaryl, $C_1$-$C_{12}$-Alkyloxy, $C_3$-$C_{12}$-Cycloalkyloxy, $C_6$-$C_{20}$-Aryloxy, Di-$C_1$-$C_4$-alkylamino, Halogen, Nitro, M, X, $R^9$, $R^{10}$ und $R^{11}$ wie oben definiert sind.

Weitere bevorzugte Verbindungen sind solche, die durch die allgemeine Formel **11a** dargestellt sind

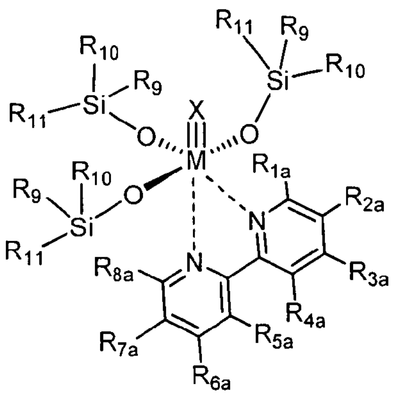

**11a**

worin

$R^{1a}$, $R^{2a}$, $R^{3a}$, $R^{4a}$, $R^{5a}$, $R^{6a}$, $R^{7a}$, $R^{8a}$ die gleiche Bedeutung haben wie für $R^1$, $R^2$, $R^3$, $R^4$, $R^5$, $R^6$, $R^7$, $R^8$ in der Formel **11** definiert ist, und
M, X, $R^9$, $R^{10}$ und $R^{11}$ wie oben definiert sind.

Im Rahmen der vorliegenden Erfindung bedeuten:

$C_1$-$C_{12}$-Alkyl einen geradkettigen oder verzweigten Alkylrest mit 1 bis 12 Kohlenstoffatomen;

$C_1$-$C_{12}$-Alkoxy einen geradkettigen oder verzweigten Alkoxyrest mit 1 bis 12 Kohlenstoffatomen;

Di-$C_1$-$C_4$-Alkylamino eine Amino-Gruppe mit zwei gleichen oder verschiedenen geradkettigen oder verzweigten Alkylsubstituenten, die jeweils 1 bis 4 Kohlenstoffatome aufweisen;

$C_3$-$C_{12}$-Cycloalkyl eine monocyclische, gesättigte Cycloalkylgruppe mit 3 bis 12 Kohlenstoffatomen;

$C_3$-$C_{12}$ Cycloalkyloxy eine monocyclische, gesättigte Cycloalkyloxygruppe mit 3 bis 12 Kohlenstoffatomen;

6- bis 18-gliedriges Aryl einen mono-, bi- oder tricyclischen carbocyclischen Aromaten, der seinerseits 0 bis 5 Substituenten tragen kann aus der Liste. $C_1$-$C_{12}$-Alkyl, $C_1$-$C_{12}$-Alkoxy, Di-$C_1$-$C_4$-Alkylamino, $C_3$-$C_{12}$-Cycloalkyl, $C_3$-$C_{12}$ Cycloalkyloxy, 6- bis 18-gliedriges Aryl, 5- bis 10-gliedriges Heteroaryl, Halogen, Cyano, Nitro;

5- bis 10-gliedriges Heteroaryl einen mono- oder gegebenenfalls bicyclischen aromatischen Heterocyclus (Heteroaromaten) mit insgesamt 5 bis 10 Ringatomen, der bis zu drei Ring-Heteroatome aus der Reihe N, O und/oder S enthält und über ein Ring-Kohlenstoffatom oder gegebenenfalls über ein Ring-Stickstoffatom verknüpft ist. Beispielhaft seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl, Triazinyl, Benzofuranyl, Benzothienyl, Benzimidazolyl, Benzoxazolyl, Benzothiazolyl, Benzotriazolyl, Indolyl, Indazolyl, Chinolinyl, Isochinolinyl, Naphthyridinyl, Chinazolinyl, Chinoxalinyl, Phthalazinyl, Pyrazolo[3,4-b]pyridinyl.

Halogen bedeutet im Rahmen der Erfindung Fluor, Chlor, Brom und Iod.

Bevorzugte Alkylreste im Rahmen dieser Erfindung sind geradkettige oder verzweigte Alkylreste mit 1 bis 6 Kohlenstoffatomen. Beispielhaft und vorzugsweise seien genannt: Methyl, Ethyl, n-Propyl, Isopropyl, n-Butyl, *iso*-Butyl, *sec.*-Butyl, *tert.*-Butyl, 1-Ethylpropyl, n-Pentyl und n-Hexyl.

Bevorzugte Alkoxyreste im Rahmen dieser Erfindung sind geradkettige oder verzweigte Alkoxyreste mit 1 bis 6 Kohlenstoffatomen. Beispielhaft und vorzugsweise seien genannt: Methoxy, Ethoxy, n-Propoxy, Isopropoxy, n-Butoxy, *tert.*-Butoxy, n-Pentoxy und n-Hexoxy.

Bevorzugte Cycloalkylreste im Rahmen dieser Erfindung sind Cycloalkylreste mit 3 bis 7 Kohlenstoffatomen. Beispielhaft und vorzugsweise seien genannt: Cyclopropyl, Cyclobutyl, Cyclopentyl, Cyclohexyl und Cycloheptyl.

Bevorzugte Cycloalkyloxyreste im Rahmen dieser Erfindung sind Cycloalkyloxyreste mit 3 bis 7 Kohlenstoffatomen. Beispielhaft und vorzugsweise seien genannt: Cyclopropyloxy, Cyclobutyloxy, Cyclopentyloxy, Cyclohexyloxy und Cycloheptyloxy.

Bevorzugte Di-$C_1$-$C_4$-Alkylamino Reste im Rahmen dieser Erfindung sind folgende Di-$C_1$-$C_4$-Alkylamino Reste: *N,N*-Dimethylamino, *N,N*-Diethylamino, *N*-Ethyl-*N*-methylamino, *N*-Methyl-N-n-propylamino, *N*-Isopropyl-N-methylamino, *N*-Isopropyl-*N*-ethylamino, *N*-Isopropyl-*N*-n-propylamino, *N,N*-Diisopropylamino, *N*-n-Butyl-*N*-methylamino und *N*-*tert.*-Butyl-*N*-methylamino.

Bevorzugte Arylreste im Rahmen dieser Erfindung sind: Phenyl, Naphthyl, Anthryl, Methylphenyl, Dimethylphenyl, Trimethylphenyl, Methoxyphenyl, Dimethoxyphenyl, Trimethoxyphenyl, Fluorphenyl, Chlorphenyl, Bromphenyl, Iodphenyl, Pentafluorphenyl, Trifluormethylphenyl, Dimethylaminophenyl, $C_1$-$C_{12}$ Alkoxycarbonylphenyl.

Bevorzugte Heteroaryl Reste im Rahmen dieser Erfindung sind mono- oder gegebenenfalls bicyclische 5- bis 10-gliedrige Heteroaryl-Reste mit bis zu zwei Heteroatomen aus der Reihe N, O und/oder S. Besonders bevorzugt sind monocyclische 5- oder 6-gliedrige Heteroaryl-Reste mit bis zu zwei Heteroatomen aus der Reihe N, O und/oder S wie beispielsweise Furyl, Thienyl, Thiazolyl, Oxazolyl, Isothiazolyl, Isoxazolyl, Pyrazolyl, Imidäzolyl, Pyridyl, Pyrimidinyl, Pyridazinyl, Pyrazinyl.

Beispiele für bevorzugte Silanolat-Liganden des allgemeinen Typs $R^9R^{10}R^{11}SiO$- sind im Rahmen dieser Erfindung: Triphenylsilanolat, Phenyldimethylsilanolat, Phenyldi(tert-butyl)silanolat, Diphenylmethylsilanolat, Diphenyl(tert-butyl)silanolat, Tris(methoxyphenyl)silanolat, Tris(dimethoxyphenyl)silanolat, Tris(dimethylphenyl)silanolat, Tris(trimethylphenyl)silanolat, Tris(dimethylaminophenyl)silanolat, Tris(fluorphenyl)silanolat, Tris(difluorphenyl)silanolat, Tris(pentafluorphenyl)silanolat, Tris(3,5-di-tertbutyl-6-methoxyphenyl)silanolat, Tris(1-naphthyl)silanolat, Tris(2-furyl)silanolat, Tris(2-thienyl)silanolat.

Bevorzugte Verbindungen im Rahmen der vorliegenden Erfindung sind Komplexe der allgemeinen Formel **12:**

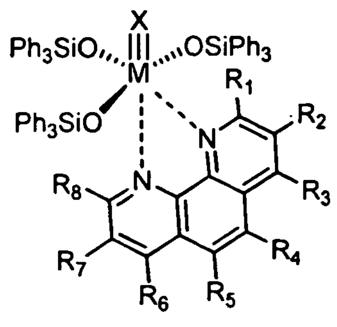

**12**

worin

M = Mo, W

X = N, $CR^{13}$, wobei

$R^{13}$ Methyl, Ethyl, Propyl, Butyl, iso-Propyl, tert-Butyl, Trimethylsilylmethyl, Benzyl, Furyl, Phenyl sein kann, wobei Phenyl seinerseits bis zu fünf identische oder verschiedene Reste aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl tragen kann,

$R^1$, $R^2$, $R^3$, $R^4$, $R^5$, $R^6$, $R^7$, $R^8$ frei und unabhängig voneinander gewählt werden können aus: H, $C_1$-$C_4$ Alkyl, $C_3$-$C_6$ Cycloalkyl, Phenyl, Naphthyl, Methylphenyl, Dimethylphenyl, Trimethylphenyl, Methoxyphenyl, Dimethoxyphenyl, Fluorphenyl, Pentafluorphenyl, Trifluormethylphenyl, Dimethylaminophenyl, Furyl, Halogen, Nitro, Cyano, Trifluormethyl, - $COOR^{12}$, mit

$R^{12}$ = $C_1$-$C_{12}$ Alkyl, $C_3$-$C_{12}$ Cycloalkyl, 6- bis 18-gliedriges Aryl, 5- bis 10-gliedriges Heteroaryl; bevorzugt sind Methyl, Ethyl, Propyl, iso-Propyl, Butyl, tert-Butyl, Pentyl, Hexyl, Heptyl, Octyl, Benzyl, Phenyl.

Beispiele für bevorzugte 1,10-Phenanthroline sind: das unsubstituierte 1,10-Phenanthrolin, 4-Methyl-1,10-phenanthrolin, 5-Methyl-1,10-phenanthrolin, 2,9-Dimethyl[1,10]phenanthrolin, 5,6-Dimethyl-1,10-phenanthrolin, 5-Chlor[1,10]phenanthrolin, 4,7-Dichloro-1,10-phenanthrolin, 4,7-Dichlor-1,10-phenanthrolin, 3,4,7,8-Tetramethyl-1,10-phenanthrolin, 4,7-Diphenyl[1,10]phenanthrolin, 2,9-Dimethyl-4,7-diphenyl[1,10]phenanthrolin, 5-Nitro-1,10-phenanthrolin, 4,7-Dimethoxy-1,10-phenanthrolin.

Ebenfalls bevorzugte Verbindungen im Rahmen der vorliegenden Erfindung sind Komplexe der allgemeinen Formel **12a:**

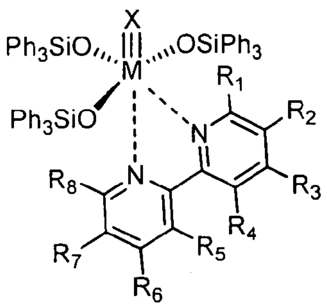

**12a**

worin

M = Mo, W

X = N, $CR^{13}$, wobei

$R^{13}$ Methyl, Ethyl, Propyl, Butyl, iso-Propyl, tert-Butyl, Trimethylsilylmethyl, Benzyl, Furyl, Phenyl sein kann, wobei Phenyl seinerseits bis zu fünf identische oder verschiedene Reste aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl tragen kann,

$R_1$, $R_2$, $R_3$, $R_4$, $R_5$, $R_6$, $R_7$, $R_8$ frei und unabhängig voneinander gewählt werden können aus: H, $C_1$-$C_4$. Alkyl, $C_3$-$C_6$ Cycloalkyl, Phenyl, Naphthyl, Methylphenyl, Dimethylphenyl, Trimethylphenyl, Methoxyphenyl, Dimethoxyphenyl, Fluorphenyl, Pentafluorphenyl, Trifluormethylphenyl, Dimethylaminophenyl, Furyl, Halogen, Nitro, -$COOR^{12}$, mit

$R^{12}$ = $C_1$-$C_{12}$ Alkyl, $C_3$-$C_{12}$ Cycloalkyl, 6- bis 18-gliedriges Aryl, 5- bis 10-gliedriges Heteroaryl; bevorzugt sind Methyl, Ethyl, Propyl, iso-Propyl, Butyl, tert-Butyl, Pentyl, Hexyl, Heptyl, Octyl, Benzyl, Phenyl,

Beispiele für bevorzugte 2,2'-Bipyridine (2,2'-Dipyridyle) sind: 2,2'-Bipyridin, 5,5'-Dimethyl-2,2'-dipyridyl, 4,4'-Dimethyl-2,2'-dipyridyl, 6,6'-Dimethyl-2,2'-dipyridyl, 4-4'-Dimethoxy-2-2'-bipyridin, 2,2'-Bichinolin, 4,4'-Di-tert-butyl-2,2'-dipyridyl, 2,2'-Bipyridinyl-4,4'-dicarbonsäuredimethylester, 4,4'-Diphenyl-2,2'-dipyridyl, 6,6'-Dibromo-2,2'-dipyridyl, 4,4'-Dinonyl-2,2'-dipyridyl, 2,2'-Bichinolinyl-4,4'-dicarbonsäuredibutylester, 2,2'-Bichinolinyl-4,4'-dicarbonsäurediheptylester, 6-Methyl-2,2'-dipyridyl, 2-(2-Pyridinyl)chinolin, 2-Pyridin-2-yl-4-pyrrolidin-1-yl-chinolin, 4-Piperidin-1-yl-2-pyridin-2-yl-chinolin, 4-Morpholin-4-yl-2-pyridin-2-yl-chinolin.

Besonders bevorzugte Verbindungen im Rahmen der vorliegenden Erfindung sind Komplexe der Formeln **13a, 13b, 14a, 14b, 15a, 15b, 16a, 16b, 13e:**

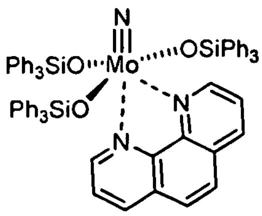
**13a**

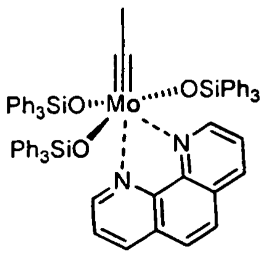
**14a**

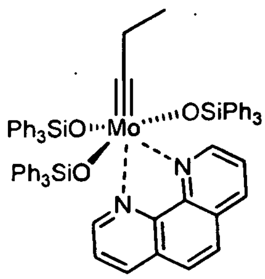
**15a**

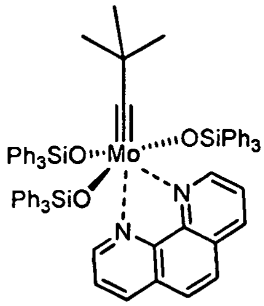
**16a**

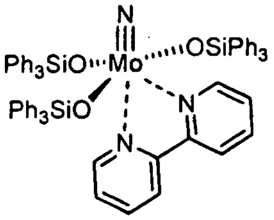
**13b**

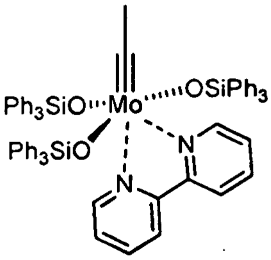
**14b**

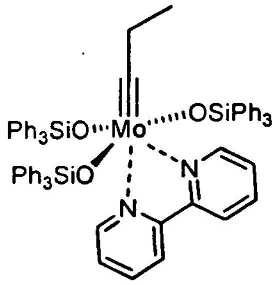
**15b**

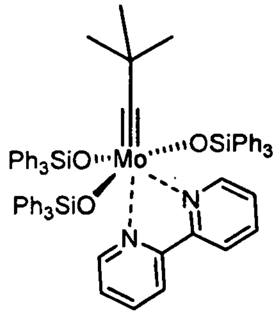
**16b**

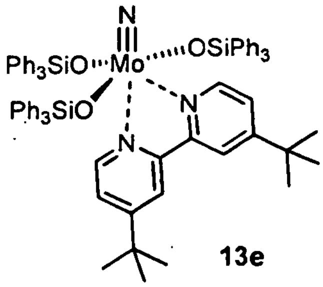
**13e**

Ebenfalls besonders bevorzugt sind Verbindungen der allgemeinen Formeln 17a und 17b,

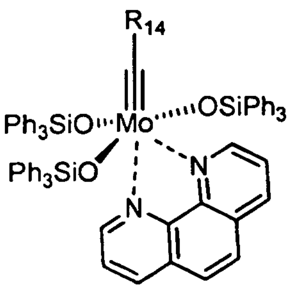
**17a**

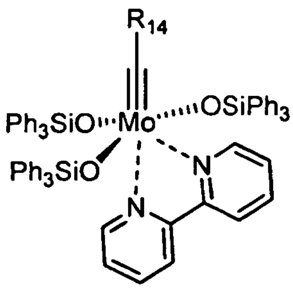
**17b**

wobei

$R^{14}$ ein Phenylring ist, der 1 bis 5 gleiche oder verschiedene Substituenten trägt, ausgewählt aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl.

Beispiele für besonders bevorzugte Verbindungen sind die im Abschnitt "Beispiele" näher beschriebenen Verbindungen **24a, 24b, 24c, 24d, 24e, 34a**:

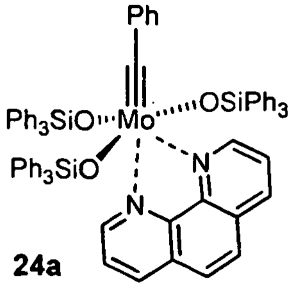
24a

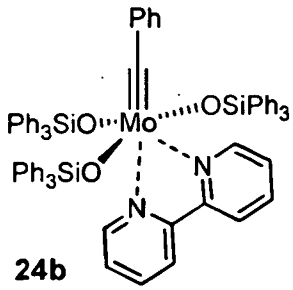
24b

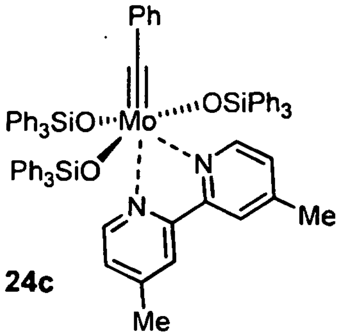
24c

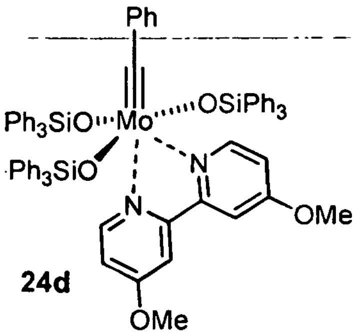
24d

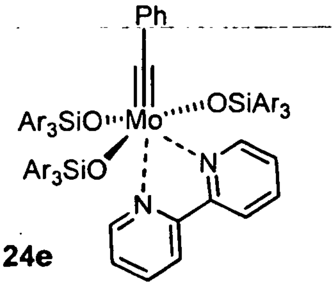
24e

Ar = 4-Methoxyphenyl

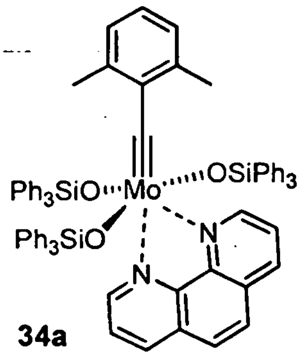
34a

Ein weiterer Gegenstand der vorliegenden Erfindung ist ein Verfahren zur Herstellung der Verbindungen der allgemeinen Formel I

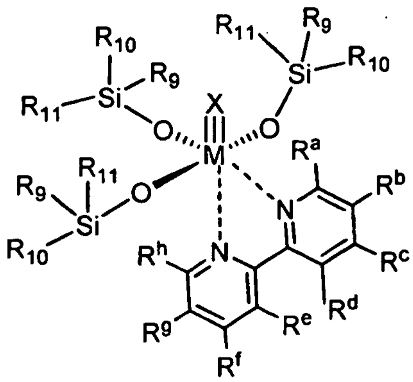
**(I)**

worin

M, $R^a$, $R^b$, $R^c$, $R^d$, $R^e$, $R^f$, $R^g$, $R^h$, $R^9$, $R^{10}$, $R^{11}$ und X wie oben definiert sind,

in welchem eine Verbindung der allgemeinen Formel **18**

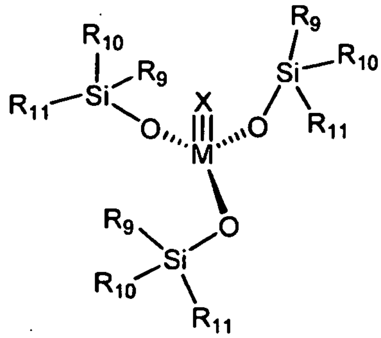

**18**

worin
M, $R_9$, $R_{10}$, $R_{11}$ und X wie oben definiert sind,
mit einer Verbindung der allgemeinen Formel **19**

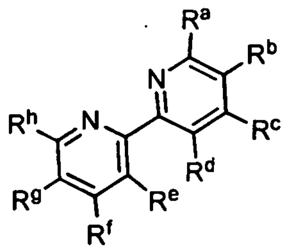

**19**

worin
$R^a$, $R^b$, $R^c$, $R^d$, $R^e$, $R^f$, $R^g$ und $R^h$ wie oben definiert sind umgesetzt wird.

Die Umsetzung der Verbindungen der Formel **18** mit Verbindungen mit der Formel **19** erfolgt unter Schutzgas Atmosphäre (zum Beispiel: Stickstoff, Argon) in einem inerten Lösungsmittel bei Temperaturen zwischen -40°C und +100°C, bevorzugt bei Temperaturen zwischen 0°C und 50°C. Bevorzugte inerte Lösungsmittel sind aromatische und aliphatische Kohlenwasserstoffe, Halogenkohlenwasserstoffe oder Ether; besonders bevorzugt sind Pentane, Hexane, Heptane, Octane, Petrolether, Benzol, Toluol, Xylole, Cumol, Decalin, Chlorbenzol, Brombenzol, Fluorbenzol, Trifluormethylbenzol, Dichlorbenzole, Trichlorbenzole, Dichlormethan, Chloroform, Tetrachlorkohlenstoff, 1,2-Dichlorethan, Tetrahydrofuran, 1,4-Dioxan, Dimethoxyethan, Diethylether, tert-Butylmethylether. Auch Mischungen dieser Lösungsmittel können eingesetzt werden.

Beispiele für die Herstellung von gemäß der vorliegenden Erfindung besonders bevorzugter Verbindungen sind in Schemata 5 und 6 sowie in den Beispielen aufgeführt. So können die besonders bevorzugten Nitrid-Komplexe **13a, 13b, 13c, 13d** aus dem gut zugänglichen Komplex **20** (Chiu, H.-T. et al. Adv. Mater. 1998, 10, 1475) oder aus den entsprechenden Halogenkomplexen 21 (Close M. R. et al., Inorg. Chem. 1994, 33, 4198; Geyer, A. M. et al. J. Am. Chem. Soc. 2008, 130, 8984) durch Umsetzung mit $Ph_3SiOH$ oder $Ph_3SiOM'$ (M' = Li, Na, K, Cs) und anschließender Zugabe eines komplexierenden Liganden der allgemeinen Formel **19** erhalten werden. In Schema 5 ist die Darstellung der entsprechenden Komplexe mit 1,10-Phenanthrolin und 2,2'-Bipyridin dargestellt, die in fester Form an der Luft über mehrere Wochen stabil sind.

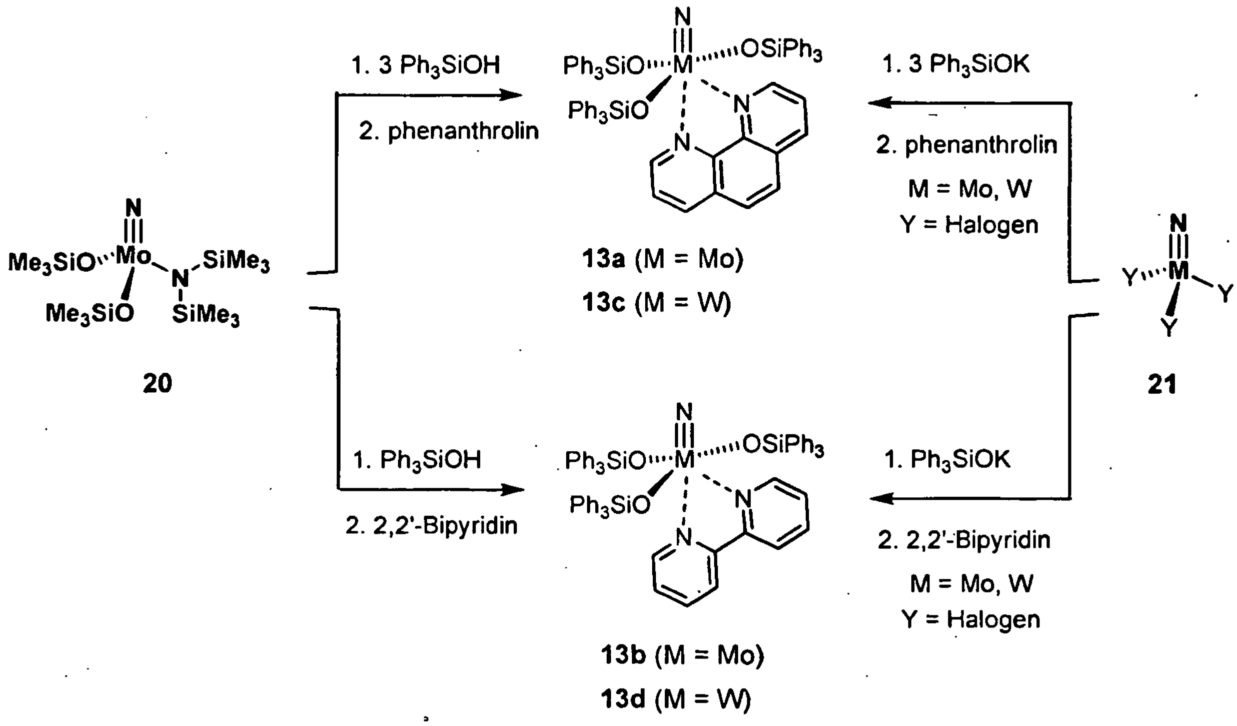


Schema 5. Nitrid-Komplexe des Wolfram und Molybdän mit stabilisierendem Phenanthrolin- oder oder 2,2-Bipyridinliganden, sowie repräsentative Darstellungsmethoden solcher Komplexe.

Analog kann der äußerst luft- und feuchtigkeitsempfindliche Komplex **23a** durch Zugabe von Liganden der Formel **19** in die entsprechenden Addukte überführt werden. Beispielhaft ist in Schema 6 die Umsetzung mit 1,10-Phenanthrolin und 2,2'-Bipyridin unter Bildung der besonders bevorzugten Komplexe **24a** und **24b** dargestellt, die in fester Form an der Luft über mehrere Wochen stabil sind.

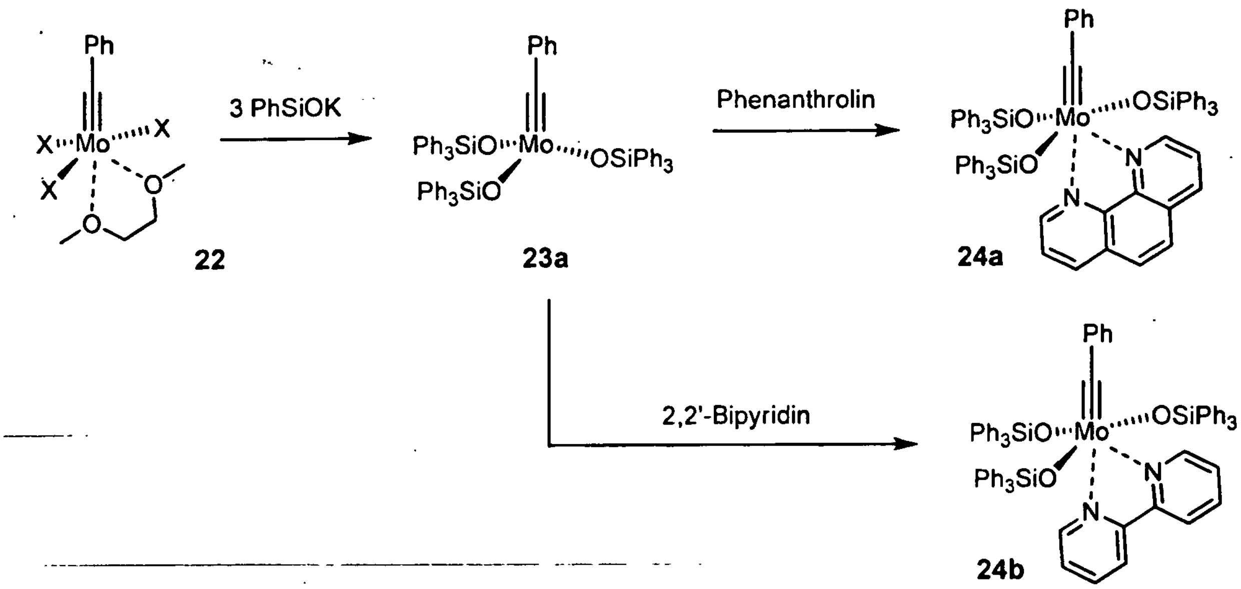


Schema 6. Beispiele einer bevorzugten Darstellungsweise für besonders bevorzugte Addukte von Schrock-Alkylidinkomplexen mit stabilisierenden Liganden; X = Halogen.

Der Komplex **23a** ist neu und lässt sich unter anderem aus den bekannten Komplexen des Typs $X_3Mo{\equiv}CPh$

(X = Cl, Br) (Mayr, A. et al. J. Am. Chem. Soc. 1986, 108, 548; McCullough, L. G. et al., J. Am. Chem. Soc. 1985, 107, 5987; Stevenson, M. A. et al., Organometallics 1997, 16, 3572) durch Umsetzung mit $Ph_3SiOM'$ (M' = Li, Na, K, Cs) in reiner Form oder, bevorzugt, in Form von Addukten mit einem Donorliganden wie zum Beispiel Acetonitril, Benzonitril, Pivalonitril, tert-Butylmethylether, Diethylether, Di-isopropylether, Diphenylether, Methoxybenzol, Tetrahydrofuran, Dioxan, Dimethoxyethan erzeugen.

Es wurde festgestellt, dass der Komplex **23a** sowie seine Addukte mit den genannten Donorliganden wie zum Beispiel Acetonitril, Benzonitril, Pivalonitril, tert-Butylmethylether, Diethylether, Di-isopropylether, Diphenylether, Methoxybenzol, Tetrahydrofuran, Dioxan, Dimethoxyethan hohe katalytische Aktivität in der Alkinmetathese selbst bei Raumtemperatur aufweisen. Hingegen zeigen die entsprechenden Komplexe **24a** und **24b** unter diesen Bedingungen nur geringe katalytische Aktivität. Wie oben erwähnt und unten weiter ausgeführt, haben wir jedoch gefunden, dass sich die katalytische Aktivität durch Zugabe von wasserfreien Metallsalzen oder Metallkomplexen als Additiva wieder herstellen lässt. Daher stellen Komplexe **24a** und **24b** neue Präkatalysatoren für die Alkinmetathese dar.

Ein weiterer Gegenstand der vorliegenden Erfindung sind Alkylidinkomplexe der allgemeinen Formel **25** sowie ihre Verwendung als Katalysatoren für die Alkinmetathese,

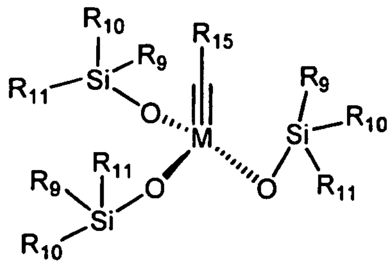

**25**

wobei

M = Mo, W,
die Substituenten $R^9$, $R^{10}$, $R^{11}$ gleich oder verschieden sein können und die oben angegebene Bedeutung haben, und
$R^{15}$ = $C_1$-$C_{12}$ Alkyl, $C_3$-$C_{12}$ Cycloalkyl, 6- bis 18-gliedriges Aryl, 5- bis 10-gliedriges Heteroaryl sein kann.

Ebenso sind Gegenstand der vorliegenden Erfindung die Addukte der Alkylidinkomplexe der allgemeinen Formel **25** mit Donor-Lösungsmitteln, die ausgewählt werden können aus der Liste: Acetonitril, Benzonitril, Pivalonitril, Diethylether, Di-isopropylether, tert-Butylmethylether Diphenylether, Methoxybenzöl, Tetrahydrofuran, Dioxan, Dimethoxyethan, sowie ihre Verwendung als Katalysatoren für die Alkinmetathese.

Bevorzugte Verbindungen der allgemeinen Formel **25** sind solche, in denen $R^{15}$ ausgewählt wird aus der Liste: Methyl, Ethyl, Propyl, Butyl, iso-Propyl, tert-Butyl, Trimethylsilylmethyl, Benzyl, (Dimethyl)(phenyl)methyl, Phenyl, Methylphenyl, Dimethylphenyl, Trimethylphenyl, Methoxyphenyl, Dimethoxyphenyl, Fluorphenyl, Pentafluorphenyl, Trifluormethylphenyl, Furyl.

Sofern die Existenz von Verbindungen der Formel **25** in der Literatur postuliert wurde, konnten sie jedoch weder durch Isolierung noch mit Hilfe spektroskopischer Methoden nachgewiesen werden. Daher sind Verbindungen der Formel **25** neu. Wir haben festgestellt, dass diese Komplexe sowie ihre Addukte mit Donor-Lösungsmitteln hohe katalytische Aktivität in Alkinmetathese Reaktionen aufweisen, weshalb diese Verbindungen ebenfalls Gegenstand der vorliegenden Erfindung bilden.

Besonders bevorzugt sind Schrock-Alkylidinkomplexe der allgemeinen Formeln **23, 26, 27, 28, 29, 30, 34** sowie ihre Addukte mit Acetonitril, Benzonitril, Pivalonitril, tert-Butylmethylether, Diethylether, Di-isopropylether, Diphenylether, Methoxybenzol, Tetrahydrofuran, Dioxan, Dimethoxyethan,

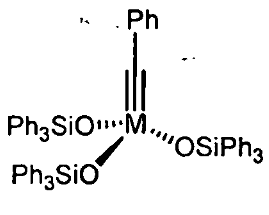

**23**

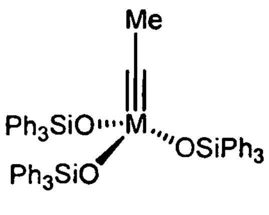

**26**

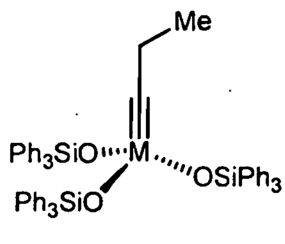

**27**

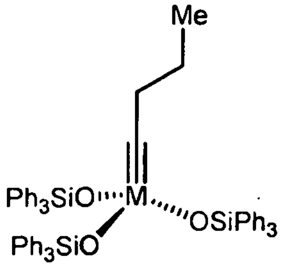

28

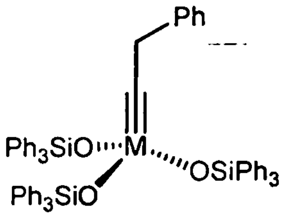

29

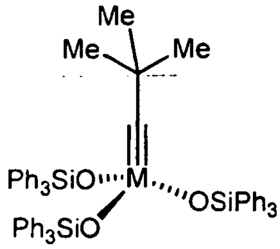

30

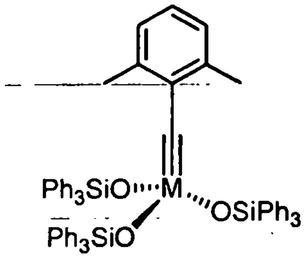

34

mit M = Mo, W.

Besonders bevorzugt sind ebenfalls Komplexe der allgemeinen Formel **31** sowie ihre Addukte mit Acetonitril, Benzonitril, Pivalonitril, tert-Butylmethylether, Diethylether, Di-isopropylether, Diphenylether, Methoxybenzol, Tetrahydrofuran, Dioxan, Dimethoxyethan,

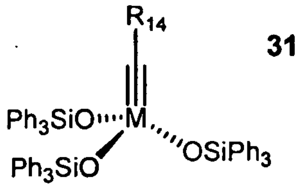

**31**

wobei

M = Mo, W

und $R^{14}$ gleich definiert ist wie oben für Komplexe der allgemeinen Formel **17a** und **17b**.

Exemplarisch für besonders bevorzugte Verbindungen stehen die in den Beispielen näher beschriebenen Komplexe **23a**($Et_2O$), **30a**(MeCN), **33**($Et_2O$):

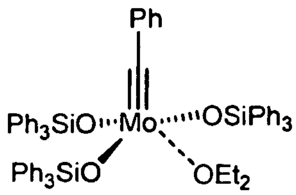

**23a**($Et_2O$)

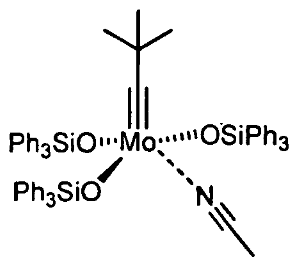

**30a**(MeCN)

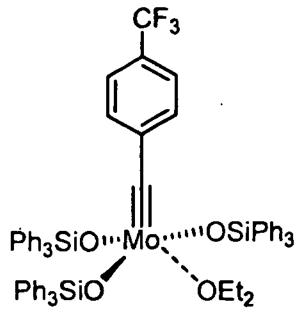

**33**($Et_2O$)

Ebenfalls Gegenstand der vorliegenden Erfindung ist ein Verfahren zur Durchführung von Alkinmetathese Reaktionen unter Verwendung eines Komplexes der allgemeinen Formeln **I, 11, 11a, 12, 12a, 17a, 17b** oder der Komplexe **13a, 13b, 13c, 13d, 13e, 14a, 14b, 15a, 15b, 16a, 16b, 24a, 24b, 24c, 24d, 24e** oder **34a** als Präkatalysatoren. Das Verfahren ist dadurch gekennzeichnet, dass eine Lösung oder eine Suspension der genannten Präkatalysatoren in einem geeigneten Lösungsmittel mit wasserfreien Metallsalzen oder Metallverbindungen als Additiva umgesetzt wird, die ihrerseits stabile Komplexe mit den als stabilisierenden Liganden verwendeten Verbindungen der allgemeinen Formel **19** bilden können. Dadurch erfolgt eine Aktivierung der als Präkatalysatoren dienenden Komplexe, vermutlich durch Dekomplexierung des stabilisierenden Liganden der allgemeinen Formel **19** aus dem jeweils verwendeten Präkatalysator. Die dabei gebildeten Addukte des Liganden der allgemeinen Formel **19** mit dem zugesetzten Additiv können wahlweise im Reaktionsgemisch verbleiben oder vor der Alkinmetathese aus dem Gemisch abgetrennt werden. Für diese Aktivierung kommen grundsätzlich alle wasserfreien Metallsalze oder Metallverbindungen als Additiva in Betracht,

die stabile Komplexe mit Liganden der allgemeinen Formel **19** bilden. Beispiele für dieses neue Verfahren zur Durchführung von Alkinmetathese Reaktionen sind in Schema 7 gezeigt und im Abschnitt "Beispiele" beschrieben.

Zu den bevorzugten Additiva zählen: $MnY_2$, $FeY_2$, $FeY_3$, $CoY_2$, $CuY_2$, $ZnY_2$, $MgY_2$, $NiY_2$, $PdY_2$, $PtY_2$, $RuY_2$, $RuY_3$, $EuY_3$, mit Y = F, Cl, Br, I, Acetylacetonat, Sulfat, Sulfonat, Nitrat, Acetat, Trifluoracetat;

Besonders bevorzugte Additiva sind: $MnCl_2$, $FeCl_2$, $FeCl_3$, $CoCl_2$, $CuCl_2$, $ZnCl_2$.

Abhängig vom gewählten Additiv kann die Aktivierung bei unterschiedlichen Temperaturen durchgeführt werden, bevorzugt bei Temperaturen zwischen -20°C und +130°C, besonders bevorzugt bei Temperaturen zwischen 20°C und 100°C. Als Lösungsmittel kommen alle Lösungsmittel in Betracht, die nicht mit Metallnitriden oder Metallalkylidinen reagieren; bevorzugte Lösungsmittel sind Kohlenwasserstoffe, Halogenkohlenwasserstoffe und Ether; besonders bevorzugt sind Pentane, Hexane, Heptane, Octane, Petrolether, Benzol, Toluol, Xylole, Cumol, Decalin, Chlorbenzol, Brombenzol, Fluorbenzol, Trifluormethylbenzol, Dichlorbenzole, Trichlorbenzole, Dichlormethan, Chloroform, Tetrachlorkohlenstoff, 1,2-Dichlorethan, Tetrahydrofuran, 1,4-Dioxan, Dimethoxyethan, Diethylether, tert-Butylmethylether. Auch Mischungen dieser Lösungsmittel können eingesetzt werden.

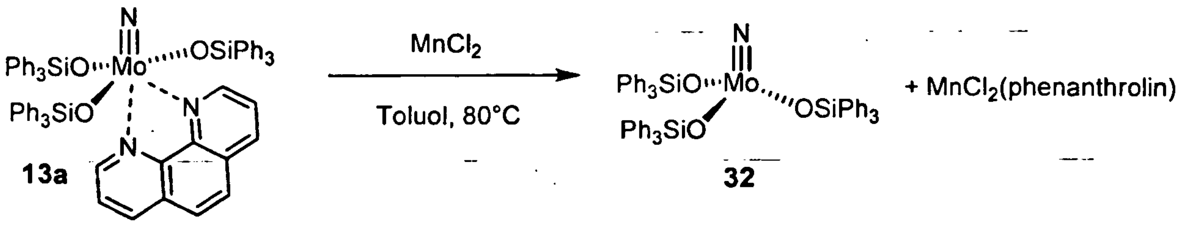


13a

32

Schema 7. Repräsentatives Beispiel der Aktivierung eines Nitrid-Komplexes des Molybdäns mit stabilisierenden Phenanthrolinligand durch Ligandentausch mit $MnCl_2$ als einem bevorzugten Additiv.

Ein weiterer Gegenstand der vorliegenden Erfindung ist ein Verfahren zur Durchführung von Alkinmetathese Reaktionen, in deren Verlauf 2-Butin als eines der Metatheseprodukte gebildet wird, dadurch gekennzeichnet, dass die Reaktion in Gegenwart von Molsieben als Additiva durchgeführt wird, die 2-Butin physikalisch oder chemisch binden.

Das Prinzip der Metathese wird durch das bereits oben dargestellte Schema 1 wiedergegeben:

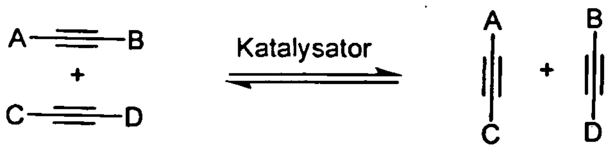


Die nachfolgenden Schemata 8 und 9 zeigen repräsentative Beispiele für AlkinMetathesereaktionen unter Freisetzung von 2-Butin.

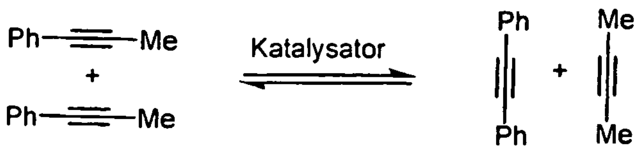


Schema 8: Intermolekulare Alkinmetathese-Reaktion

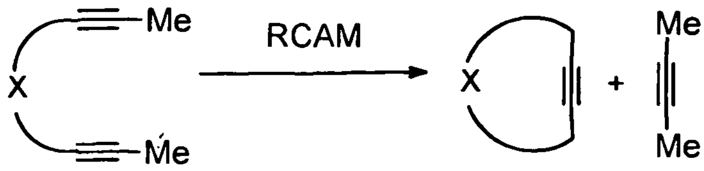


Schema 9: Ringshluss-Alkinmetathese (RCAM) unter Bildung von 2-Butin als Nebenprodukt

Überraschenderweise haben wir gefunden, dass der Zusatz von vorzugsweise getrocknetem Molsieb (auch Molekularsieb genannt) eine Beschleunigung der Reaktion sowie eine erhebliche Verbesserung der Ausbeute bewirkt. Das Molsieb kann dabei in Form von Pellets, Stäbchen, Kügelchen oder als Pulver zugesetzt werden; bevorzugt ist die Verwendung von Pulvern. Bevorzugt ist die Verwendung von Molsieb 5 Angström (MS 5 Å) sowie von Molsieb 4 Angström (MS 4 Å).

Der vorteilhafte Effekt des Molsiebs ist wahrscheinlich auf die Absorption oder Adsorption des 2-Butin zurückzuführen, wobei zusätzliche Effekte nicht ausgeschlossen werden können. Die Menge an Molsieb richtet sich nach der Menge des freiwerdenden 2-Butins und kann in einem weiten Bereich variiert werden. Bevorzugt ist die Verwendung von 1-4 Milligramm Molsieb pro Mikromol frei werdenden 2-Butins. Die Verwendung größerer Überschüsse an Molsieb ist möglich.

Figur 1 zeigt den Vergleich der alkinmetathetischen Umsetzung von 1-Propinylbenzol zu Tolan mit $Mo(\equiv CPh)(OSiPh_3)_3 \cdot OEt_2$ (**23a**$\cdot Et_2O$) (1 mol%) bei Raumtemperatur in Toluol. Die Ausbeuten wurden durch GC bestimmt (gegen Biphenyl als internem Standard). Die Kurve unter "Zusatz von Molsieb 5 Å" bezieht sich auf die Verwendung von gepulvertem MS 5 Å (2 mg pro µmol freiwerdendem 2-Butin). Wie in Figur 1 am Beispiel einer von Komplex **23a** katalysierten Alkinmetathese Reaktion gezeigt, lassen sich auf diese Weise selbst bei Raumtemperatur sehr hohe Umsätze erreichen, die unter sonst gleichen Bedingungen in Abwesenheit von Molsieb nicht erreicht werden:

Der in dieser Erfindung beschriebene Effekt des Molsiebs geht nicht auf eine Entfernung von Wasserspuren aus dem Reaktionsgemisch zurück. Damit unterscheidet sich die vorliegende Erfindung grundsätzlich von Verfahren zur Durchführung von Alkinmetathese Reaktionen, in denen MS 4 Å verwendet wurde, um Feuchtigkeit im Reaktionsgemisch zu binden (Huc, V. et al. New J. Chem. 2003, 27, 1412). In diesem Fall führt die Verwendung von MS 4 Å lediglich zu Ergebnissen, wie sie auch bei Verwendung getrockneter und destillierter Lösungsmittel in

Abwesenheit von MS 4 Å erreicht werden (Maraval, V. et al. Tetrahedron Lett. 2006, 47, 2155). Die Tatsache, dass der in der vorliegenden Erfindung beschriebene Effekt des Molsiebs, der zu einer Erhöhung der Reaktionsgeschwindigkeit sowie Verbesserung des Umsatzes führt, nicht auf Entfernung von Wasserspuren zurückgeht, ist auch daraus abzuleiten, dass die Verwendung von Molsieb 3Å keinen nennenswerten Effekt auf die Umsetzung von 1-Propinylbenzol zu Tolan ausübt. Molsieb 3Å bindet zwar Feuchtigkeit, kann jedoch Kohlenwasserstoffe mit 4 Kohlenstoffatomen wie 2-Butin nicht effizient binden.

Die gemäß der vorliegenden Erfindung durch Zusatz von Molsieb erreichte Erhöhung der Reaktionsgeschwindigkeit von Alkinmetathesereaktionen sowie Verbesserung des Umsatzes und damit der erzielbaren Ausbeute des jeweiligen Reaktionsproduktes beruht somit vermutlich auf der Entfernung des sonst im Gleichgewicht vorliegenden 2-Butins aus dem Reaktionsgemisch.

Das entstehende 2-Butin wird bisher bei Alkinmetathesen üblicherweise dadurch aus dem Reaktionsgemisch erreicht, dass die Reaktionen bei erhöhter Temperatur und/oder bei Unterdruck durchgeführt werden (Fürstner, A. et al., Angew Chem. 1998, 110, 1758; Beer, S. et al. Organometallics 2009, 28, 1534). Alternative Methoden zur Verbesserung des Umsatzes in Alkinmetathese Reaktionen beruhen bisher auf der Vermeidung von 2-Butin als Nebenprodukt: durch Verwendung modifizierter Substrate können an dessen Stelle Alkine gebildet werden, die aus dem Reaktionsgemisch ausfallen (Präzipitationsmethode) (Zhang, W. Org. Synth. 2007, 84, 163; Zhang, W. et al. J. Am. Chem. Soc. 2004, 126, 12796). Die dafür nötigen Substrate müssen jedoch in mehreren Stufen aufwendig hergestellt werden und sind auch aus der Sicht der Atomökonomie nicht ideal. Durch die vorliegende Erfindung kann die Herstellung solcher spezieller Substrate vermieden werden. Ebenso kann durch Zusatz von Molsieb, bevorzugt MS 4 Å oder MS 5 Å, bei der Durchführung der Alkinmetathese Reaktion auf erhöhte Temperaturen und/oder auf das Anlegen von Vakuum verzichtet werden.

Die Wirkung von Molsieb auf Alkinmetathese Reaktionen, in deren Verlauf 2-Butin als eines der Metatheseprodukte gebildet wird, ist unabhängig vom eingesetzten Präkatalysator oder Katalysator. Bevorzugt ist die Verwendung von Molsieben, besonders von MS 4 Å oder MS 5 Å, in Kombination mit einem der in der vorliegenden Erfindung genannten Präkatalysatoren oder Katalysatoren zur Durchführung von Alkinmetathese Reaktionen.

Ebenso bevorzugt ist die Verwendung von Molsieben, besonders von MS 4 Å oder MS 5 Å, in Kombination mit einem der folgenden Präkatalysatoren oder Katalysatoren zur Durchführung von Alkinmetathese Reaktionen: **1**

(Listemann, M. L. et al. Organometallics 1985, 4, 74), *2* (Beer, S. et al. Organometallics 2009, 28, 1534), **3** (Zhang, W. Org, Synth. 2007, 84, 163), **4** (Fürstner, A. et al. J. Am. Chem. Soc. 1999, 121, 9453), **5** (Geyer, A. M. et al. J. Am. Chem. Soc. 2008, 130, 8984), 6 (Geyer, A. M. et al. J. Am. Chem. Soc. 2008, 130, 8984), **9** (Fürstner A. et al., J. Am. Chem. Soc. 2009, 131, 9468).

Beispiele zur Darstellung von beanspruchten Katalysatoren und Präkatalysatoren, sowie für die Durchführung von Alkinmetathesereaktionen gemäß der vorliegenden Erfindung sind in Tabellen 1 - 3 sowie im Abschnitt "Beispiele" zusammengefasst.

Tabelle 1. Intermolekulare Alkinmetäthese Reaktionen.

| Substrat | Produkt | | **13a** [a] | **13b** [a] | **23a**·$Et_2O$ [b] | **24a** [c] | **24b** [c] |
|---|---|---|---|---|---|---|---|
| 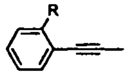 | 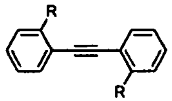 | R = H | 93-99% | 99% | 99% | 99% | 99% |
| | | R = OMe | 96% | 94% | 97% | 97% | 97% |
| | | R = SMe | 87% | 98% | 98%[d] | 96%[d] | 95%[d] |
| | | R = COOMe | | | | 92% | 94% |
| 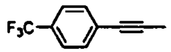 | 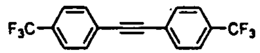 | | | 93% | 93% | | 94% |
| 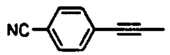 | 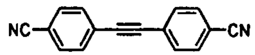 | | | | | | 94% |
| 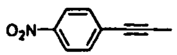 | 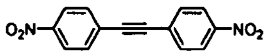 | | | | | | 69% |
| 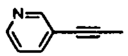 | 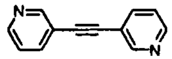 | | 85%[e] | | 90%[d] | 88%[d] | |
| 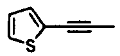 | 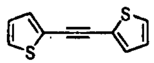 | | 96% | 93% | 88% | 87% | 90% |
| 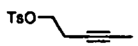 | 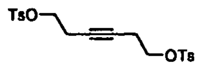 | | | | 92% | 92% | |
| 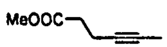 | 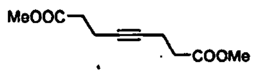 | | | 83% | 89% | 91% | 92% |
| 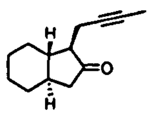 | 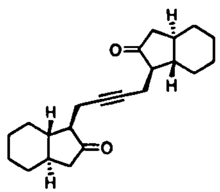 | | | | 92% | 88% | |

(fortgesetzt)

| Substrat | Produkt | **13a** [a] | **13b** [a] | **23a**·$Et_2O$ [b] | **24a** [c] | **24b** [c] |
|---|---|---|---|---|---|---|
| 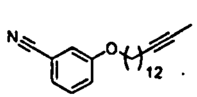 . | 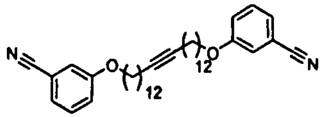 | | 81% | 87% | 89% | 89% |

[a] **13a** oder **13b** (10 mol%), $MnCl_2$ (10 mol%), MS 5 Å, Toluol, 80-100°C.
[b] **23a**·$Et_2O$, (2 mol%), Toluol, Raumtemperatur, MS 5 Å
[c] **24a** oder **24b** (5 mol%), $MnCl_2$ (5 mol%), Toluol, 80-100°C, 30 min; dann Zugabe des Substrates und Reaktion bei Raumtemperatur
[d] bei 50°C
[e] bei 100°C

Tabelle 2. Intramolekulare Alkinmetathese Reaktionen.

| Substrat | Produkt | **13a** [a] | **13b** [d] | **23a**·$Et_2O$ [b] | **24a** [c] | **24b** [c] |
|---|---|---|---|---|---|---|
| 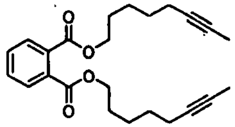 | 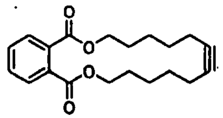 | | 69% | 97% | 94% | 90% |
| 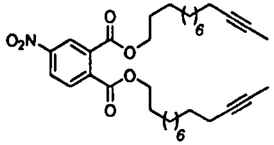 | 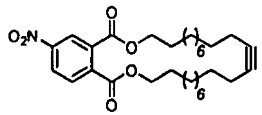 | | | 85% | | |
| 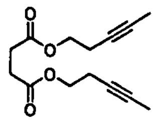 | 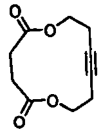 | 60% | 81% | 73% | 78% | 81% |
| 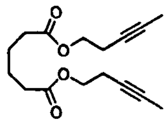 | 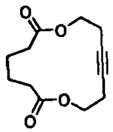 | | 86% | 92% | | 91% |
| 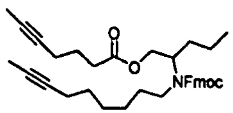 | 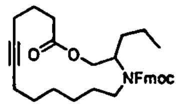 | | | 90% | | |
| 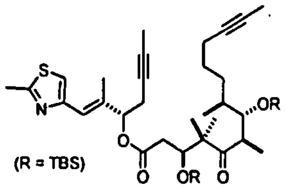 (R = TBS) | 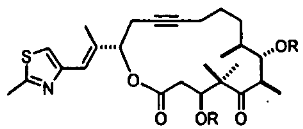 | | | 91% | | |

(fortgesetzt)

| Substrat | Produkt | **13a** [a] | **13b** [d] | **23a**·$Et_2O$ [b] | **24a** [c] | **24b** [c] |
|---|---|---|---|---|---|---|
| 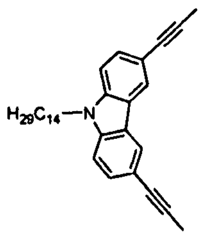 | 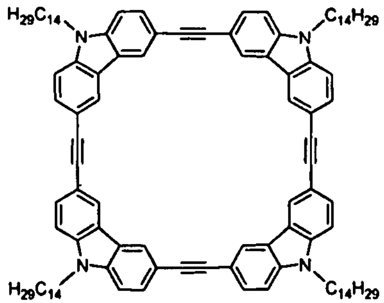 | | 80% | 82% | | 82% |

[a] **13a** (10 mol%), $MnCl_2$ (10 mol%), MS 5 Å, Toluol, 80-100°C.
[b] **23a**·$Et_2O$, (2 mol%), Toluol, Raumtemperatur, MS 5 Å
[c] **24a** (5 mol%), $MnCl_2$ (5 mol%), Toluol, 80-100°C. 30 min; danach Zugabe des Substrates, MS 5 Å und Durchführung der Alkinmetathese-Reaktion bei Raumtemperatur
[d] **13b** (10 mol%), $MnCl_2$ (10 mol%), Toluol, 80-100°C, ohne Zugabe von MS 5 Å.

Tabelle 3. Alkin-Kreuzmetathese Reaktionen.

| Substrate | | Produkt | **13a** [a] | **23a**·$Et_2O$ [b] | **24b** [c] |
|---|---|---|---|---|---|
| 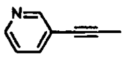 | 5-Decin | 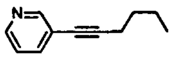 | | 65% | 75% |
| 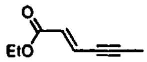 | Tolan | 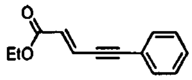 | | 62% | |
| 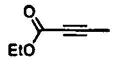 | 5-Decin | 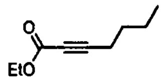 | 60% | | |
| 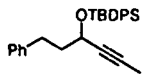 | Tolan | 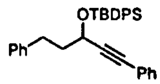 | | | 71% |

[a] **13a** (10 mol%), $MnCl_2$ (10 mol%), Toluol, 80-100°C, ohne MS 5 Å
[b] **23a**·$Et_2O$ (cat.), Toluol, Raumtemperatur, MS 5 Å
[c] **24b** (5 mol%), $MnCl_2$ (5 mol%), Toluol, 80-100°C, 30 min; danach Zugabe des Substrates, MS 5 Å und Durchführung der Alkinmetathese-Reaktion bei Raumtemperatur

## Beispiele

Abkürzungen: phen = 1,10-Phenanthrolin; bipy = 2,2'-Bipyridin; Ts = Tosyl; TMS = Trimethylsilyl; TBS = tert-Butyldimethylsilyl; TBDPS = tert-Butyldiphenylsilyl,;THP = Tetrahydropyranyl; Fmoc = 9-Fluorenylmethoxycarbonyl; dme = 1,2-Dimethoxyethan

Die zur Durchführung der Alkinmetathesen verwendeten Molsiebe wurden durch Erhitzen auf mindestens 200°C im Vakuum aktiviert und unter Argon aufbewahrt.

### I. Herstellung und Verwendung bevorzugter Katalysatoren sowie von davon abgeleiteten Phenanthrolin-Addukten

### 1. Darstellung von $Mo(\equiv N)(OSiPh_3)_3$ • phen (13a)

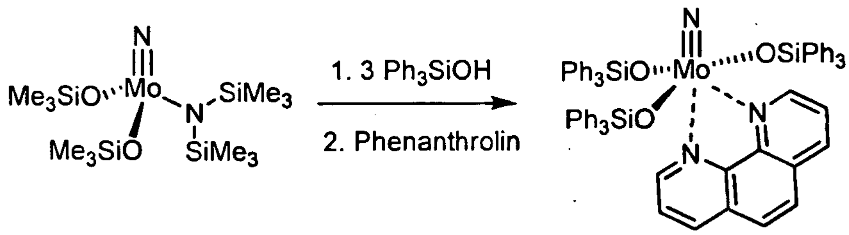


In einem trockenen und mit Argon gefluteten 500 mL Schlenk-Kolben wurde $[Mo(\equiv N)(OTMS)_2(N(TMS)_2)]$ (8.97 g, 20.0 mmol) vorgelegt und in 200 mL trockenem Toluol gelöst. $Ph_3SiOH$ (16.58 g, 60.0 mmol) wurde im Argon-Gegenstrom zugegeben und die erhaltene gelbliche Lösung 30 min bei Raumtemperatur gerührt. Die Lösung wurde in einen 1 L *Schlenk*-Kolben, der bereits 1,10-Phenanthrolin (doppelt sublimiert, 3.60 g, 20.0 mmol) enthielt, transferiert. Die erhaltene Reaktionsmischung wurde nach wenigen Minuten zu einer klaren gelblichen Lösung, einige Minuten später bildete sich langsam ein hellgelber Niederschlag. Nach 30 min wurde das Lösungsmittel entfernt und der erhaltene Rückstand 1 h bei Raumtemperatur im Hochvakuum ($10^{-3}$ mbar) und 1 h bei 60 °C im Hochvakuum (ca. $10^{-3}$ mbar) getrocknet. Der so erhaltene gelbe Feststoff wurde dreimal aus heißem Toluol umkristallisiert. Hierzu wurde er zunächst in 500 mL heißem Toluol (105 °C) gelöst, ein verbleibender unlöslicher Rest wurde durch Filtration der heißen Lösung entfernt. Die Lösung wurde dann langsam auf Raumtemperatur gekühlt und 2 Tage stehen gelassen. Im Anschluss wurde die grüne Lösung von den gelben Kristallen (14.44 g) ab dekantiert und die Kristalle im Vakuum getrocknet. Die Mutterlauge wurde bei Raumtemperatur zur Trockene eingeengt und der zuvor beschriebene Vorgang mit 200 ml heißem Toluol wiederholt. Es wurden weitere 2.95 g Produkt erhalten. Ein dritter Kristallisationsdurchgang mit 100 mL Toluol

führte zu weiteren 1.59 g Produkt. Insgesamt wurden 18.98 g [Mo(≡N)($OSiPh_3$)$_3$(phen)] in Form gelber Kristalle erhalten. Je nach Trocknungsgrad kann der Feststoff wechselnde Mengen an Toluol enthalten. Dieser Komplex kann über Monate an der Luft ohne erkennbare Zersetzung gelagert werden. $^1$H NMR (400 MHz, $CD_2Cl_2$): δ = 9.20 (dd, *J* = 5.0, 1.6 Hz, 1H), 8.98 (dd, *J* = 4.5, 1.6 Hz, 1H), 8.24 (dd, *J* = 8.2, 1.6 Hz, 1 H), 7.89-7.85 (m, 6H), 7.83 (dd, *J* = 8.2, 1.6 Hz, 1 H), 7.67 (d, *J* = 8.9 Hz, 1 H), 7.59 (dd, *J* = 8.2, 4.5 Hz, 1 H), 7.46 (d, *J* = 8.9 Hz, 1H), 7.31-7.25 (m, 3H), 7.14-7.10 (m, 6H), 7.10-7.05 (m, 18H), 6.98 (dd, *J* = 8.1, 5.0 Hz, 1H), 6.91-6.85 (m, 12H); $^{13}$C NMR (100 MHz, $CD_2Cl_2$): δ= 156.2, 147.7, 142.2, 141.5, 139.0, 138.0, 137.7, 137,2, 136.1, 135.2, 134.9, 129.6, 129.5, 129.0, 128.3, 127.9, 127.6, 127.4, 127.1, 127.1, 124.3, 124.2; IR (ATR): 3044, 3021, 1588, 1518, 1495, 1483, 1426, 1341, 1298, 1262, 1222, 1188, 1113, 1104, 1056, 1028, 1011, 998, 942, 893, 865, 840, 768, 740, 727, 708, 697 cm$^{-1}$; MS (ESI$^+$): *m/z* (%): 1118 (100) [M+H$^+$], 1043 (10); HRMS (ESI$^+$): *m/z:* berechnet für $C_{66}H_{54}MoN_3O_3Si_3$ [*M+H$^+$*]: 1118.2513, gefunden: 1118.2523. Die Struktur von Mo(≡N)($OSiPh_3$)$_3$(phen) wurde durch Kristallstrukturanalyse bestätigt (Figur 2).

**2. Darstellung von Mo(≡CPh)($OSiPh_3$)$_3$ • $OEt_2$ (23a·$Et_2O$)**

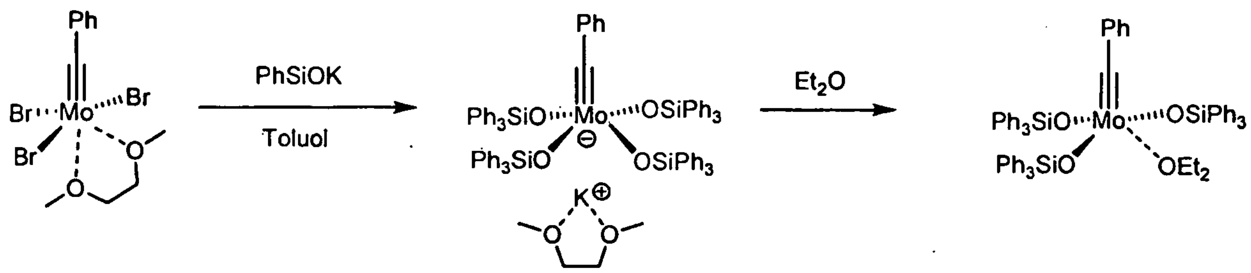


In einem trockenem und mit Argon gefluteten 100 mL *Schlenk*-Kolben wurde Mo(≡CPh)$Br_3$(dme) (472 mg, 0.92 mmol) in 10 mL Toluol gelöst und mit einer Lösung von $Ph_3SiOK$ (925 mg, 2.94 mmol) in 10 mL Toluol versetzt. Nach 1 h bei Raumtemperatur wurde filtriert und das Lösungsmittel im Vakuum entfernt. Der Rückstand wurde mit 10 mL Diethylether versetzt, wobei ein hell-violetter Feststoff ausfiel. Nach Waschen mit 10 mL Diethylether und Trocknen im Vakuum wurde Mo(≡CPh)($OSiPh_3$)$_3$$OEt_2$ als hell-violetter Feststoff (751 mg, 75 %) erhalten. $^1$H-NMR (400 MHz, [$D_8$]-Toluol): δ (ppm) = 7.71 (dd, *J* = 8.0, 1.3 Hz, 18 H); 7.08 (tt, *J*=7.4, 1.4 Hz, 9H); 6.93 (t, *J* = 7.6 Hz, 18H); 6.84 (t, *J = 7.7 Hz,* 2H); 6.68 (tt, *J* = 7.4, 1.2 Hz, 1 H); 6.56 (d, *J* = 7.6 Hz, 2H); 3.29 (q, *J* = 7.0 Hz, 4H); 1.12 (t, *J* = 7.0 Hz, 6H); $^{13}$C-NMR (100 MHz, [$D_8$]-Toluol): δ (ppm) = 282.6, 145.7, 140.4, 138.2, 137.4, 131.2, 130.8, 129.7, 128.8, 128.4, 128.1, 127.5, 126.3, 66.6, 16.2.

**3. Darstellung von Mo(≡$CC_6H_4CF_3$)($OSiPh_3$)$_3$ • $OEt_2$ (33·$Et_2O$)**

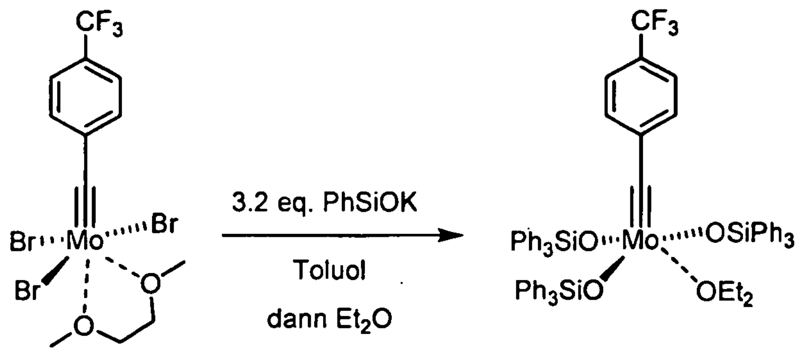


Die Darstellung erfolgte analog zur obigen Vorschrift. Der Komplex zeigt folgendes charakteristisches NMR Signal: $^{19}$F-NMR (300 MHz, [$D_8$]-Toluol): δ (ppm) = -62.8 (s).

**4. Darstellung von Mo(≡CtBu)($OSiPh_3$)$_3$(MeCN) (30·MeCN)**

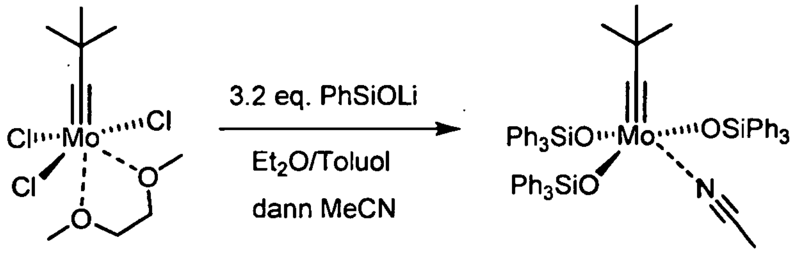


Der Komplex wurde analog zur obigen Vorschrift dargestellt, wobei während der Aufarbeitung Acetonitril statt Diethylether verwendet wurde. Charakteristische Daten: $^1$H-NMR (300 MHz, [$D_8$]-Toluol): δ (ppm) = 7.83 (dd, *J* = 7.7, 1.3 Hz, 18 H); 7.21-7.10 (m, 27H), 0.56 (s, 3H), 0.38 (s, 9H); $^{13}$C-NMR (75 MHz, [$D_8$]-Toluol): δ (ppm) = 323.5, 136.9, 135.9, 130.1, 128.2, 127.9, 54.9, 29.4, 1.4. Die Struktur von Komplex **30•MeCN** wurde durch Kristallstrukturanalyse bestätigt (Figur 3).

**5. Darstellung von Mo(≡CPh)($OSiPh_3$)$_3$(phen) (24a)**

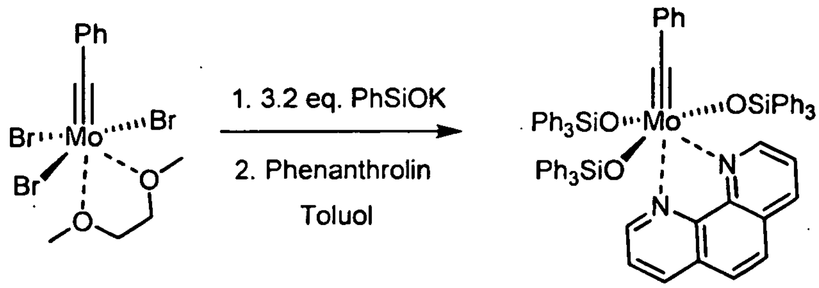


In einem trockenen und mit Argon gefluteten 100 mL *Schlenk*-Kolben wurde Mo(≡CPh)$Br_3$(dme) (492 mg, 0.96 mmol) in 15 mL Toluol gelöst und mit einer Lösung von $Ph_3SiOK$ (962 mg, 3.06 mmol) in 15 mL Toluol versetzt. Nach 1 h bei Raumtemperatur wurde filtriert und das Filtrat zu einer Lösung von Phenanthrolin (156 mg, 0.86 mmol) in 5 mL Toluol getropft, wobei intensive Violettfärbung auftritt; mit zunehmender Konzentration erscheint die Lösung beinahe schwarz. Nach 1 h bei Raumtemperatur wurden der Lösung 35 mL Diethylether zugegeben und die Reaktionsmischung über Nacht zur Kristallisation stehen gelassen. Der Feststoff wurde zweimal mit jeweils 10 mL Diethylether gewaschen. Nach Trocknung im Hochvakuum wurde Mo(≡CPh)($OSiPh_3$)$_3$(phen) als violetter Feststoff (483 mg, 43 %) erhalten. $^1$H-NMR (400 MHz, $CD_2Cl_2$): δ (ppm) = 9.26 (dd, *J* = 4.6, 1.6 Hz, 1 H), 9.14 (dd, *J* = 4.3, 1.8 Hz, 1 H), 8.90 (dd, *J* = 5.0, 1.8 Hz, 1 H), 8.33 (dd, *J* = 8.2, 1.6 Hz, 1 H), 8.30 (dd, *J* = 8.1, 1.8 Hz, 1 H), 7.89 (dd, *J* = 8.0, 1.3 Hz, 3H), 7.82 (dd, *J* = 8.2, 1.5 Hz, 1 H), 7.74 (d, *J* = 8.8 Hz, 1 H), 7.66-7.58 (m, 2H), 7.53 (d, *J* = 8.8 Hz, 1H), 7.49-7.43 (m, 6H), 7.41-7.37 (m, 4H), 7.28 (tt, *J* = 7.6, 1.3 Hz, 5H), 7.19 (tt, *J* = 7.4, 1.4 Hz, 2H), 7.06 (tt, J = 7.4, 1.3 Hz, 4H), 7.03 (dd, *J*=8.0, 1.4 Hz, 8H), 6.96-6.92 (m, 5H), 6.86 (tt, J = 7.4, 1.2 Hz, 1 H), 6.80 (t, *J* = 7.9 Hz, 8H), 6.31 (dd, *J* = 8.4, 1.4 Hz, 2H). $^{13}$C-NMR (100 MHz, $CD_2Cl_2$): δ (ppm) = 142.7, 139.4, 136.2, 135.8, 135.5, 135.3, 135.2, 131.2, 130.2, 129.2, 128.6, 128.3, 128.1, 127.7, 127.3, 127.2, 127.0; der Alkylidinköhlenstöff konnte nicht detektiert werden; IR (Film): 3065, 3047, 3022, 1958, 1895, 1823, 1649, 1625, 1587, 1566, 1516, 1483, 1427, 1381, 1342, 1302, 1260, 1221, 1186, 1141, 1110, 1031, 1017, 998, 927, 867, 836, 754, 739, 725, 696 $cm^{-1}$. Die Struktur von Komplex **24a** wurde durch Kristallstrukturanalyse bestätigt (Figur 4).

**6. Darstellung von W(≡CPh)($OSiPh_3$)$_3$·(phen)**

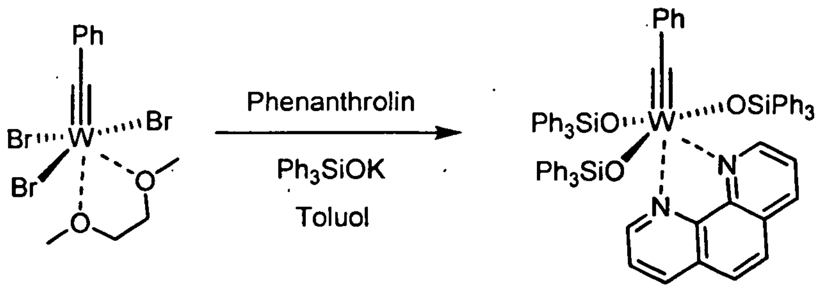


In einem trockenem und mit Argon gefluteten 25 mL *Schlenk*-Kolben wurde [W(≡CPh)$Br_3$·(dme)] (349 mg, 0.58 mmol) in Toluol (3 mL) gelöst und mit einer Lösung von Phenanthrolin (104 mg, 0.58 mmol) in Toluol (2 mL) versetzt. Nach 1 h bei Raumtemperatur wurde eine Lösung von $Ph_3SiOK$ (546 mg, 1.74 mmol) in Toluol zugegeben (5 mL) und das Gemisch 1 h bei 80°C gerührt. Anschließend wurde das Lösungsmittel im Vakuum entfernt und der Rückstand mit Diethylether (20 mL) gewaschen. Der erhaltene Feststoff wurde in Dichlormethan (5 mL) gelöst und das Produkt mit Pentan (25 mL) ausgefällt. Nach Waschen des abfiltrierten Feststoffes mit Pentan (5 mL) und Trocknen im Vakuum wurde [W(≡CPh))$(OSiPh_3)_3$(phen)] als rot-brauner Feststoff (158 mg, 21%) erhalten. $^1H$ NMR (400 MHz, $CD_2Cl_2$): δ = 9.35 (dd, *J* = 5.1, 1.6 Hz, 1 H), 9.27 (dd, *J* = 4.6, 1.6 Hz, 1H), 8.34 (dd, *J* = 8.2, 1.5 Hz, 1 H), 7.92 (dd, *J* = 8.0, 1.4 Hz, 6H), 7.86 (dd, *J* = 8.3, 1.5 Hz, 1 H), 7.73 (d, *J* = 8.8 Hz, 1H), 7.60 (dd, *J*= 8.2, 4.6 Hz, 1 H), 7.52 (d, *J* = 8.9 Hz, 1H), 7.21 (tt, *J*= 7.5, 1.4 Hz, 3H), 7.14-7.03 (m, 20H), 6.96 (t, *J* = 7.7 Hz, 6H), 6.89 (dd, *J* = 8.2, 5.1 Hz, 1 H), 6.80 (t, *J* = 7.7 Hz, 12H), 6.58 (tt, *J* = 7.4, 1.2 Hz, 1H), 6.21 (dd, *J* = 8.3, 1.2 Hz, 2H); $^{13}C$ NMR (100 MHz, $CD_2Cl_2$): δ = 281.7, 158.1, 147.3, 145.5, 143.6, 143.2, 139.0, 138.5, 138.4, 137.7, 136.3, 135.7, 135.3, 135.0, 134.8, 130.3, 129.9, 129.3, 128.7, 128.3, 127.8, 127.7, 127.5, 127.3, 127.2, 126.3, 125.6, 124.9, 124.1; IR (Film, $cm^{-1}$): 3047, 2996, 1585, 1516, 1485, 1426, 1260, 1110, 1017, 999, 932, 836, 739, 695.
Die Kristallstruktur von [W(≡CAr)$(OSiPh_3)_3$(phen)] ist in Figur 5 dargestellt.

**7. Darstellung von Mo(≡CAr)$(OSih_3)_3$ mit Ar = 2,6-Dimethyphenyl (34)**

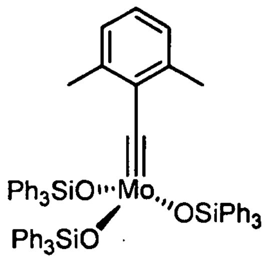

In einem trockenem und mit Argon gefluteten 25 mL *Schlenk*-Kolben wurde [Mo(≡CAr)$Br_3$ (dme)] (243 mg, 448 μmol) in Toluol (3 mL) gelöst und mit einer Lösung von $Ph_3SiOK$ (422 mg, 1.34 mmol) in Toluol (2 mL) versetzt. Nach 1 h bei Raumtemperatur wurde filtriert und das Filtrat eingeengt. Der Rückstand wurde aus Pentan (25 mL) umkristallisiert. Nach dem Trocknen im Vakuum wurde [Mo(sCAr)$(OSiPh_3)_3$] als gelber Feststoff erhalten (289 mg, 62%). $^1H$ NMR (400 MHz, $CD_2Cl_2$): δ = 7.56 (d, *J* = 7.2 Hz, 18H), 7.36 (t, *J* = 7.8 Hz, 9H), 7.21 (t, *J* = 7.4 Hz, 18H), 6.64 (t, *J* = 7.6 Hz, 1H) 6.55 (dd, *J* = 7.6, 0.6 Hz, 2H), 1.71 (s, 6H); $^{13}C$ NMR (100 MHz, $CD_2Cl_2$): δ = 307.3, 144.7, 139.6, 136.5, 136.0, 136.7, 135.8, 135.5, 135.4, 135.2, 130.5, 130.3, 129.8, 128.7, 128.3, 128.1, 127.7, 127.2, 126.1, 20.2.

Die Kristallstruktur von Mo(≡CAr)$(OSiPh_3)_3$ mit Ar = 2,6-Dimethylphenyl ist in Figur 6 dargestellt.

**8. Darstellung von [Mo(≡CAr)$(OS:Ph_3)_3$·(phen)] mit Ar = 2,6-Dimethylphenyl (34a)**

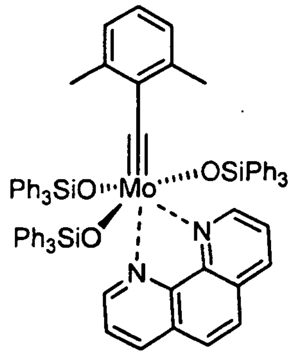

In einem trockenem und mit Argon gefluteten 25 mL *Schlenk*-Kolben wurde [Mo(≡CAr)$Br_3$'(dme)] (89 mg, 164 μmol) in Toluol (3 mL) gelöst und mit einer Lösung von $Ph_3SiOK$ (206 mg, 656 μmol) in Toluol (2 mL) versetzt. Nach 1 h bei Raumtemperatur wurde filtriert und zum Filtrat eine Lösung von Phenanthrolin (29 mg, 164 μmol) in Toluol (1 mL) zugegeben. Anschließend wurde 1 h bei Raumtemperatur gerührt, bevor das Lösungsmittel im Vakuum entfernt wurde. Der Rückstand wurde mit Diethylether (5 mL) gewaschen, in Dichlormethan (1 mL) aufgenommen und das Produkt durch Zugabe von Pentan (5 mL) ausgefällt. Nach Waschen des Niederschlags mit Pentan (5 mL) und Trocknen im Vakuum wurde [Mo(≡CAr))$(OSiPh_3)_3$(phen)] als grüner Feststoff erhalten (146 mg, 73%). IR (Film, $cm^{-1}$): 3048, 2995, 1588, 1515, 1482, 1426, 1262, 1110, 1017, 999, 932, 836, 739, 695. $^1H$ NMR (400 MHz, $CD_2Cl_2$): δ = 9.38 (dd, *J* = 5.1, 1.6 Hz, 1H), 8.86 (dd, *J* = 4.6, 1.6 Hz, 1H), 7.99(dd, *J* = 8.2, 1.6 Hz, 1 H), 7.92 (dd, *J* = 8.2, 1.6 Hz, 1 H), 7.89 (dd, *J* = 8.0, 1.4 Hz, 6H), 7.59 (d, *J* = 8.8 Hz, 1 H), 7.55 (d, *J* = 8.8 Hz, 1 H), 7.21 (tt, *J* = 7.5, 1.4 Hz, 3H), 7.18 (dd, *J* = 8.2, 5.0 Hz, 1H), 7.14 (dd, *J* = 8.2, 4.6 Hz, 1H), 7.06-6.99 (m, 12H), 6.85-6.72 (m, 27H), 2.30 (s, 6H); $^{13}C$ NMR (100 MHz, $CD_2Cl_2$): δ = 297.9, 156.4, 148.0, 143.4, 142.9, 142.6, 142.4, 139.1, 138.9, 138.8, 137.2, 136.4, 135.5, 135.3, 130.5, 130.3, 130.1, 129.9, 128.4, 128.3, 128.1, 127.7, 127.6, 127.1, 127.0, 126.8, 126.4, 124.2, 124.1, 21.4.

Die Kristallstruktur von von [Mo(≡CAr)$(OSiPh_3)_3$·(phen)] mit Ar = 2,6-Dimethylphenyl ist in Figur 7 dargestellt. Der Kristall enthält co-kristallisiertes $CH_2Cl_2$.

**9. Alkin-Ringschlussmetathese unter Verwendung von [Mo(≡N)$(OSiPh_3)_3$(phen)].**

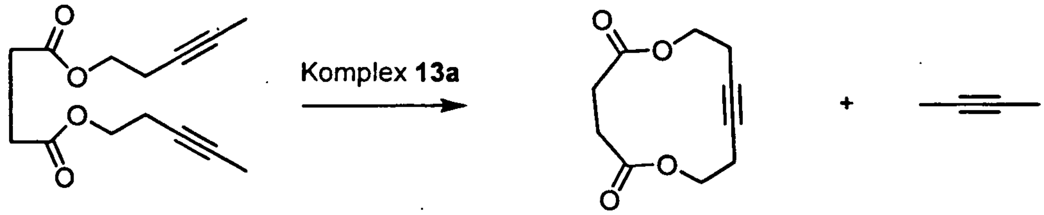


In einem trockenen und mit Argon gefluteten 500 mL *Schlenk*-Kolben wurden Bernsteinsäuredipent-3-inylester (5.00 mmol, 1.251 g), [Mo(≡N)$(OSiPh_3)_3$(phen)]·0.5 Toluol (0.500 mmol, 581.2 mg), $MnCl_2$ (0.500 mmol, 62.9 mg) und Molsieb (10.0 g, 5 A, Pulver) in 250 ml Toluol suspendiert. Die Reaktionslösung wurde 24 h bei 80 °C gerührt und nach dem Erkalten über ein kurzes Kieselgel-Polster filtriert. Das Filtrat wurde eingeengt und der Rückstand säulenchomatographisch gereinigt (Hexan/EtOAc 4:1). Das 1,6-Dioxazyklododec-9-in-2,5-dion wurde als farbloses Öl isoliert (Ausbeute 60%). $^1H$ NMR (400 MHz, $CD_2Cl_2$): δ = 4.21-4.14 (m, 4H), 2.61 (s, 4H), 2.44-2.37 (m, 4H); $^{13}C$ NMR (100 MHz, $CD_2Cl_2$): δ = 171.9 (2xC), 79.0 (2xC), 61.6 (2x$CH_2$), 30.2 (2x$CH_2$), 19.7 (2x$CH_2$); IR (film): $\tilde{\nu}$ = 2965, 2915, 2840, 1729, 1458, 1421, 1383, 1353, 1336, 1267, 1251, 1158, 1053, 1030, 1000, 952, 837 $cm^{-1}$; MS (EI) *m/z* (%): 166 (1), 101 (14), 78 (100), 66 (59), 65 (16), 55 (7), 40 (12), 28 (7); HRMS (ESI): *m/z:* berechnet für $C_{10}H_{12}O_4$ + Na: 219.0628; gefunden: 219.0627.

**10. Alkin-Ringschlussmetathese unter Verwendung von [Mo(≡CPh)$(OSiPh_3)_3(Et_2O)$].**

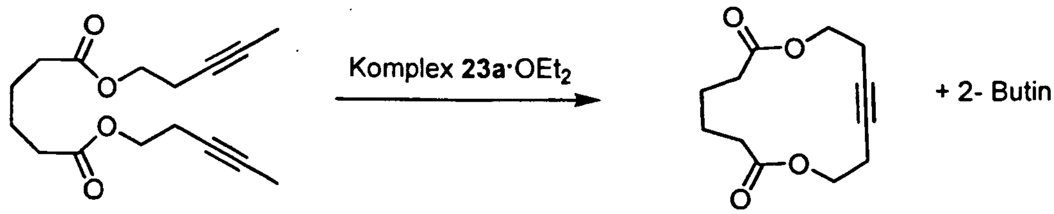


In einem trockenen und mit Argon gefluteten 100 mL *Schlenk*-Kolben wurden Adipinsäuredipent-3-inylester (135 mg, 485 $\mu$mol), Komplex **23a**·$OEt_2$ (10.5 mg, 9.67 $\mu$mol, 2 mol%) und Molsieb (970 mg, 5 A, Pulver) in 24.2 mL Toluol suspendiert. Die Reaktionslösung wurde 24 h bei Raumtemperatur gerührt, zur Aufarbeitung über ein kurzes Kieselgelpolster filtriert, das Filtrat wurde eingeengt und der Rückstand säulenchromatographisch ($SiO_2$, Hexan/EtOAc, 20:1) gereinigt. Man erhält 1,8-Dioxazyklotetradec-11-in-2,7-dion (100 mg, 92%) als farblosen Feststoff. M. p. = 109-110°C; $^1H$ NMR (400 MHz, $CD_2Cl_2$): $\delta$ = 4.13-4.06 (m, 4H), 2.53-2.47 (m, 4H), 2.39-2.30 (m, 4H), 1.76-1.67 (m, 4H); $^{13}C$ NMR (100 MHz, $CD_2Cl_2$): $\delta$ = 173.2 (2xC), 78.2 (2xC), 62.8 (2x$CH_2$), 35.2 (2x$CH_2$), 25.4 (2x$CH_2$), 19.4 (2x$CH_2$); IR (film) $\tilde{v}$ = 2995, 2954, 2937, 2918, 2901, 2872, 1721, 1458, 1425, 1384, 1341, 1272, 1236, 1167, 1140, 1080, 1065, 1021, 981, 931, 843, 824, 699 $cm^{-1}$; MS (EI) *m/z* (%): 129 (3), 111 (8), 78 (100), 66 (20), 55 (15), 41 (8); HRMS (ESI): *m/z:* berechnet für $C_{12}H_{16}O_4$ + Na: 247.0941; gefunden: 247.0938.

Die folgenden Verbindungen wurden analog dargestellt. Für die je nach gewählter Methode erhaltenen Ausbeuten, siehe Tabelle 2

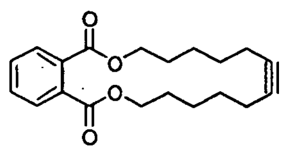

Farbloses Öl. $^1H$ NMR (400 MHz, $CD_2Cl_2$): $\delta$ = 7.72 (dd, *J* = 5.7, 3.3 Hz, 2H), 7.55 (dd, *J* = 5.7, 3.3 Hz, 2H), 4.32 (t, *J* = 6.0 Hz, 4H), 2.21-2.14 (m. 4H), 1.82-1.73 (m, 4H), 1.60-1.47 (m, 8H); $^{13}C$ NMR (100 MHz, $CD_2Cl_2$): $\delta$ = 168.0 (2xC), 132.8 (2xC), 131.3 (2xCH), 129.1 (2xCH), 80.9 (2xC), 66.7 (2x$CH_2$), 28.7 (2x$CH_2$), 28.5 (2x$CH_2$), 26.3 (2x$CH_2$), · 18.9 (2x$CH_2$); IR (film): $\tilde{v}$ = 2928, 2859, 1720, 1600, 1579, 1488, 1460, 1447, 1433, 1385, 1269, 1122, 1070, 1039, 957, 734, 703 $cm^{-1}$; MS (EI) *m/z* (%): 328 [$m^+$] (8), 180 (9), 162 (30), 149 (100), 133 (17), 122 (18), 121 (26), 119 (14), 108 (43), 107 (18), 105 (15), 95 (11), 94 (24), 93 (44), 91 (29), 81 (19), 80 (28), 79 (34), 77 (13), 67 (19), 55 (13); HRMS (ESI): *m/z*: calcd for $C_{20}H_{24}O_4$ + Na: 351.1567; found: 351.1567; Elementaranalyse (%) berechnet für $C_{20}H_{24}O_4$: C 73.15, H 7.37; gefunden: C 73.26, H 7.28.

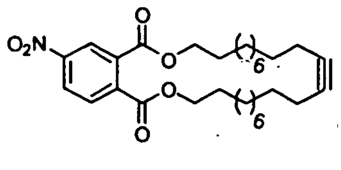

Weißer Feststoff. M. p. = 81-82°C; $^1H$ NMR (400 MHz, $CDCl_3$): $\delta$ = 8.60 (d, *J* = 2.2 Hz, 1 H), 8.37 (dd, *J* = 8.4, 2.2 Hz, 1 H), 7.85 (d, *J* = 8.4 Hz, 1 H), 4.35-4.30 (m, 4H), 2.15 (br s, 4H), 1.74 (sext, *J* = 7.1 Hz, 4H), 1.45-1.29 (m, 28H); $^{13}C$ NMR (100 MHz, $CDCl_3$): $\delta$ = 166.3 (C), 165.2 (C), 148.8 (C), 138.2 (C), 133.2 (C), 130.1 (CH), 125.8 (CH), 124.4 (CH), 80.6 (2xC), 66.7 (2x$CH_2$), 29.6 (2x$CH_2$), 29.5 ($CH_2$), 29.4 ($CH_2$), 29.3 (2x$CH_2$), 29.1 ($CH_2$), 29.1 ($CH_2$), 28.6 ($CH_2$), 28.6 ($CH_2$), 28.5 ($CH_2$), 28.4 ($CH_2$), 28.4 ($CH_2$), 28.4 ($CH_2$), 25.8$^2$ ($CH_2$), 25.7 ($CH_2$), 18.5 (2x$CH_2$); IR (film): $\tilde{v}$ = 3108, 3077, 3046, 2918, 2851, 1741, 1720, 1613, 1548, 1532, 1468, 1354, 1298, 1272, 1241, 1136, 1061, 997, 962, 932, 861, 835, 733, 725 $cm^{-1}$; MS (EI) *m/z* (%): 496 (19), 195 (37), 194 (32), 192 (33), 191 (11), 180 (13), 179 (19), 178 (69). 177 (15), 164 (22), 149 (22), 148 (11), 136 (12), 135 (30), 123 (12), 122 (14), 121 (43), 111 (23), 110 (16), 109 (29), 108 (14), 107 (29), 105 (11), 97 (15), 96 (28), 95 (64), 94 (26), 93 (46), 91 (19), 83 (38), 82 (30), 81 (84), 80 (42), 79 (54), 69 (92), 68 (25), 67 (84), 57 (20), 56 (11), 55 (100), 54 (24), 43 (30), 41 (61); HRMS (ESI): *m/z*: berechnet für $C_{30}H_{43}NO_6$ + Na: 536.2982; gefunden: 536.2989.

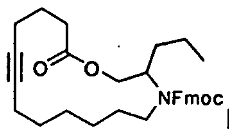

Farbloses Öl. $^1H$ NMR (Rotamere, 400 MHz, $CDCl_3$): δ = 7.76 (d, *J* = 7.4 Hz, 2H), 7.61-7.53 (m, 2H), 7.42-7.28 (m, 4H), 4.72-4.49 (m, 2H), 4.34-4.24 (m, 2H), 3.88-3.79 (m, 1.5H), 3.41 (br s, 0.5H), 3.02-2.91 (m, 1H), 2.65-2.40 (m, 2H), 2.28-2.15 (m, 3H), 2.13-2.01 (m, 1H), 1.83-1.70 (m, 2H), 1.64-0.77 (m, 14.5H), 0.70 (br s, 1.5H); $^{13}C$ NMR (Rotamere, 150 MHz, $CDCl_3$): δ = 173.3 (C), 173.2 (C), 156.8 (C), 156.4 (C), 144.1 (C), 144.0 (C), 143.9 (C), 141.4 (C), 141.3 (C), 127.6 (CH), 127.5 (CH), 127.1 (CH), 127.0 (CH), 124.6 (CH), 119.9 (CH), 119.8 (CH), 82.7 (C), 79.6 (C), 79.4 (C), 66.5 ($CH_2$), 66.2 ($CH_2$), 63.4 ($CH_2$), 47.5 (CH), 47.3 (CH), 34.9 ($CH_2$), 34.6 ($CH_2$), 34.5 ($CH_2$), 32.4 ($CH_2$), 32.3 ($CH_2$), 28.4 ($CH_2$), 28.2 ($CH_2$), 26.7 ($CH_2$), 26.6 ($CH_2$), 23.5 ($CH_2$), 23.4 ($CH_2$), 19.5 ($CH_2$), 18.2 ($CH_2$), 17.7 ($CH_2$), 14.0 ($CH_3$), 13.8 ($CH_3$); IR (film): $\tilde{\nu}$ = 3065, 3043, 3020, 2931, 2859, 1734, 1688, 1450, 1410, 1386, 1328, 1312, 1292, 1268, 1241, 1222, 1155, 1135, 1054, 1010, 758, 737 $cm^{-1}$; MS (EI) *m/z* (%): 236 (3), 180 (4), 179 (36), 178 (100), 165 (2); HRMS (ESI): *m/z:* berechnet für $C_{32}H_{39}NO_4$ + Na: 524.2771; gefunden: 524.2776.

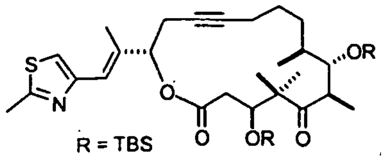

R = TBS

Amorpher Feststoff. $[\alpha]_D^{20} = -18.5$ (c = 0.6 in $CHCl_3$); $^1H$ NMR (600 MHz, $CDCl_3$): δ = 6.96 (s, 1 H), 6.55 (br s, 1 H), 5.33 (dd, *J* = 7.3, 3.1 Hz, 1H), 4.70 (dd, *J* = 6.4, 5.0 Hz, 1H), 3.93 (dd, *J* = 6.8, 1.9 Hz, 1 H), 3.24 (quin, *J* = 6.9 Hz, 1 H), 2.78 (dq, *J* = 17.2, 2.8 Hz, 1 H), 2.71 (s, 3H), 2.69-2.63 (m, 2H), 2.57 (dd, *J* = 15.3, 5.0 Hz, 1 H), 2.27-2.21 (m, 1H), 2.18 (d, *J* = 1.2 Hz, 3H), 2.13-2.07 (m, 1H), 1.78-1.71 (m, 1H), 1.59-1.53 (m, 1H), 1.30-1.20 (m, 2H), 1.41-1.34 (m, 1H), 1.16 (d, *J* = 7.0 Hz, 3H), 1.14 (s, 3H), 1.10 (s, 3H), 0.93 (d, *J* = 6.9 Hz, 3H), 0.91 (s, 9H), 0.86 (s, 9H), 0.10 (s, 3H), 0.09 (s, 3H), 0.08 (s, 3H), 0.07 (s. 3H); $^{13}C$ NMR (150 MHz, $CDCl_3$): δ = 216.7 (C), 170.1 (C), 164.9 (C), 152.3 (C), 136.9 (C), 120.4 (CH), 116.8 (CH), 82.2 (C), 78.0 (CH), 76.7 (CH), 76.3 (C), 72.6 (CH), 54.5 (C), 44.4 (CH), 41.6 ($CH_2$), 38.9 (CH), 29.5 ($CH_2$), 26.2 (3x$CH_3$), 26.1 (3x$CH_3$), 25.9 ($CH_2$), 24.1 ($CH_2$), 20.9 ($CH_3$), 20.5 ($CH_3$), 19.3 (C), 18.6 ($CH_3$), 18.5 ($CH_2$), 18.3 (C), 17.0 (2x$CH_3$), 15.0 ($CH_3$), -3.2 ($CH_3$), -3.7 ($CH_3$), -4.0 ($CH_3$), -4.1 ($CH_3$); IR (film): $\tilde{\nu}$ = 2952, 2929, 2894, 2856, 1739, 1702, 1505, 1472, 1463, 1385, 1361, 1254, 1182, 1152, 1097, 1085, 1039, 1018, 1007, 988, 836, 775 $cm^{-1}$; MS (EI) *m/z* (%): 703 [$M^+$] (5), 648 (23), 647 (47), 646 (97), 604 (11), 446 (14), 445 (33), 444 (100), 402 (22), 344 (15), 270 (38), 195 (12), 185 (18), 178 (12), 151 (21), 143 (13), 115 (13), 101 (12), 75 (45), 73 (51); HRMS (ESI): *m/z*: berechnet für $C_{38}H_{65}NO_5SSiO_2$ + Na: 726.4014; gefunden: 726.4015.

**11. Alkinmetathese durch Aktivierung von [Mo(≡N)(OSiPh$_3$)$_3$(phen)] mit Hilfe von $MnCl_2$ vor Zugabe des Substrats**

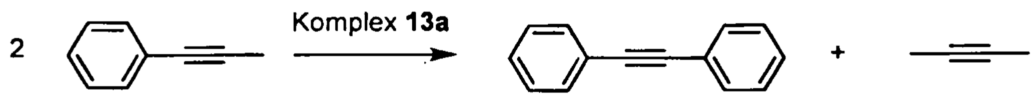


In einem trockenen und mit Argon gefluteten 25 mL *Schlenk*-Kolben wurden [Mo(≡N)(OSiPh$_3$)$_3$(phen)]·0.5 Toluol (0.100 mmol, 116.2 mg), $MnCl_2$ (0.100 mmol, 12.6 mg) und Molsieb (1.00 g, 5 Å, Pulver) in 5 ml Toluol suspendiert und das Gemisch auf 80 °C erhitzt. Nach 30 min wurde 1-Phenyl-1-propin (1.00 mmol, 116.2 mg) zugegeben, das Reaktionsgemisch 3 h bei 80 °C gerührt und nach dem Erkalten über ein kurzes Kieselgel Polster filtriert. Das Filtrat wurde eingeengt und der Rückstand säulenchromatographisch (Hexan) gereinigt. Es wurden 88.2 mg Tolan (99%) als weißer Feststoff isoliert, dessen spektroskopische und analytische Daten mit jenen einer kommerziellen Probe übereinstimmten. M. p. = 59-61 °C.

**12. Alkinmetathese durch Aktivierung von [Mo(≡N)($OSiPh_3$)$_3$(phen)] mit Hilfe von $MnCl_2$ in Gegenwart des Substrats**

In einem trockenen und mit Argon gefluteten 25 mL *Schlenk*-Kolben wurden 1-Phenyl-1-propin (1.00 mmol, 116.2 mg), [Mo(≡N)($OSiPh_3$)$_3$(phen)]·0.5 Toluol (0.100 mmol, 116.2 mg), $MnCl_2$ (0.100 mmol, 12.6 mg) und Molsieb (1.00 g, 5 Å, Pulver) in 5 ml Toluol suspendiert. Die Reaktionslösung wurde 3 h bei 80 °C gerührt und nach dem Erkalten über ein Kieselgel Polster filtriert. Das Filtrat wurde eingeengt und der Rückstand säulenchromatographisch (Hexan) gereinigt. Es wurden 88.1 mg Tolan (98%) als weißer Feststoff isoliert, dessen spektroskopische und analytische Daten mit jenen einer kommerziellen Probe übereinstimmten. M. p. = 59-61°C.

**13. Alkinmetathese ohne Zugabe von Molsieb durch Aktivierung von [Mo(=N)($OSiPh_3$)$_3$(phen)] mit Hilfe von $MnCl_2$ in Gegenwart des Substrats.**

In einem trockenen und mit Argon gefluteten 10_mL *Schlenk*-Kolben wurden [Mo(≡N)($OSiPh^3$)$_3$(phen)]·0.5 Toluol (50.0 µmol, 58.1 mg) und $MnCl_2$ (50.0 µmol, 6.3 mg) in 2.5 ml Toluol suspendiert. Nach Zugabe von 1-Phenyl-1-propin (500 µmol, 58.1 mg) wurde die Reaktionslösung 24h bei 80 °C gerührt und nach dem Erkalten über ein kurzes Kieselgelpolster filtriert. Das Filtrat wurde eingeengt und der Rückstand säulenchromatographisch (Hexan) gereinigt. Es wurden 41.6 mg Tolan (93%) als weißer Feststoff isoliert, dessen spektroskopische und analytische Daten mit jenen einer kommerziellen Probe übereinstimmten. M. p. = 59-61°C.

**14. Alkinmetathese ohne Zugabe von Molsieb durch Aktivierung von [Mo(≡N)($OSiPh_3$)$_3$(phen)] mit Hilfe von $CuCl_2$ in Gegenwart des Substrats.**

In einem trockenen und mit Argon gefluteten 10 mL *Schlenk*-Kolben wurden [Mo(≡N)($OSiPh_3$)$_3$(phen)]·0.5 Toluol (58.8 µmol, 68.3 mg) und getrocknetes $CuCl_2$ (58.8 µmol, 7.9 mg) in 2.9 ml Toluol suspendiert. Nach Zugabe von 1-Phenyl-:1-propin (588 µmol, 68.3 mg) wurde die Reaktionslösung 24 h bei 80 °C gerührt und nach dem Erkalten über ein kurzes Kieselgelpolster (2 cm) filtriert. Das Filtrat wurde eingeengt und der Rückstand säulenchromatographisch (Hexan) gereinigt. Es wurden 41.0 mg Tolan (78%) als weißer Feststoff isoliert, dessen spektroskopische und analytische Daten mit jenen einer kommerziellen Probe übereinstimmten. M. p. = 59-61 °C.

**15. Alkinmetathese unter Verwendung von Komplex 23a-$Et_2O$,**

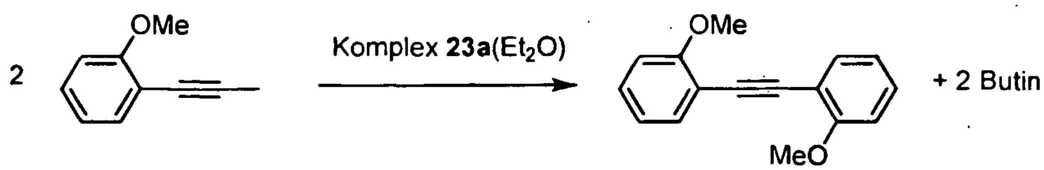


In einem trockenen und mit Argon gefluteten 25 mL *Schlenk*-Kolben wurden 1-Methoxy-2-(prop-1-yl)benzol (146 mg, 1 mmol), Komplex **23a**·$Et_2O$ (22 mg, 0.02 mmol, 2 mol%) und Molsieb (1 g, 5 A, Pulver) in 5 mL Toluol suspendiert. Das Reaktionsgemisch wurde 2 h bei Raumtemperatur gerührt, über ein kurzes Kieselgelpolster (2 cm) filtriert, das Filtrat wurde eingeengt, und der Rückstand mittels Säulenchromatographie ($SiO_2$, Hexan/EtOAc, 20:1) gereinigt. 1,2-Bis(2-methoxyphenyl)ethin (115 mg, 0.48 mmol, 97%) wurde als farbloser Feststoff erhalten. M. p. = 126-127°C; $^1H$ NMR (400 MHz, $CD_2Cl_2$): $\delta$ = 7.49 (ddd, *J* = 7.3, 1.6, 0.5 Hz, 2H), 7.33 (ddd, *J* = 7.9, 7.7, 1.9 Hz, 2H), 6.95 (td *J* = 7.9, 1.0 Hz, 4H), 3.92 (s, 6H); $^{13}C$ NMR (100 MHz, $CD_2Cl_2$): $\delta$ = 160.3 (2xC), 133.7 (2xCH), 130.1 (2xCH), 120.8 (2xCH), 113.0 (2xC), 111.3 (2xCH), 90.1 (2xC), 56.2 (2x$CH_3$); IR (film): $\tilde{v}$ = 3105, 3033, 2998, 2963, 2937, 2833, 1945, 1903, 1863, 1598, 1574, 1498, 1464, 1456, 1432, 1274, 1241, 1184, 1162, 1115, 1047, 1020, 937, 750 $cm^{-1}$; MS (EI) *m*/*z* (%):238 [$M^+$] (100), 237 (32), 223 (23), 221 (15), 207 (10), 195 (5), 178 (8), 165 (14), 152 (9), 131 (19), 111 (6), 97 (3), 89 (3); HRMS (ESI): *m*/*z*: berechnet für $C_{16}H_{14}O_2$ + Na: 261.0886; gefunden: 261.0884.

**17. Alkinmetathese durch Aktivierung von [Mo(≡CPh)($OSiPh_3$)$_3$(phen)] (24a) mit Hilfe von $MnCl_2$ vor Zugabe des Substrats**

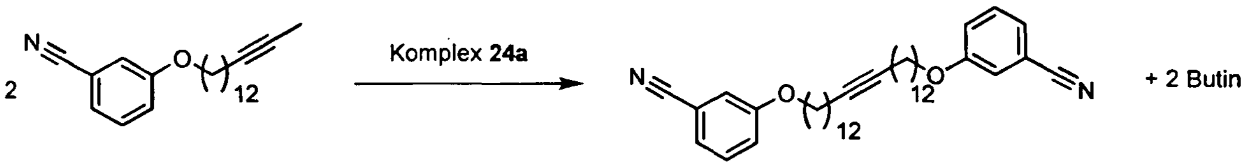


In einem trockenen und mit Argon gefluteten 25 mL *Schlenk*-Kolben wurden Komplex **24a** (60 mg, 0.05 mmol, 5 mol%) und $MnCl_2$ (6.3 mg, 0.05 mmol) in 1 mL Toluol gelöst. Anschließend wurde das Gemisch für 30 min auf 100 °C erhitzt und wieder auf Raumtemperatur abgekühlt. Die Katalysator-Lösung wurde in eine Suspension von 3-Cyanobenzoesäure-pentadec-13-inylester (325 mg, 1 mmol) und Molsieb (1 g, 5 A, Pulver) in 4 mL Toluol überführt und das Reaktionsgemisch für 2 h bei Raumtemperatur gerührt. Schließlich wurde über ein kurzes Kieselgelpolster (2 cm) filtriert, das Filtrat eingeengt, und der Rückstand säulenchromatographisch gereinigt ($SiO_2$ Hexan/EtOAc, 20:1). Man erhält das Produkt (265 mg, 89%) als farblosen Feststoff. $^1$H-NMR (400 MHz, $CDCl_3$): 6 (ppm) = 7.35 (td, *J* = 7.6, 1.2 Hz, 2 H); 7.22 (dt, *J* = 7.6, 1.2 Hz, 2H), 7.12-7.10 (m, 4H), 3.95 (t, *J* = 6.5 Hz, 4H), 2.13 (t, *J* = 7.1 Hz, 4H), 1.78 (quint., *J* = 8.0 Hz, 4H), 1.50-1.41 (m, 8H), 1.36-1.28 (m, 28H). $^{13}$C-NMR (100 MHz, $CDC_3$): δ(ppm) = 159.2, 134.9, 130.2, 130.1, 127.9, 124.2, 119.8, 118.8, 117.3, 113.1, 80.2, 68.4, 29.6, 29.5, 29.3, 29.1, 29.0, 28.8, 25.9, 18.7. IR (film): v = 3087, 2918, 2851, 2230, 1957, 1739, 1606, 1576, 1523, 1473, 1444, 1428, 1405, 1319, 1291; 1256, 1185, 1163, 1144, 1118, 1048, 1026, 999, 988, 904, 870, 834, 799, 782, $76_1$, 738, 718, 698, 681 $cm^{-1}$. MS (EI) m/z (%): 596 [M+] (100), 553 (6), 478 (7), 394 (6), 380 (11), 366 (17), 352 (15), 339 (47), 325 (73), 310 (15), 296 (13), 283 (8), 151 (6), 137 (11), 132 (9), 123 (18), 109 (34), 95 (66), 81 (67), 67 (49), 55 (68), 41 (21).

Folgende Verbindungen sind auf analoge Weise dargestellt worden; für die je nach verwendeter Methode erhaltenen Ausbeuten siehe Tabelle 1:

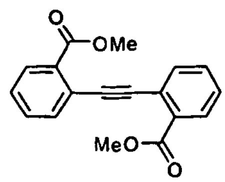

**Dimethyl 2,2'-(ethin-1,2-diyl)dibenzoat.** Gelber Feststoff, m. p. = 83-84°C; $^1$H NMR (400 MHz, $CDCl_3$): δ = 7.97-8.00 (m, 2H), 7.73 (ddd, *J* = 7.7, 1.2, 0.4 Hz, 2H), 7.51 (dt, *J* = 7.6, 1.4 Hz, 2H), 7.39 (dt, *J* = 7.7, 1.3 Hz, 2H), 3.96 (s, 6H); $^{13}$C NMR (100 MHz, $CDCl_3$): δ = 166.6, 134.3, 131.8, 131.7, 130.4, 128.1, 123.8, $93_{\cdot 1}$, 52.2; IR (ATR) $\tilde{v}$ = 2950, 1725, 1714, 1595, 1567, 1491, 1448, 1431, 1292, 1250, 1189, 1128, 1077, 1041, 963, 755, 699 $cm^{-1}$; MS (EI) *m/z* (%): 294 (11) [$M^+$], 280 (18), 279 (100), 265 (5), 264 (24), 248 (20), 220 (16), 176 (5), 163 (8), 132 (10), 102 (7), 88(9); HRNES (ESI): *m/z*: berechnet für $C_{18}H_{14}O_4$ + Na: 317.0784; gefunden: 317.0785.

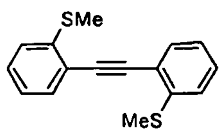

**1,2-Bis(2-(methylthio)phenyl)ethin.** Farbloser Feststoff. M. p. = 123-124°C; $^1$H NMR (400 MHz, $CD_2Cl_2$): δ = 7.52 (ddd, *J* = 7.9, 1.5, 0.5 Hz, 2H), 7.34 (ddd, *J* = 8.1, 7.4, 1.5 Hz, 2H), 7.22 (dd, *J* = 8.1, 0.7 Hz, 2H), 7.14 (td, *J* = 7.4, 1.2, Hz, 2H), 2.52 (s, 6H); $^{13}$C NMR (100 MHz, $CD_2Cl_2$): δ = 142.2 (2xC), 132.8 (2xCH), 129.4 (2xCH), 124.6 (2xCH), 124.5 (2xCH), 121.4 (2xC), 93.4 (2xC), 15.3 (2x$CH_3$); IR (film): $\tilde{v}$ = 3086, 3053, 3011, 2915, 2854, 2830, 1954, 1909, 1868, 1827, 1781, 1584, 1555, 1470, 1432, 1279, 1245, 1125, 1074, 1036, 972, 954 $cm^{-1}$; MS (EI) *m/z* (%): 270 [$M^+$] (16), 255 (53), 241 (17), 240 (100), 221 (19), 208 (6), 195 (5), 163 (5), 120 (15); HRMS (EI): *m/z:* berechnet für $C_{16}H_{14}S_2$: 270.0537; gefunden: 270.0535.

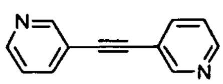

**1,2-Di(pyridin-3-yl)ethin.** Hellgelber Feststoff. $^1$H NMR (400 MHz, $CDCl_3$): δ =8.79 (br s, 2H); 8.58 (d, *J* = 4.0 Hz, 2H), 7.83 (dt, *J* = 7.8, 1.8 Hz, 2H), 7.31 (dd, *J* = 7.8, 5.0 Hz, 2H); $^{13}$C NMR (100 MHz, $CDCl_3$): δ = 152.4, 149.2, 138.7, 123.3,

119.9, 89.3; IR (film): 3029, 2924, 1987, 1952, 1916 1881, 1771, 1727, 1558, 1480, 1410, 1328, 1295, 1254, 1187, 1127, 1097, 1040, 1018, 959, 926, 882, 850, 809, 701 $cm^{-1}$; MS (EI) *m*/*z* (%): 180 (100) [$M^+$], 153 (7), 152 (6), 127 (11), 126 (5), 100 (6), 99(5), 76 (5), 74 (12), 63 (5); HRMS (EI): *m*/*z*: berechnet für $C_{12}H_8N_2$ [$M^+$]: 180.0687, gefunden: 180.0686.

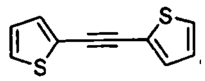

**1,2-Di(thiophene-2-yl)ethin.** Farbloser Feststoff. M. p. = 98-99°C; $^1$H NMR (400 MHz, $CD_2Cl_2$): $\delta$ = 7.35 (dd, *J* = 5.2, 1.1 Hz, 2H), 7.29 (dd, *J* = 3.6, 1.1 Hz, 2H), 7.04 (dd, *J* = 5.2, 3.6 Hz, 2H); $^{13}$C NMR (100 MHz, $CD_2Cl_2$): $\delta$ = 132.5 (2xCH), 128.2 (2xCH), 127.6 (2xCH), 123.1 (2xC), 86.4 (2xC); IR (film): $\tilde{v}$ = 3100, 3082, 1791, 1723, 1651, 1596, 1432, 1406, 1195, 1098, 1040, 1028, 849, 824, 692 $cm^{-1}$; MS (EI) *m*/*z* (%): 192 (9), 191 (13), 190 (100), 158 (8), 145 (13), 114 (9), 95 (8), 69 (6), 45 (5); HRMS (EI): *m*/*z*: berechnet für $C_{10}H_6S_2$: 189.9911; gefunden: 189.9912.

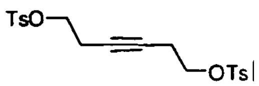

**Hex-3-in-1,6-diyl bis(4-methylbenzolsulfonat).** Farbloser Feststoff. M. p. = 83-84°C; $^1$H NMR (400 MHz, $CDCl_3$): $\delta$ = 7.77-7.80 (m, 4H), 7.34-7.36 (m, 4H), 4.01 (t, *J* = 7.0 Hz, 4H), 2.44-2.47 (m. 10H); $^{13}$C NMR (100 MHz, $CDCl_3$): $\delta$ = 145.0, 132.9, 129.9, 127.9, 76.8, 67.7, 21.6, 19.6; IR (film): $\tilde{v}$ = 2955, 2920, 1597, 1453, 1352, 1293, 1190, 1170, 1096, 969, 898, 841, 813, 761, 662 $cm^{-1}$; MS (EI): *m*/*z* (%): 423 (5), 422 (21) [$M^+$], 251 (11), 250 (10), 186 (6), 156 (8), 155 (86), 139 (21), 92 (9), 91 (100), 90 (7), 79 (10), 78 (62), 77 (9), 66 (7), 65 (47); HRMS (ESI): *m*/*z:* berechnet für [$C_{20}H_{22}O_6S_2$ + $Na^+$]: 445.0750; gefunden: 445.0750.

**17. Alkin-Kreuzmetathese unter Verwendung von [Mo(≡CPh)($OSiPh_3$)$_3$($Et_2O$)] (23a $Et_2O$)**

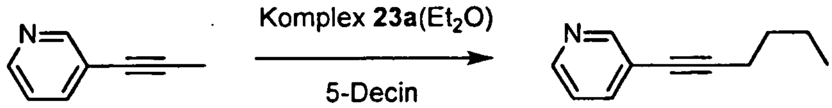


In einem trockenen und mit Argon gefluteten 25 mL *Schlenk*-Kolben wurden 3-(Prop-1-yl)-pyridin (117 mg, 1.00 mmol), 5-Decin (346 mg, 2.50 mmol), Komplex **23a**·$Et_2O$ (10.5 mg, 9.67 µmol, 2 mol%) und Molsieb (1 g, 5 Å, Pulver) in 5 mL Toluol suspendiert. Die Reaktionslösung wurde 4 h bei Raumtemperatur gerührt und zur Aufarbeitung durch ein kurzes Kieselgel-Polster filtriert. Das Filtrat wird eingeengt und der Rückstand säulenchromatographisch ($SiO_2$, Hexan/EtOAc, 20:1) gereinigt. Man erhält das Metatheseprodukt (103 mg, 65%) als farbloses Öl. $^1$H-NMR (400 MHz, $CDCl_3$): $\delta$ (ppm) = 8.61 (d, *J* = 1.5 Hz, 1H), _8.46 (dd, *J* = 4.9, 1.6 Hz, 1H), 7.66 (dt, *J* = 7.9, 1.9 Hz, 1H), 7.19 (ddd, *J* = 7.9, 4.9, 0.8 Hz, 1 H), 2.42 (t, *J* = 7.0 Hz, 2H), 1.63-1.56 (m, 2H), 1.52-1.43 (m, 2H), 0.95 (t, *J* = 7.2 Hz, 3H). $^{13}$C-NMR (100 MHz, $CDCl_3$): $\delta$ (ppm) = 152.3, 147.8, 138.4, 127.8, 122.8, 121.2, 94.0, 30.6, 22.0, 19.1, 13.6. IR (film): v = 3030, 2957, 2932, 2872, 2229, 1584, 1558, 1475, 1429, 1406, 1379, 1363, 1328, 1301, 1263, 1185, 1118, 1101, 1023, 1007, 949, 923, 889, 802, 747, 704 $cm^{-1}$. MS (EI) m/z (%): 159 [M+] (52), 144 (100), 130 (46), 116 (45), 103 (25), 89 (32), 77 (13), 63 (39), 51 (14), 41 (15), 39 (15), 27 (17).

**18. Cyclooligomerisierung durch Alkinmetathese unter Verwendung von [Mo(≡CPh)($OSiPh_{3h}$($Et_2O$)] (23a·$Et_2O$)**

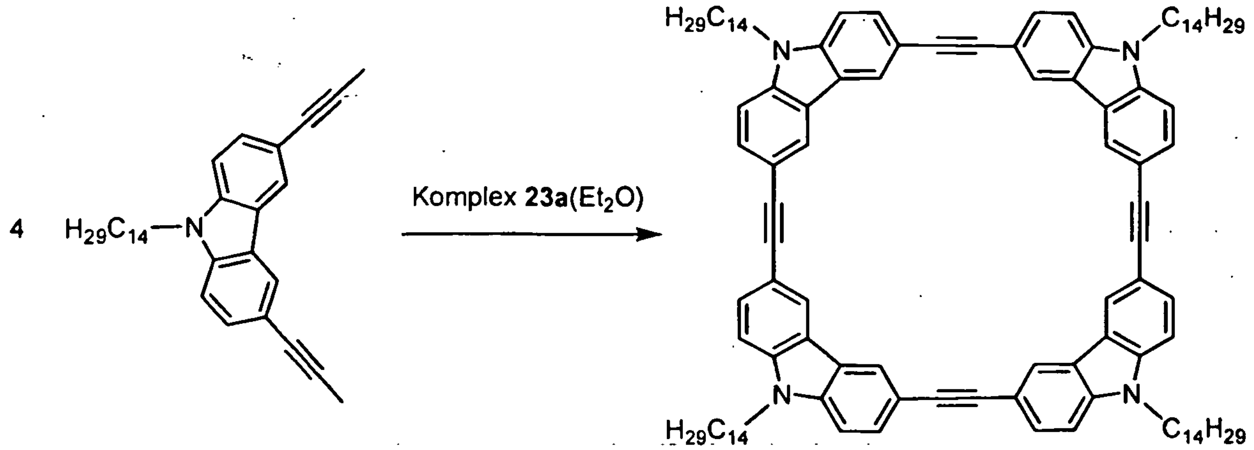


In einem trockenen und mit Argon gefluteten 25 mL *Schlenk*-Kolben wurden 3,6-Di(prop-1-inyl)-9-tetradecyl-9*H*-carbazol (439 mg, 100 mmol), Komplex **23a**·$Et_2O$ (22 mg, 0.02 mmol, 2 mol%) und Molsieb (1 g, 5 A, Pulver) in 5 mL Toluol suspendiert. Die Reaktionslösung wurde 4 h bei Raumtemperatur gerührt und zur Aufarbeitung über ein kurzes Kieselgel-Polster filtriert. Nach dem Einengen des Filtrates und säulenchromatographischer Reinigung des Rückstands ($SiO_2$, Hexan/$CHCl_3$, 1:1) wird aus $CH_2Cl_2$/Methanol (1:1) umkristallisiert und das Tetramer (316 mg, 82%) als farbloser Feststoff erhalten. $^1$H-NMR (400 MHz, $CD_2Cl_2$): δ (ppm) = 8.43 (s, 2H), 7.72 (dd, *J* = 8.3, 1.1 Hz, 2H), 7.40 (d, *J* = 8.4 Hz, 2H), 4.32 (t, *J* = 6.8 Hz, 2H), 1.91 (quin, *J* = 6.8 Hz, 2H), 1.44-1.25 (m, 22H), 0.88 (t, *J* = 6.5 Hz, 2H). $^{13}$C-NMR (100 MHz, $CD_2Cl_2$): δ (ppm) = 140.2, 129.3, 124.0, 122.7, 114.5, 108.9, 89.0, 31.9, 29.7, 29.67, 29.64, 29.60, 29.55, 29.50, 29.4, 29.0, 27.3, 22.7, 14.1.

**II. Herstellung und Verwendung weiterer bevorzugter Katalysatoren und deren Bipyridin-Addukten**

Die zur Darstellung der Komplexe verwendeten Bipyridin Derivate wurden in Toluol gelöst und die Lösung über Molsieb 5A (MS 5A) getrocknet. Anschließend wurde das Toluol im Vakuum abdestilliert und das verbleibende Bipyridin Derivat unter Argon aufbewahrt.

**1. Darstellung von [Mo(≡N)($OSiPh_3$)$_3$ (bipy)] (13b)**

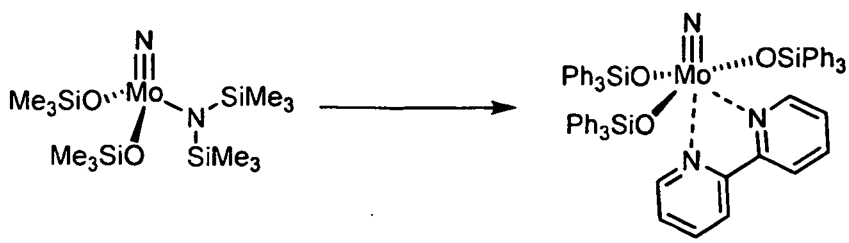

In einem trockenen und mit Argon gefluteten 50 mL *Schlenk*-Kolben wurde [Mo(≡N)(OTMS)$_2$(N(TMS)$_2$)] (1.15 g, 2.57 mmol) vorgelegt und in 25 mL trockenem Toluol gelöst. $Ph_3SiOH$ (7.13 g, 7.71 mmol) wurde im Argon-Gegenstrom zugegeben und die erhaltene gelbliche Lösung 30 min bei Raumtemperatur gerührt. Die Lösung wurde in einen 100 mL *Schlenk*-Kolben, der bereits 2,2'-Bipyridin (0.401 g, 2.57 mmol) enthielt, transferiert. Die erhaltene Reaktionsmischung wurde nach wenigen Minuten zu einer klaren gelblichen Lösung, einige Minuten später bildete sich langsam ein hellgelber Niederschlag. Nach 30 min wurde das Lösungsmittel entfernt und der erhaltene Rückstand 1 h bei Raumtemperatur im Hochvakuum ($10^{-3}$ mbar) und 1 h bei 60 °C im Hochvakuum ($10^{-3}$ mbar) getrocknet. Der so erhaltene gelbe Feststoff wurde zweimal aus heißem Toluol umkristallisiert. Hierzu wurde er zunächst in ca. 50 mL heißem Toluol (105 °C) gelöst, ein verbleibender unlöslicher Rest wurde durch Filtration der heißen Lösung entfernt. Die Lösung wurde dann langsam auf Raumtemperatur gekühlt und 2 Tage stehen gelassen. Im Anschluss wurde die grüne Lösung von den gelben Kristallen (0.743 g) abdekantiert und die Kristalle im Vakuum getrocknet. Die Mutterlauge wurde bei Raumtemperatur zur Trockene eingeengt und der zuvor beschriebene Vorgang mit ca. 25 ml heißem Toluol wiederholt. Es wurden weitere 1.355 g Produkt erhalten. Insgesamt wurden 2.098 g [Mo(≡N)($OSiPh_3$)$_3$(bipy)] in Form gelber Kristalle erhalten. Je nach

Trocknungsgrad kann der Feststoff wechselnde Mengen an Toluol enthalten. Dieser Komplex kann über Monate an der Luft ohne erkennbare Zersetzung gelagert werden. $^1$H NMR (400 MHz, $CD_2Cl_2$): $^1$H NMR (400 MHz, $CD_2Cl_2$): $\delta$ = 8.93 (dd, *J* = 5.5, 1.1 Hz, 1 H), 8.72-8.71 (m, 1 H), 7.81 (dd, *J* = 8.0, 1.6 Hz, 6H), 7.77 (td, *J* = 7.9, 1.6 Hz, 1 H), 7.48 (d, *J* = 8.2 Hz, 1H), 7.39 (td, *J* = 7.9, 1.6 Hz, 1 H), 7.27-7.16 (m, 23H), 7.07 (t, *J* = 7.8 Hz, 6H), 6.97 (t, *J* = 7.8 Hz, 12H), 6.73-6.70 (m, 1H). $^{13}$C NMR (100 MHz, $CD_2Cl_2$): $\delta$ = 156.5, 150.1, 149.9, 147.8, 139.5, 138.3, 138.0, 137.9, 136.1, 135.5, 135.3, 135.2, 129.6, 129.5, 129.8, 127.9, 127.8, 127.6, 125.6, 125.4, 122.1, 122.0.

Die Kristallstruktur von Mo(≡N)$(OSiPh_3)_3$(bipy) ist in Figur 8 dargestellt.

**2. Darstellung von [Mo(≡N)$(OSiPh_3)_3$ (4,4'-di-tert-butyl-2,2'-dipyridyl)] (13e)**

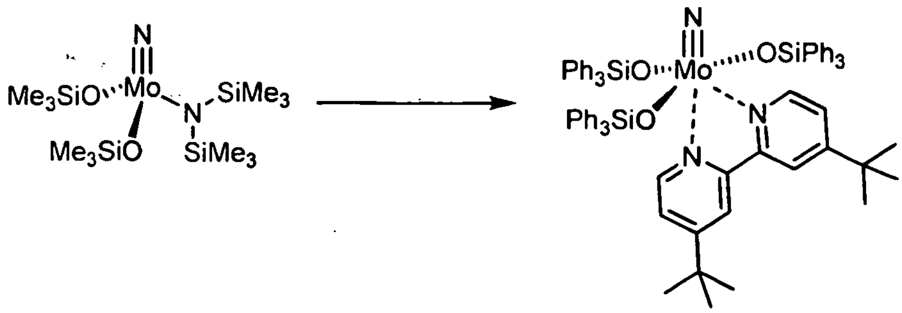

In einem trockenen und mit Argon gefluteten 10 ml *Schlenk*-Kolben wurde (Mo(≡N)$(OTMS)_2$(N$(TMS)_2$)] (67.3 mg, 0.15 mmol) vorgelegt und in 1.5 ml trockenem Toluol gelöst. $Ph_3SiOH$ (124.4 mg, 0.45 mmol) wurde im Argon-Gegenstrom zugegeben und die erhaltene gelbliche Lösung 30 min bei Raumtemperatur gerührt. Die Lösung wurde in einen 10 ml *Schlenk*-Kolben, der bereits 4,4'-di-tert-Butyl-2,2'-dipyridyl (40.3 mg, 0.15 mmol) enthielt, transferiert. Die erhaltene Reaktionsmischung wurde nach wenigen Minuten zu einer klaren gelblichen Lösung, nach ca. 1 h bildete sich langsam ein hellgelber Niederschlag. Nach 1.5 h wurde das Lösungsmittel entfernt und der erhaltene Rückstand 1 h bei Raumtemperatur im Hochvakuum ($10^{-3}$ mbar) und 1 h bei 60 °C im Hochvakuum ($10^{-3}$ mbar) getrocknet. Der so erhaltene Feststoff wurde aus heißem Toluol (2 mL) umkristallisiert. Es wurden Kristalle erhalten, die für eine Kristallstrukturanalyse geeignet Waren. Die Struktur des Komplexes im Festkörper ist in Figur 9 gezeigt.

**3. Darstellung von [Mo(≡CPh)$(OSiPh_3)_3$(bipy)] (24b)**

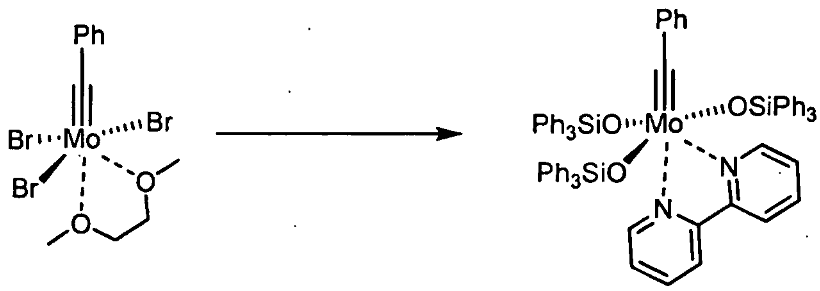

In einem trockenen und mit Argon gefluteten 100 mL *Schlenk*-Kolben wurde Mo(≡CPh)$Br_3$(dme) (855 mg, 1.66 mmol) in 25 mL Toluol gelöst und mit einer Lösung von $Ph_3SiOK$ (2.09 g, 6.64 mmol) in 25 mL Toluol versetzt. Nach 1 h bei Raumtemperatur wurde filtriert und das Filtrat zu einer Lösung von 2,2'-Bipyridin (259 mg, 1.66 mmol) in 5 mL Toluol getropft, wobei intensive Violettfärbung auftritt; mit zunehmender Konzentration erscheint die Lösung beinahe schwarz. Nach 1 h bei Raumtemperatur wurden der Lösung 35 mL Diethylether zugegeben und die Reaktionsmischung über Nacht zur Kristallisation stehen gelassen. Der Feststoff wurde zweimal mit jeweils 10 mL Diethylether gewaschen. Nach Trocknung im Hochvakuum wurde Mo(≡CPh)$(OSiPh_3)_3$(bipy) als violetter Feststoff (1.46 g, 75%) erhalten. $^1$H NMR (600 MHz, $CD_2Cl_2$): $\delta$ = 9.04 (dd, *J* = 5.0, 1.0 Hz, 1 H), 8.62 (dd, *J* = 5.4, 1.1 Hz, 1H), 7.87 (dd, *J* = 8.0, 1.4 Hz, 5H), 7.82 (td, *J* = 7.9, 1.7 Hz, 1 H), 7.65 (d, *J* = 6.9 Hz, 1H), 7.61 (d, *J* = 8.3 Hz, 1 H), 7.51 (dd, *J* = 8.0, 1.4 Hz, 2H), 7.41 (tt, *J* = 8.9, 1.4 Hz, 2H), 7.35 (td, *J* = 7.9, 1.7 Hz, 1 H), 7.31-7.28 (m, 4H), 7.23 (dd, *J* = 7.9, 1.4 Hz, 10H), 7.19 (tt, *J* =

7.5, 1.3 Hz, 3H), 7.15 (tt, *J* = 7.4, 1.3 Hz, 5H), 6.96 (t, *J* = 7.5 Hz, 6H), 6.93 (6.91 (t, *J* = 7.9 Hz, 11 H), 6.85 (tt, *J* = 7.4, 1.3 Hz, 1 H), 6.51 (tt, *J* = 7.4, 1.3 Hz, 1 H), 6.33 (dd, *J* = 8.3, 1.3 Hz, 2H). $^{13}C$ NMR (150 MHz, $CD_2Cl_2$): δ = 291.7, 155.7, 151.6, 151.0, 147.6, 143.3, 139.3, 139.0, 138.6, 138.4, 136.1, 135.7, 135.5, 135.4, 135.2, 131.1, 130.4, 130.2, 129.3, 129.1, 128.7, 128.5, 128.2, 128.1, 127.7, 127.4, 127.1, 126.6, 125.4, 125.0, 122.2, 122.1

Die Struktur des Komplexes wurde durch Kristallstrukturanalyse bestätigt (Figur 10).

**4. Darstellung von [Mo(≡CPh)($OSiPh_3$)$_3$(4,4'-Dimethyl-2,2'-dipyridyl)] (24c)**

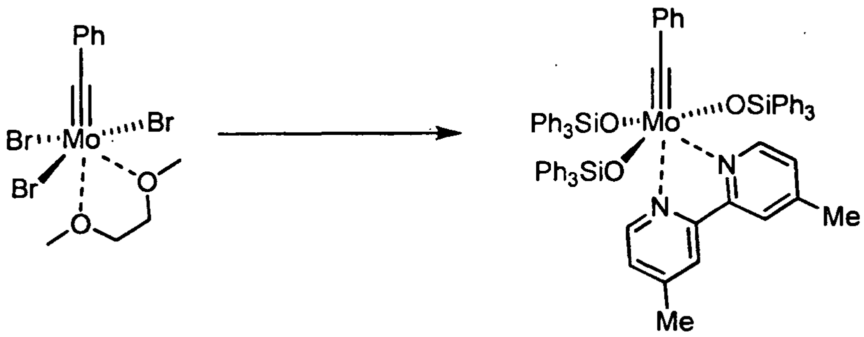

In einem trockenen und mit Argon gefluteten 100 mL *Schlenk*-Kolben wurde Mo(≡CPh)$Br_3$(dme) (46 mg, 89 μmol) in 0.5 mL Toluol gelöst und mit einer Lösung von $Ph_3SiOK$ (112 mg, 357 μmol) in 0.5 mL Toluol versetzt. Nach 1 h bei Raumtemperatur wurde filtriert und das Filtrat zu einer Lösung von 4,4'-Dimethyl-2,2'-bipyridin (16 mg, 89 μmol) in 1 mL Toluol getropft. Nach 1 h bei Raumtemperatur wurde der ausgefallene violette Feststoff abfiltriert und mit 5 mL Diethylether gewaschen. Nach Trocknung im Hochvakuum wurde [Mo(≡CPh)($OSiPh_3$)$_3$(4,4'-Dimethyl-2,2'-bipyridyl)] als violetter Feststoff (83 mg, 78 %) erhalten. $^1H$ NMR (600 MHz, $CD_2Cl_2$): δ = 8.85 (d, *J* = 7.9 Hz, 1H), 8.49 (d, *J* = 7.5 Hz, 1H), 8.47 (d, *J* = 8.5 Hz, 1 H), 7.86 (dd, *J*= 12.1, 2.1 Hz, 5H), 7.50 (dd, *J* = 12.1, 2.1 Hz, 2H), 7.41-7.36 (m, 2H), 7.32-7.26 (m, 4H), 7.23 (dd, *J*= 12.0, 2.1 Hz, 10H), 7.20-7.09 (m, 8H), 7.04 (m, 1 H), 6.96-6.88 (m, 16H), 6.83 (tt, *J* = 11.1, 2.0 Hz, 2H), 6.34 (dd, *J* = 12.6, 2.0 Hz, 2H), 6.29-6.27 (m, 1 H), 2.43 (s, 3H), 2.18 (s, 3H). $^{13}C$ NMR (150 MHz, $CD_2Cl_2$): δ = 291.5, 156.5, 155.3, 151.7, 151.0, 150.5, 150.1, 149.3, 148.5, 147.4, 143.5, 139.5, 139.3, 136.3, 135.9, 135.7, 135.5, 135.4, 135.3, 131.3, 130.3, 129.4, 129.1, 128.7, 128.6, 128.4, 128.2, 127.7, 127.4, 127.2, 126.6, 126.1, 125.7, 125.0, 122.9, 122.2, 66.1, 15.5

**5. Darstellung von [Mo(≡CPh)($OSiPh_3$)$_3$(4,4'-Dimethoxy-2,2'-dipyridyl)] (24d)**

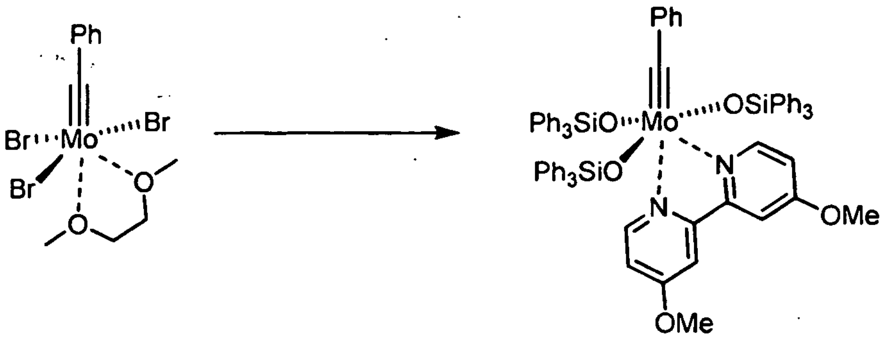

In einem trockenen und mit Argon gefluteten 100 mL *Schlenk*-Kolben wurde Mo(≡CPh)$Br_3$(dme) (46 mg, 89 μmol) in 0.5 mL Toluol gelöst und mit einer Lösung von $Ph_3SiOK$ (112 mg, 357 μmol) in 0.5 mL Toluol versetzt. Nach 1 h bei Raumtemperatur wurde filtriert und das Filtrat zu einer Lösung von 4,4'-Dimethoxy-2,2'-bipyridin (19 mg, 89 μmol) in 1 mL Toluol getropft. Nach 1 h bei Raumtemperatur wurde der Lösung 1 mL Diethylether zugesetzt und das Gemisch über Nacht zur Kristallisation stehen gelassen. Der Niederschlag wurde abfiltriert und ergab nach Trocknung im Hochvakuum Mo(≡CPh)($OSiPh_3$)$_3$(4,4'-dimethoxy-2,2'-bipyridyl) als violetten kristallinen Feststoff (78 mg, 71 %). Die Struktur von [Mo(≡CPh)($OSiPh_3$)$_3$(4,4'-Dimethoxy-2,2'-dipyridyl)] wurde durch Kristallstrukturanalyse bestätigt (Figur

11).

**6. Darstellung von [Mo(≡CPh)(OSiAr$_3$)$_3$·(bipy)] (Ar = 4-Methoxyphenyl) (24e)**

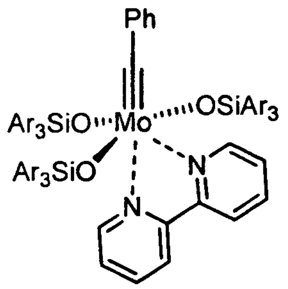

Ar = 4-Methoxyphenyl

Eine Lösung von [Mo(≡CPh)Br$_3$(dme)] (0.21 g, 0.40 mmol) und Ar$_3$SiOK (Ar = 4-Methoxyphenyl, 0.65 g, 1.6 mmol) in Toluol (10 mL) wurde 1 h bei Raumtemperatur unter Ar gerührt. Anschließend wurde der ausgefallene Feststoff unter Ar abfiltriert, das Filtrat mit einer Lösung von 2,2'-Bipyridin (0.13 g, 0.80 mmol) in Et$_2$O (10 mL) versetzt, und das Reaktionsgemisch 2 h bei Raumtemperatur gerührt. Anschließend wurden die Lösungsmittel im Vakuum abdestilliert und der Rückstand mit Et$_2$O (30 mL) versetzt. Die gebildete rote Lösung wurde von ungelösten Bestandteilen abfiltriert, das Filtrat eingeengt und der Rückstand aus CH$_2$Cl$_2$/Pentan umkristallisiert. Man erhält das Produkt als dunkelroten Feststoff (0.21 g, 36%). $^1$H NMR (400 MHz, CD$_2$Cl$_2$): $\delta$ = 9.00 (ddd, *J* = 5.0, 1.7, 0.8 Hz, 1 H), 8.69 (ddd, *J* = 5.4, 1.6, 0.6 Hz, 1H), 7.86 (ddd, 7.8, 7.8, 1.8 Hz, 1 H), 7.72 (d, *J* = 8.6 Hz, 6H), 7.67 (d, *J* = 8.3 Hz, 1H), 7.44-7.29 (m, 3H), 7.11 (d, *J* = 8.6 Hz, 12H), 6.94 (dd, *J* = 7.4, 7.4 Hz, 2H), 6.84 (tt, *J* = 7.4, 1.3 Hz, 1 H), 6.62 (ddd, *J* = 7.2, 5.4, 1.5 Hz, 1 H), 6.47 (d, *J* = 8.6 Hz, 6H), 6.45 (d, *J* = 8.6 Hz, 12H), 6.29 (dd, 7.4, 1.3 Hz, 2H), 3.68 (s, 18H), 3.62 (s, 9H); $^{13}$C NMR (100 MHz, CD$_2$Cl$_2$): $\delta$ = 291.0, 160.9, 160.6, 156.2, 152.0, 151.5, 148.0, 143.8, 138.5, 138.5, 137.9, 137.3, 131.5, 131.3, 131.0, 127.3, 126.6, 125.4, 125.2, 122.3, 122.3, 113.6, 113.3, 55.4, 55.3.

Die Struktur von von [Mo(≡CPh)(OSiAr$_3$)$_3$·(bipy)] (Ar = 4-Methoxyphenyl) wurde durch Kristallstrukturanalyse bestätigt (Figur 12).

**7. Alkin-Ringschlussmetathese unter Verwendung von [Mo(≡N)(OSiPh$_3$)$_3$(bipy)].**

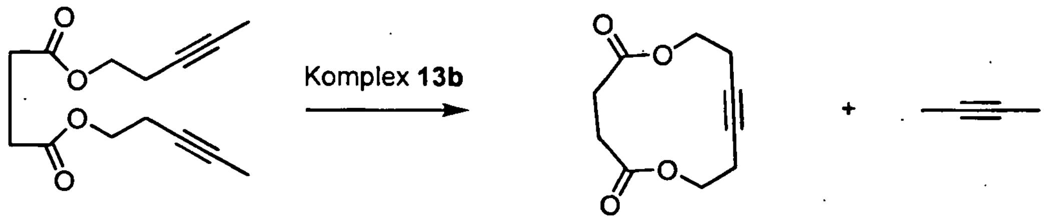


In einem trockenen und mit Argon gefluteten 100 ml *Schlenk*-Kolben wurden [Mo(≡N)(OSiPh$_3$)$_3$(bipy)] (109.2 mg, 0.10 mmol) und MnCl$_2$ (12.6 mg, 0.10 mmol) in trockenem Toluol (15 ml) suspendiert und das Gemisch auf 80 °C erhitzt. Nach 30 min wurden zusätzliches trockenes Toluol (35 ml) und Bernsteinsäuredipent-3-inylester (278.3 mg, 1.00 mmol) zugegeben, das Reaktionsgemisch 24 h bei 80 °C gerührt und nach dem Erkalten über ein kurzes Kieselgel Polster filtriert. Das Filtrat wurde eingeengt und der Rückstand säulenchromatographisch gereinigt (Hexan/EtOAc 4:1). Das 1,6-Dioxazyklododec-9-in-2,5-dion wurde als farbloses Öl isoliert (Ausbeute: 81%). $^1$H NMR (400 MHz, CD$_2$Cl$_2$): $\delta$ = 4.21-4.14 (m, 4H), 2.61 (s, 4H), 2.44-2.37 (m, 4H); $^{13}$C NMR (100 MHz, CD$_2$Cl$_2$): $\delta$ = 171.9 (2xC), 79.0 (2xC), 61.6 (2xCH$_2$), 30.2 (2xCH$_2$), 19.7 (2xCH$_2$); IR (Film): $\tilde{\nu}$ = 2965, 2915, 2840, 1729, 1458, 1421, 1383, 1353, 1336, 1267, 1251, 1158, 1053, 1030, 1000, 952, 837 cm$^{-1}$; MS (EI) *m/z* (%): 166 (1), 101 (14), 78 (100), 66 (59), 65 (16), 55 (7), 40 (12), 28 (7); HRMS (ESI): *m/z:* berechnet für C$_{10}$H$_{12}$O$_4$ + Na: 219.0628; gefunden: 219.0627.

**8. Alkin-Ringschlussmetathese unter Verwendung von [Mo(≡CPh)($OSiPh_3$)$_3$(bipy)] (24b) und $MnCl_2$.**

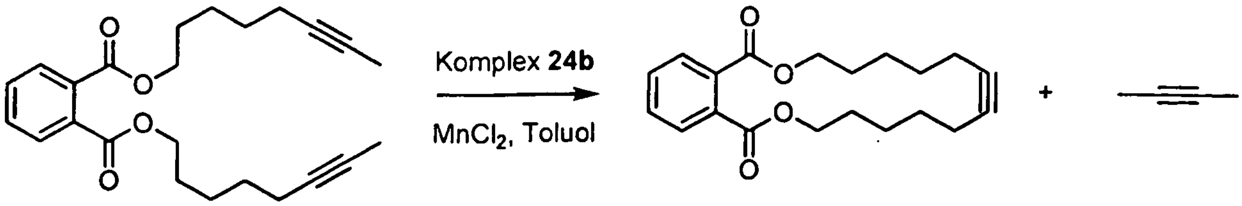


In einem trockenen und mit Argon gefluteten 25 mL *Schlenk*-Kolben wurden Komplex **24b** (43 mg, 37 μmol, 5 mol%) und $MnCl_2$ (4.6 mg, 37 μmol) in 1 mL Toluol gelöst. Anschließend wurde das Gemisch für 30 min auf 80 °C erhitzt und wieder auf Raumtemperatur abgekühlt. Die gebildete Katalysatorlösung wurde zu einer Suspension aus Bis(oct-6-inyl)phthalat (282 mg, 737 μmol) und Molsieb (737 mg, 5 Å, Pulver) in 36 mL Toluol gegeben und das Gemisch 24 h bei Raumtemperatur gerührt. Nach dem Erkalten wurde über ein kurzes Kieselgel-Polster filtriert, das Filtrat eingeengt und der Rückstand säulenchomatographisch gereinigt (Hexan/EtOAc 9:1). Das Produkt wurde als farbloses Öl isoliert (218 mg, 90%). $^1$H NMR (400 MHz, $CD_2Cl_2$): δ = 7.72 (dd, *J* = 5.7, 3.3 Hz, 2H), 7.55 (dd, *J* = 5.7, 3.3 Hz, 2H), 4.32 (t, *J* = 6.0 Hz, 4H), 2.21-2.14 (m, 4H), 1.82-1.73 (m, 4H), 1.60-1.47 (m, 8H); $^{13}$C NMR (100 MHz, $CD_2Cl_2$): δ = 168.0 (2xC), 132.8 (2xC), 131.3 (2xCH), 129.1 (2xCH), 80.9 (2xC), 66.7 (2x$CH_2$), 28.7 (2x$CH_2$), 28.5 (2x$CH_2$), 26.3 (2x$CH_2$), 18.9 (2x$CH_2$); IR-(Film): $\tilde{v}$ = 2928, 2859, 1720, 1600, 1579, 1488, 1460, 1447, 1433, 1385, 1269, 1122, 1070, 1039, 957, 734, 703 cm$^{-1}$; MS (EI) *m/z* (%): 328 [M$^+$] (8), 180 (9), 162 (30), 149 (100), 133 (17), 122 (18), 121 (26), 119 (14), 108 (43), 107 (18), 105 (15), 95 (11), 94 (24), 93 (44), 91 (29), 81 (19), 80 (28), 79 (34), 77 (13), 67 (19), 55 (13); HRMS (ESI): *m/z:* ber. für $C_{20}H_{24}O_4$ + Na: 351.1567; gefunden: 351.1567; Elementaranalyse (%) ber. für $C_{20}H_{24}O_4$: C 73.15, H 7.37; gefunden: C 73.26, H 7.28.

Für weitere Beispiele von Verbindungen die auf analoge Weise dargestellt wurden, siehe Tabelle 2.

**9. Alkinmetathese durch Aktivierung von [Mo(≡N)($OSiPh_3$)$_3$(bipy)] mit Hilfe von $MnCl_2$ vor Zugabe des Substrats**

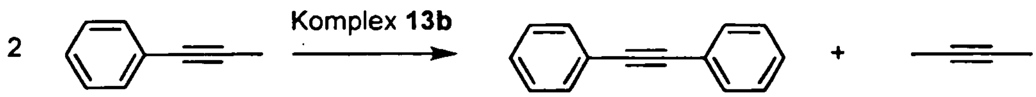


In einem trockenen und mit Argon gefluteten 25 ml *Schlenk*-Kolben wurden [Mo(≡N)($OSiPh_3$)$_3$(bipy)] (109.2 mg, 0.10 mmol), $MnCl_2$ (12.6 mg, 0.10 mmol) und Molsieb 5 A (1.0 g) in trockenem Toluol (5 ml) suspendiert und das Gemisch auf 80 °C erhitzt. Nach 30 min wurde 1-Phenyl-1-propin (116.2 mg, 1.00 mmol) zugegeben, das Reaktionsgemisch 3 h bei 80 °C gerührt und nach dem Erkalten über ein kurzes Kieselgel Polster filtriert. Das Filtrat wurde eingeengt und der Rückstand säulenchromatographisch gereinigt (Hexan). Es wurden 88.2 mg Tolan (99%) als weißer Feststoff isoliert, dessen spektroskopische und analytische Daten mit jenen einer kommerziellen Probe übereinstimmten. M. p. = 59 -61 °C.

**10. Alkinmetathese durch Aktivierung von [Mo(≡N)($OSiPh_3$)$_3$(bipy)] mit Hilfe von $MnCl_2$ in Gegenwart des Substrats**

In einem trockenen und mit Argon gefluteten 25 ml *Schlenk*-Kolben wurden [Mo(≡N)($OSiPh_3$)$_3$(bipy)] (109.2 mg, 0.10 mmol), $MnCl_2$ (12.6 mg, 0.10 mmol), Molsieb 5 A (1.0 g) und 1-Phenyl-1-propin (116.2 mg, 1.00 mmol) in trockenem Toluol (5 ml) suspendiert. Das Reaktionsgemisch wurde 3 h bei 80 °C gerührt und nach dem Erkalten über ein kurzes Kieselgel Polster filtriert. Das Filtrat wurde eingeengt und der Rückstand säulenchromatographisch gereinigt (Hexan). Es wurden 88.0 mg Tolan (98%) als weißer Festsoff isoliert, dessen spektroskopische und analytische Daten mit jenen einer kommerziellen Probe übereinstimmten. M. p. = 59 -61 °C.

Für weitere Beispiele von Verbindungen die auf analoge Weise dargestellt wurden, siehe Tabellen 1 und 2.

**11. Alkinmetathese ohne Zugabe von Molsieb durch Aktivierung von [Mo(≡N)(OSiPh$_3$)$_3$(bipy)] mit Hilfe von MnCl$_2$ in Gegenwart des Substrats.**

In einem trockenen und mit Argon gefluteten 25 ml *Schlenk*-Kolben wurden [Mo(≡N)(OSiPh$_3$)$_3$(bipy)] (109.2 mg, 0.10 mmol), MnCl$_2$ (12.6 mg, 0.10 mmol) und 1-Phenyl-1-propin (116.2 mg, 1.00 mmol) in trockenem Toluol (5 ml) suspendiert. Das Reaktionsgemisch wurde 3 h bei 80 °C gerührt und nach dem Erkalten über ein kurzes Kieselgel Polster filtriert. Das Filtrat wurde eingeengt und der Rückstand säulenchromatographisch gereinigt (Hexan). Es wurden 77.3 mg Tolan (87%) als weißer Feststoff isoliert, dessen spektroskopische und analytische Daten mit jenen einer kommerziellen Probe übereinstimmten. M. p. = 59 -61 °C.

**12. Alkinmetathese ohne Zugabe von Molsieb durch Aktivierung von [Mo(≡N)(OSiPh$_3$)$_3$(bipy)] mit Hilfe von CuCl$_2$ in Gegenwart des Substrats.**

In einem trockenen und mit Argon gefluteten 25 ml *Schlenk*-Kolben wurden [Mo(≡N)(OSiPh$_3$)$_3$(bipy)] (109.2 mg, 0.10 mmol), CuCl$_2$ (13.4 mg, 0.10 mmol) und 1-Phenyl-1-propin (116.2 mg, 1.00 mmol) in trockenem Toluol (5 ml) suspendiert. Das Reaktionsgemisch wurde 3 h bei 80 °C gerührt und nach dem Erkalten über ein kurzes Kieselgel Polster filtriert. Das Filtrat wurde eingeengt und der Rückstand säulenchromatographisch gereinigt (Hexan). Es wurden 76.1 mg Tolan (85%) als weißer Feststoff isoliert, dessen spektroskopische und analytische Daten mit jenen einer kommerziellen Probe übereinstimmten. M. p. = 59 -61 °C.

**13. Alkinmetathese durch Aktivierung von [Mo(≡CPh)(OSiPh$_3$)$_3$(bipy)] (24b) mit Hilfe von MnCl$_2$**

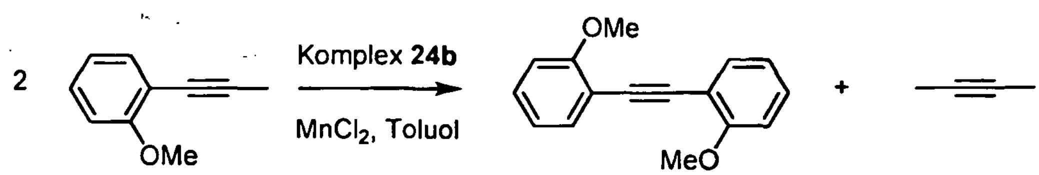


In einem trockenen und mit Argon gefluteten 25 mL *Schlenk*-Kolben wurden Komplex **24b** (58 mg, 0.05 mmol, 5 mol%) und MnCl$_2$ (6.3 mg, 0.05 mmol) in 1 mL Toluol gelöst. Anschließend wurde das Gemisch für 30 min auf 80 °C erhitzt und wieder auf Raumtemperatur abgekühlt. Die Katalysator-Lösung wurde in eine Suspension von 1-Methoxy-2-(prop-1-inyl)benzol (325 mg, 1 mmol) und Molsieb (1 g, 5 Å, Pulver) in 4 mL Toluol überführt und das Reaktionsgemisch für 2 h bei Raumtemperatur gerührt. Schließlich wurde über ein kurzes Kieselgelpolster (2 cm) filtriert, das Filtrat eingeengt, und der Rückstand säulenchromatographisch gereinigt (SiO$_2$, Hexan/EtOAc, 20:1). Man erhält 1,2-Bis(2-methoxyphenyl)ethin (116 mg, 97%) als farblosen Feststoff. M. p. = 126-127°C; $^1$H NMR (400 MHz, CD$_2$Cl$_2$): $\delta$ = 7.49 (ddd, *J* = 7.3, 1.6, 0.5 Hz, 2H), 7.33 (ddd, *J* = 7.9, 7.7, 1.9 Hz, 2H), 6.95 (td *J* = 7.9, 1.0 Hz, 4H), 3.92 (s, 6H); $^{13}$C NMR (100 MHz, CD$_2$Cl$_2$): $\delta$ = 160.3 (2xC), 133.7 (2xCH), 130.1 (2xCH), 120.8 (2xCH), 113.0 (2xC), 111.3 (2xCH), 90.1 (2xC), 56.2 (2xCH$_3$); IR (Film): $\tilde{v}$ = 3105, 3033, 2998, 2963, 2937, 2833, 1945, 1903, 1863, 1598, 1574, 1498, 1464, 1456, 1432, 1274, 1241, 1184, 1162, 1115, 1047, 1020, 937, 750 cm$^{-1}$; MS (EI) *m*/*z* (%): 238 [M$^+$] (100), 237 (32), 223 (23), 221 (15), 207 (10), 195 (5), 178 (8), 165 (14), 152 (9), 131 (19), 111 (6), 97 (3), 89 (3); HRMS (ESI): *m*/*z*: ber. für C$_{16}$H$_{14}$O$_2$ + Na: 261.0886; gefunden: 261.0884.

Für weitere Beispiele von Verbindungen die auf analoge Weise dargestellt wurden, siehe Tabellen 1 und 2.

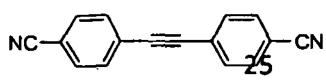

25

Oranger Feststoff. M. p. = 252-255°C; $^1$H NMR (CDCl$_3$, 400 MHz): $\delta$ = 8.27 (d, *J* = 8.8 Hz, 4H), 7.73 (d, *J* = 8.8 Hz, 4H); $^{13}$C NMR (CDCl$_3$, 100 MHz): $\delta$ = 147.3, 134.6, 132.3, 128.5, 127.6, 123.4 91.6; IR (Film, cm$^{-1}$): 3058, 2920, 2225, 1928, 1675, 1606, 1502, 1408, 1273, 1182, 1022, 841, 553; MS (EI) *m*/*z* (%): 228 [M$^+$] (100), 201 (8), 175 (4), 151 (3), 137 (1), 100 (3), 87 (4), 74 (3), 63 (1). Die analytischen und spektroskopischen Daten entsprechen jenen der Literatur (Pschirer, N. G.; Bunz, U. H. F. Tetrahedron Lett. 1999, 40, 2481).

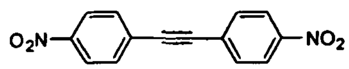

Gelber Feststoff. M. p.= 205-207°C; $^{1}H$ NMR ($CDCl_3$, 400 MHz): $\delta$ = 7.68 (d, *J* = 8.5 Hz, 4H), 7.64 (d, *J* = 8.5 Hz, 4H); $^{13}C$ NMR ($CDCl_3$, 100 MHz): $\delta$ = 132.4, 132.3, 127.2, 118.4, 112.6, 91.7; IR (Film, $cm^{-1}$): 3058, 2920, 2254, 2225, 1928, 1675, 1606, 1502, 1408, 1273, 1182, 1022, 841, 553; MS (EI) *m*/*z* (%): 268 [$M^+$] (100), 238 (23), 222 (7), 210 (4), 192 (6), 176 (42), 163 (19), 150 (15), 137 (4), 126 (5), 111 (2), 99 (4), 75 (7), 63 (4), 51 (3); HRMS (ESI): *m*/*z*: berechnet für $C_{14}H_8N_2O_4$: 268.0484; gefunden: 268.0485. Die analytischen und spektroskopischen Daten entsprechen jenen der Literatur (Akiyama, S.; Tajima, K.; Nakatsuji, S.; Nakashima, K.; Abiru, K.; Watanabe, M. Bull. Chem. Soc. Jpn. 1995, 68, 2043).

**14. Alkin-Kreuzmetathese unter Verwendung von [Mo(≡CPh)($OSiPh_3$)$_3$(bipy)] (24b) und $MnCl_2$**

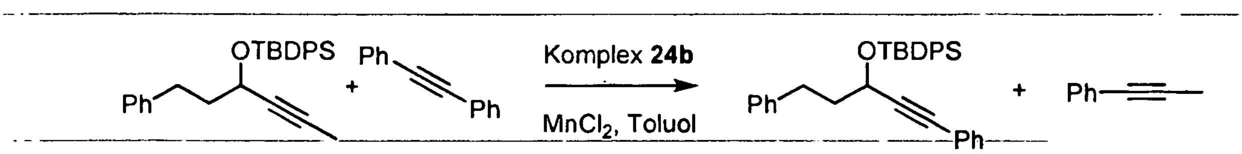


In einem trockenen und mit Argon gefluteten 25 mL *Schlenk*-Kolben wurden Komplex **24b** (58 mg, 0.05 mmol, 5 mol%) und $MnCl_2$ (6.3 mg, 0.05 mmol) in Toluol (1 mL) suspendiert und anschließend für 30 min auf 80°C erhitzt. Nach Abkühlen auf Raumtemperatur wurde die entstandene Suspension zu einer Suspension aus *tert*-Butyldiphenyl-(1-phenylhex-4-in-3-oxy)silan (412 mg, 1.00 mmol), Tolan (446 mg, 2.50 mmol), und Molsieb (1 g, 5 A, Pulver) in Toluol (4 mL) gegeben und das entstandene Gemisch 24 h bei Raumtemperatur gerührt. Zur Aufarbeitung wird durch ein kurzes Kieselgelpolster filtriert, das Filtrat eingeengt, und der Rückstand säulenchromatographisch (Hexan) gereinigt. Man erhält *tert*-Butyl-(1,5-diphenylpent-1-in-3-oxy)-diphenylsilan als farblosen Feststoff (336mg, 71%). $^{1}H$ NMR (400 MHz, $CDCl_3$): $\delta$ (ppm) = 7.82-7.80 (m, 2H), 7.75-7.72 (m, 2H), 7.50-7.34 (m, 6H), 7.29-7.15 (m, 10H), 4.63 (t, *J* = 6.1 Hz, 1 H), 2.87-2.82 (m, 2H), 2.13-2.07 (m, 2H), 1.13 (s, 9H); $^{13}C$ NMR (100 MHz, $CDCl_3$): $\delta$(ppm) = 141.8, 136.1, 135.9, 133.8, 133.7, 131.5, 129.7, 129.5, 128.4, 128,3, 128.1, 128.0, 127.6, 127.4, 125.8, 123.0, 90.4, 85.3, 63.8, 40.1, 31.3, 27.0, 19.4; IR (Film): $\tilde{v}$ = 3070, 3026, 2931, 2892, 2857, 1599, 1589, 1489, 1472, 1454, 1443, 1390, 1361, 1341, 1258, 1170, 1156, 1105, 1084, 1050, 1029, 1007, 998, 979, 938, 908, 843, 821, 798, 755, 734, 698, 689 $cm^{-1}$; MS (EI) *m*/*z* (%): 474 [$M^+$] (3), 417 (23), 369 (2), 339 (100), 313 (4), 283 (62), 223 (4), 199 (20), 181 (5), 135 (3), 91 (12), 77 (3); HRMS (ESI): *m*/*z*: berechnet für $C_{33}H_{34}OSi$: 474.2379; gefunden: 474.2379.

## Patentansprüche

1. Metallorganische Verbindungen der allgemeinen Formel **I**,

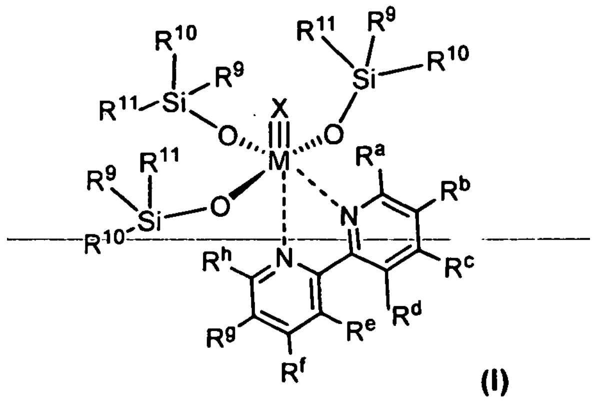

**(I)**

worin

M = Mo, W,
die Substituenten $R^a$, $R^b$, $R^c$, $R^d$, $R^e$, $R^f$, $R^g$, $R^h$ gleich oder verschieden sein können und unabhängig voneinander ausgewählt werden können aus: H, $C_1$-$C_{12}$-Alkyl, $C_3$-$C_{12}$-Cyclöalkyl, 6- bis 18-gliedriges Aryl, 5- bis 10-gliedriges Heteroaryl, $C_1$-$C_{12}$-Alkyloxy, $C_3$-$C_{12}$-Cycloalkyloxy, $C_6$-$C_{20}$-Aryloxy, Di-$C_1$-$C_4$-alkylamino, Halogen, Nitro, Cyano, Trifluormethyl, -$COOR^{12}$, worin $R^{12}$ = $C_1$-$C_{12}$ Alkyl, $C_3$-$C_{12}$ Cycloalkyl, 6- bis 18-gliedriges Aryl, 5- bis 10-gliedriges Heteroaryl sein kann,
und die Substituenten $R^a$ und $R^b$ oder $R^b$ und $R^c$ oder $R^c$ und $R^d$ sowie $R^h$ und $R^g$ oder $R^g$ und $R^f$ oder $R^f$ und $R^e$ auch unter Bildung eines gesättigten, ungesättigten oder aromatischen Ringes miteinander verbunden sein können,
und/oder die Reste $R^d$ und $R^e$ unter Bildung eines 5- bis 8-gliedrigen gesättigten, ungesättigten oder aromatischen Ringes miteinander verbunden sein können,
die Substituenten $R^9$, $R^{10}$, $R^{11}$ gleich oder verschieden sein können und unabhängig voneinander ausgewählt werden können aus folgenden Gruppen: $C_1$-$C_{12}$-Alkyl, $C_3$-$C_{12}$-Cycloalkyl, 6- bis 18-gliedriges Aryl, 5- bis 10-gliedriges Heteroaryl, mit der Maßgabe, dass mindestens einer dieser Substituenten ein 6- bis 18-gliedriger Aryl- oder 5- bis 10-gliedriger Heteroaryl Rest ist, worin dieser Aryl- oder Heteroaryl Rest am Silicium seinerseits bis zu fünf identische oder verschiedene Substituenten aufweisen kann, aus der Liste: H, $C_1$-$C_{12}$-Alkyl, $C_1$-$C_{12}$-Alkoxy, Di-$C_1$-$C_4$-Alkylamino, $C_3$-$C_{12}$-Cycloalkyl, $C_3$-$C_{12}$ Cycloalkyloxy, 6- bis 18-gliedriges Aryl, 5- bis 10-gliedriges Heteroaryl, Halogen, und
X = N, $CR^{13}$ sein kann, worin $R^{13}$ =$C_1$-$C_{12}$-Alkyl, $C_3$-$C_{12}$-Cycloalkyl, 6- bis 18-gliedriges Aryl, 5- bis 10-gliedriges Heteroaryl sein kann.

2. Metallorganische Verbindungen nach Anspruch 1, **dadurch gekennzeichnet, dass** die Reste $R^d$ und $R^e$ unter Bildung eines 5 bis 8 gliedrigen gesättigten, ungesättigten oder aromatischen Ringes miteinander verbunden sind und somit eine Brücke bilden, die $CR^{41}R^{42}$, $CR^{43}$=$CR^{44}$, $CR^{45}R^{46}$-$CR^{47}R^{48}$, $CR^{49}R^{50}$-$CR^{51}R^{52}$-$CR^{53}R^{54}$, $CR^{55}R^{56}$=$C^{57}R^{58}$-$CR^{59}R^{60}$, $CR^{61}R^{62}$-$CR^{63}R^{65}$-$CR^{65}R^{66}$-$CR^{67}CR^{68}$, $CR^{69}$=$CR^{70}$-$CR^{71}R^{72}$-$CR^{73}CR^{74}$ oder $CR^{75}R^{76}$-$CR^{77}$=$CR^{78}$-$CR^{79}CR^{80}$ sein kann, in welchen $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$, $R^{75}$, $R^{76}$, $R^{77}$, $R^{78}$, $R^{79}$ und $R^{80}$ jeweils unabhängig voneinander ausgewählt werden können und die gleiche Bedeutung haben können wie $R^a$ in Anspruch 1

3. Metallorganische Verbindungen nach Ansprüchen 1 oder 2, die durch die allgemeine Formel 11 dargestellt sind

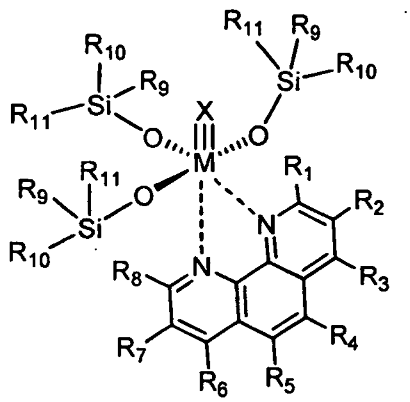

11

worin
die Substituenten $R^1$, $R^2$, $R^3$, $R^4$, $R^5$, $R^6$, $R^7$, $R^8$ gleich oder verschieden sein können und unabhängig voneinander ausgewählt werden können aus: H, $C_1$-$C_{12}$-Alkyl, $C_3$-$C_{12}$-Cycloalkyl, 6- bis 18-gliedriges Aryl, 5- bis 10-gliedriges Heteroaryl, $C_1$-$C_{12}$-Alkyloxy, $C_3$-$C_{12}$-Cycloalkyloxy, $C_6$-$C_{20}$-Aryloxy, Di-$C_1$-$C_4$-alkylamino, Halogen, Nitro, und
M, X, $R^9$, $R^{10}$ und $R^{11}$ wie in Anspruch 1 definiert sind.

4. Metallorganische Verbindungen nach Ansprüchen 2 oder 3, **dadurch gekennzeichnet, dass** der Phenanthrolin Ligand in Formel **11** ausgewählt ist aus: 1,10-Phenanthrolin, 4-Methyl-1,10-phenanthrolin, 5-Methyl-1,10-phenanthrolin, 2,9-Dimethyl[1,10]phenanthrolin, 5,6-Dimethyl-1,10-phenanthrolin, 5-Chlor[1,10]phenanthrolin, 4,7-Dichloro-1,10-phenanthrolin, 4,7-Dichlor-1,10-phenanthrolin, 3,4,7,8-Tetramethyl-1,10-phenanthrolin, 4,7-Diphenyl[1,10]phenanthrolin, 2,9-Dimethyl-4,7-diphenyl[1,10]pherianthrolin, 5-Nitro-1,10-phenanthrolin, 4,7-Dimethoxy-1,10-phenanthrolin.

**5.** Metallorganische Verbindungen nach Anspruch 1, die durch die allgemeine Formel 11a dargestellt sind

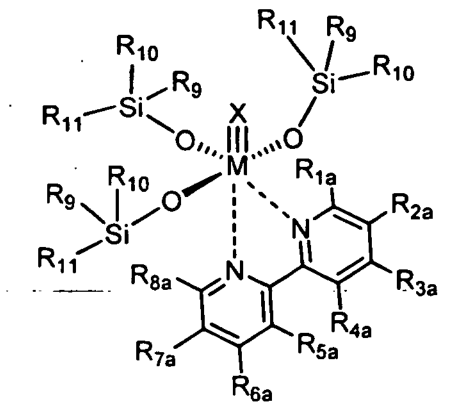

**11a**

worin

$R^{1a}$, $R^{2a}$, $R^{3a}$, $R^{4a}$, $R^{5a}$, $R^{6a}$, $R^{7a}$, $R^{8a}$ gleich oder verschieden sein können und unabhängig voneinander ausgewählt werden können aus: H, $C_1$-$C_{12}$-Alkyl, $C_3$-$C_{12}$-Cycloalkyl, 6- bis 18-gliedriges Aryl, 5- bis 10-gliedriges Heteroaryl, $C_1$-$C_{12}$-Alkyloxy, $C_3$-$C_{12}$-Cycloalkyloxy, $C_6$-$C_{20}$-Aryloxy, Di-$C_1$-$C_4$-alkylamino, Halogen, Nitro, und M, X, $R^9$, $R^{10}$ und $R^{11}$ wie in Anspruch 1 definiert sind.

**6.** Metallorganische Verbindungen nach Ansprüchen 1 oder 5, **dadurch gekennzeichnet, dass** der Bipyridyl Ligand ausgewählt ist aus: 2,2'-Bipyridin, 5,5'-Dimethyl-2,2'-dipyridyl, 4,4'-Dimethyl-2,2'-dipyridyl, 6,6'-Dimethyl-2,2'-dipyridyl, 4-4'-Dimethoxy-2-2'-bipyridin, 2,2'-Bichinolin, 4,4'-Di-tert-butyl-2,2'-dipyridyl, 2,2'-Bipyridinyl-4,4'-dicarbonsäuredimethylester, 4,4'-Diphenyl-2,2'-dipyridyl, 6,6'-Dibromo-2,2'-dipyridyl, 4,4'-Dinonyl-2,2'-dipyridyl, 2,2'-Bichinolinyl-4,4'-dicarbonsäuredibutylester, 2,2'-Bichinolinyl-4,4'-dicarbonsäurediheptylester, 6-Methyl-2,2'-dipyridyl, 2-(2-Pyridinyl)chinolin, 2-Pyridin-2-yl-4-pyrrolidin-1-yl-chinolin, 4-Piperidin-1-yl-2-pyridin-2-ylchinolin, 4-Morpholin-4-yl-2-pyridin-2-yl-chinolin.

**7.** Metallorganische Verbindungen nach einem der Ansprüche 1 bis 6, **dadurch gekennzeichnet, dass** $R^9$, $R^{10}$ und $R^{11}$ jeweils für einen Phenylrest stehen.

**8.** Metallorganische Verbindungen nach Ansprüchen 1, 2, 3, 4 oder 7, die durch die allgemeine Formel **12** dargestellt sind

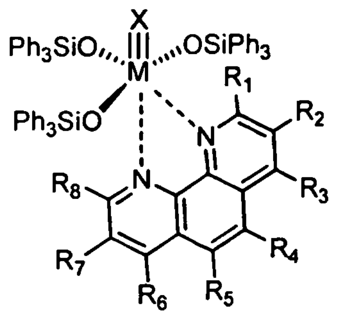

**12**

worin

M = Mo, W
X = N, $CR^{13}$, wobei
$R^{13}$ Methyl, Ethyl, Propyl, Butyl, iso-Propyl, tert-Butyl, Trimethylsilylmethyl, Benzyl, Furyl, Phenyl sein kann,

wobei Phenyl seinerseits bis zu fünf identische oder verschiedene Reste aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl tragen kann,

$R^1$, $R^2$, $R^3$, $R^4$, $R^5$, $R^6$, $R^7$, $R^8$ frei und unabhängig voneinander gewählt werden können aus: H, $C_1$-$C_4$ Alkyl, $C_3$-$C_6$ Cycloalkyl, Phenyl, Naphthyl, Methylphenyl, Dimethylphenyl, Trimethylphenyl, Methoxyphenyl, Dimethoxyphenyl, Fluorphenyl, Pentafluorphenyl, Trifluormethylphenyl, Dimethylaminophenyl, Furyl, Halogen, Nitro, Cyano, Trifluormethyl, -$COOR^{12}$, mit $R^{12}$ = $C_1$-$C_{12}$ Alkyl, $C_3$-$C_{12}$ Cycloalkyl, 6- bis 18-gliedriges Aryl, 5- bis 10-gliedriges Heteroaryl.

**9.** Metallorganische Verbindungen nach Ansprüchen 1, 5, 6, oder 7, die durch die allgemeine Formel 12a dargestellt sind

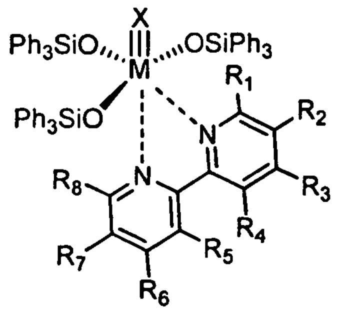

**12a**

worin

M = Mo, W

X = N, $CR^{13}$, wobei

$R^{13}$ Methyl, Ethyl, Propyl, Butyl, iso-Propyl, tert-Butyl, Trimethylsilylmethyl, Benzyl, Furyl, Phenyl sein kann, wobei Phenyl seinerseits bis zu fünf identische oder verschiedene Reste aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl tragen kann,

$R_1$, $R_2$, $R_3$, $R_4$, $R_5$, $R_6$, $R_7$, $R_8$ frei und unabhängig voneinander gewählt werden können aus: H, $C_1$-$C_4$ Alkyl, $C_3$-$C_6$ Cycloalkyl, Phenyl, Naphthyl, Methylphenyl, Dimethylphenyl, Trimethylphenyl, Methoxyphenyl, Dimethoxyphenyl, Fluorphenyl, Pentafluorphenyl, Trifluormethylphenyl, Dimethylaminophenyl, Furyl, Halogen, Nitro, -$COOR^{12}$, mit $R^{12}$ = $C_1$-$C_{12}$ Alkyl, $C_3$-$C_{12}$ Cycloalkyl, 6- bis 18-gliedriges Aryl, 5- bis 10-gliedriges Heteroaryl.

**10.** Metallorganische Verbindungen nach Ansprüchen 1 bis 9, die durch die Formeln 13a, 13b, 13c, 13d, 13e dargestellt sind

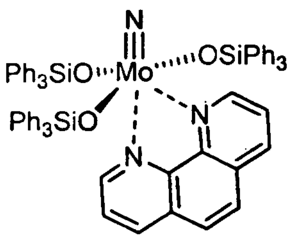

**13a**

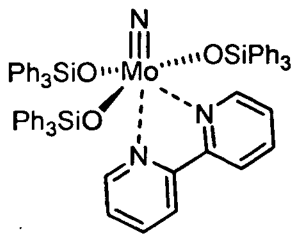

**13b**

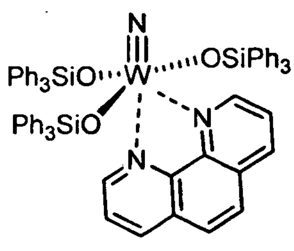

**13c**

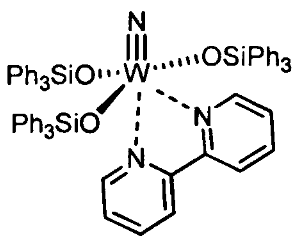

**13d**

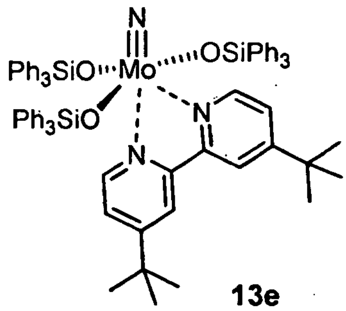

**13e**

**11.** Metallorganische Verbindungen nach Ansprüchen 1 bis 9, die durch die Formeln **14a, 14b, 15a, 15b, 16a, 16b** dargestellt sind

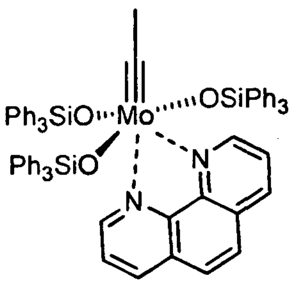

**14a**

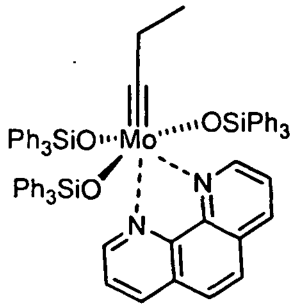

**15a**

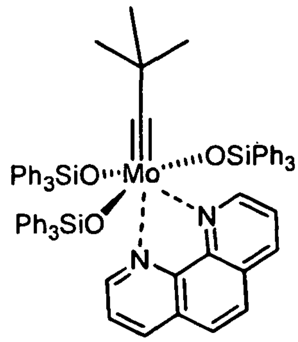

**16a**

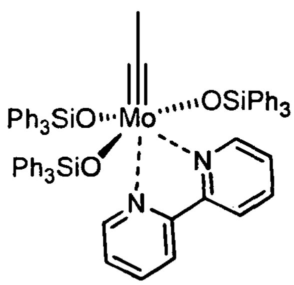

**14b**

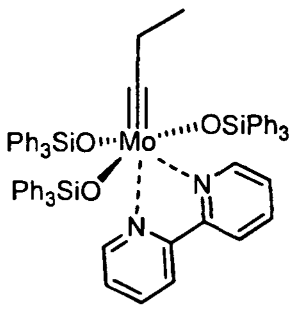

**15b**

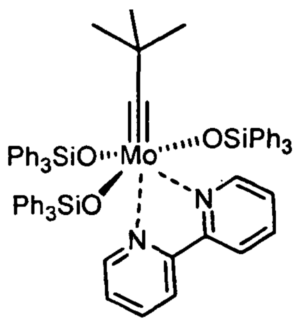

**16b**

**12.** Metallorganische Verbindungen nach Anspruch 1 bis 9, die durch die Formeln 17a oder 17b dargestellt sind

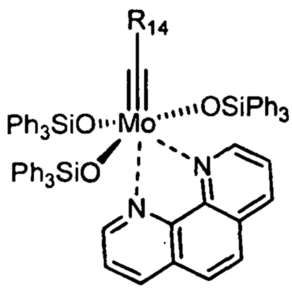

**17a**

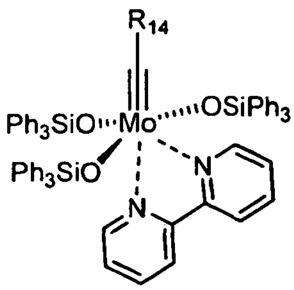

**17b**

wobei

$R^{14}$ ein Phenylring ist, der 1 bis 5 gleiche oder verschiedene Substituenten trägt,

ausgewählt aus der Liste: H, Methyl, Ethyl, Propyl, Butyl, tert-Butyl, iso-Propyl, Phenyl, Fluor, Chlor, Trifluormethyl, Methoxy, Ethoxy, Dimethylamino, Diethylamino, Trimethylsilyl.

**13.** Metallorganische Verbindungen nach Anspruch 12, die durch die Formeln **24a, 24b, 24c, 24d, 24e, 34a** dargestellt sind

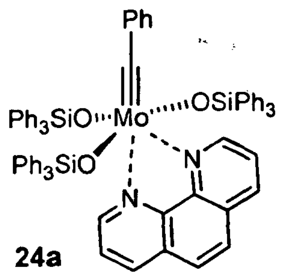

**24a**

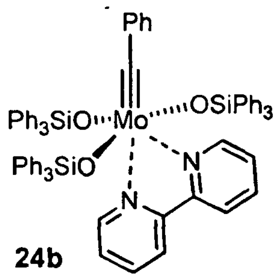

**24b**

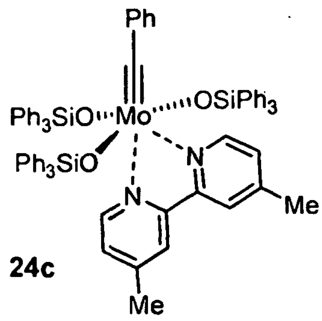

**24c**

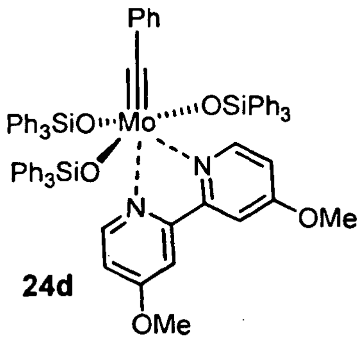

**24d**

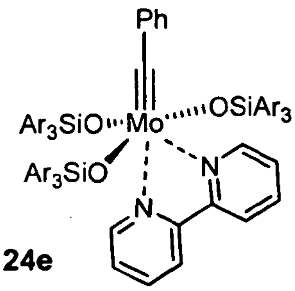

**24e**

Ar = 4-Methoxyphenyl

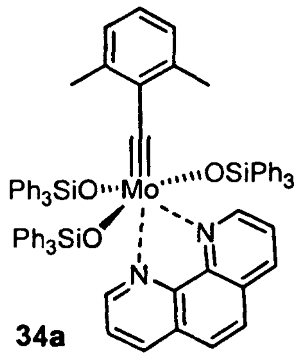

**34a**

**14.** Alkylidinkomplexe der allgemeinen Formel **25**

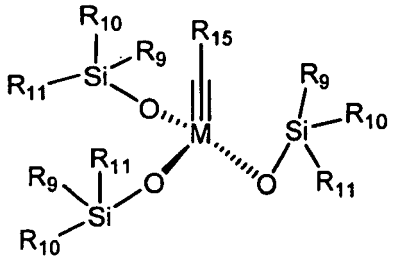

**25**

wobei

M = Mo, W,
die Substituenten $R^9$, $R^{10}$, $R^{11}$ gleich oder verschieden sein können und die in Anspruch 1 angegebene Bedeutung haben, und
$R^{15}$ = $C_1$-$C_{12}$ Alkyl, $C_3$-$C_{12}$ Cycloalkyl, 6- bis 18-gliedriges Aryl, 5- bis 10-gliedriges Heteroaryl sein kann.

**15.** Addukte von Alkylidinkomplexen der allgemeinen Formel **25** nach Anspruch 14 mit Lösungsmitteln, die ausgewählt werden können aus der Liste: Acetonitril, Benzonitril, Pivalonitril, Diethylether, Di-isopropylether, tert-Butylmethylether Diphenylether, Methoxybenzol, Tetrahydrofuran, Dioxan, Dimethoxyethan, sowie ihre Verwendung als Katalysatoren für die Alkinmetathese.

**16.** Alkylidinkomplexe nach Ansprüchen 14 und 15, **dadurch gekennzeichnet, dass** $R^9$, $R^{10}$ und $R^{11}$ jeweils für einen Phenylrest stehen.

**17.** Alkylidinkomplexe nach Ansprüchen 14 bis 16, **dadurch gekennzeichnet, dass** $R^{15}$ für Methyl, Ethyl, Propyl, Butyl, iso-Propyl, tert-Butyl, Trimethylsilylmethyl, Benzyl, (Dimethyl)(phenyl)methyl, Phenyl, Methylphenyl, Dimethylphenyl, Trimethylphenyl, Methoxyphenyl, Dimethoxyphenyl, Fluorphenyl, Pentafluorphenyl, Trifluormethylphenyl, oder Furyl steht.

**18.** Verwendung der Verbindungen mit der Formel 25 und deren Lösungsmittel-Addukte

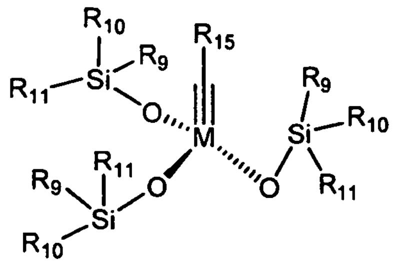

**25**

wobei

M = Mo, W,
die Substituenten $R^9$, $R^{10}$, $R^{11}$ gleich oder verschieden sein können und die in Anspruch 1 angegebene Bedeutung haben, und
$R^{15}$ = $C_1$-$C_{12}$ Alkyl, $C_3$-$C_{12}$ Cycloalkyl, 6- bis 18-gliedriges Aryl, 5- bis 10-gliedriges Heteroaryl sein kann,
als Katalysatoren für die Alkinmetathese.

**19.** Verfahren zur Herstellung der Verbindungen mit der Formel **I**

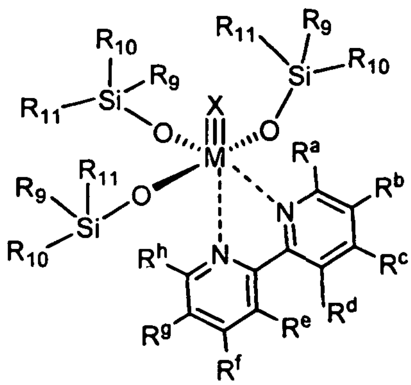

**(I)**

worin
M, $R^a$, $R^b$, $R^c$, $R^d$, $R^e$, $R^f$, $R^g$, $R^h$, $R^9$, $R^{10}$, $R^{11}$ und X wie in Anspruch 1 definiert sind,
in welchem eine Verbindung der allgemeinen Formel 18

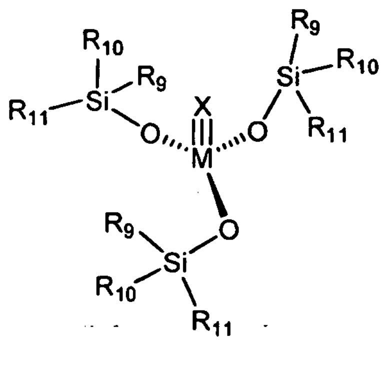

**18**

worin
M, $R_9$, $R_{10}$, $R_{11}$ und X wie in Anspruch 1 definiert sind,
mit einer Verbindung der allgemeinen Formel **19**

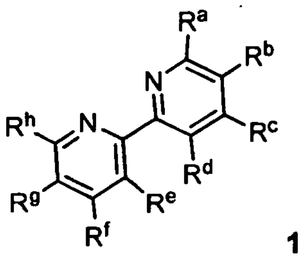

**19**

worin
$R^a$, $R^b$, $R^c$, $R^d$, $R^e$, $R^f$, $R^g$ und $R^h$ wie in Anspruch 1 definiert sind
umgesetzt wird.

**20.** Verfahren nach Anspruch 19, **dadurch gekennzeichnet, dass** die Verbindung der Formel **19** ausgewählt ist aus Derivaten des 1,10-Phenanthrölin oder 2,2'-Bipyridin wie sie in den Ansprüchen 4 und 6 definiert sind.

**21.** Verwendung der Verbindungen gemäß den Ansprüchen 1 bis 13 als Präkatalysatoren für die Durchführung von Alkinmetathese Reaktionen.

**22.** Verfahren zur Durchführung von Alkinmetathese Reaktionen, in welchem eine Verbindung der allgemeinen Formel **I**

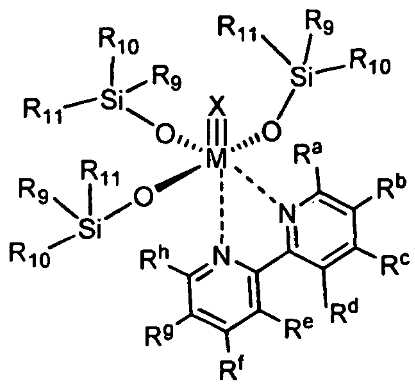

**(I)**

worin
M, $R^a$, $R^b$, $R^c$, $R^d$, $R^e$, $R^f$, $R^g$, $R^h$, $R^9$, $R^{10}$ und $R^{11}$ und X wie in Anspruch 1 definiert sind, in Gegenwart eines Metallsalzes umgesetzt wird.

**23.** Verfahren nach Anspruch 22, **dadurch gekennzeichnet, dass** das Metallsalz ausgewählt ist aus: $MnY_2$, $FeY_2$, $FeY_3$, $CoY_2$, $CuY_2$, $ZnY_2$, $MgY_2$, $NiY_2$, $PdY_2$, $PtY_2$, $RuY_2$, $RuY_3$, $EuY_3$,
worin Y = F, Cl, Br, I, acetylacetonat, sulfat, sulfonat, nitrat, acetat, trifluoracetat sein kann.

**24.** Verfahren zur Durchführung von Alkinmetathese Reaktionen laüt Ansprüche 22 in Gegenwart von Molsieben, **dadurch gekennzeichnet, dass** die Verwendung von Molsieben zu einer Steigerung der Reaktionsgeschwindigkeit und/oder der Ausbeute an Reaktionsprodukt führt im Vergleich zur gleichen Alkinmetathese Reaktion, die in Abwesenheit von Molekularsieben durchgeführt wird.

**25.** Verfahren nach Ansprüchen 21 bis 23, **dadurch gekennzeichnet, dass** die Alkinmetathese Reaktion in Gegenwart eines Molsiebs durchgeführt wird.

**26.** Verfahren nach Ansprüchen 21 bis 25, **dadurch gekennzeichnet, dass** die Alkinmetathese Reaktion in Gegenwart von Molsieb 4 Angström (MS 4 A) oder Molsieb 5 Angström (MS 5A) durchgeführt wird.

## Claims

1. Organometallic compounds of the general Formula I,

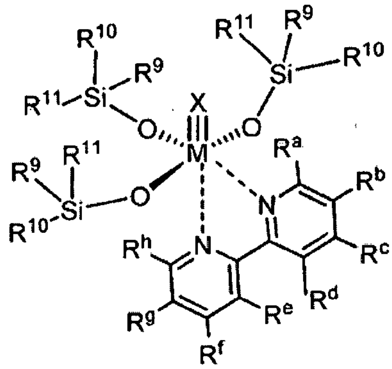

**(I)**

wherein
M = Mo, W,
the substituents $R^a$, $R^b$, $R^c$, $R^d$, $R^e$, $R^f$, $R^g$, $R^h$ may be the same or may be different from one another, and may be selected independently from one another from: H, $C_1$-$C_{12}$-alkyl, $C_3$-$C_{12}$-cycloalkyl, 6- to 18-membered aryl, 5- to 10-membered heteroaryl, $C_1$-$C_{12}$-alkyloxy, $C_3$-$C_{12}$-cycloalkyloxy, $C_6$-$C_{20}$-aryloxy, di-$C_1$-$C_4$-alkylamino, halogen, nitro, cyano, trifluoromethyl, -$COOR^{12}$, wherein $R^{12}$ may be $C_1$-$C_{12}$ alkyl, $C_3$-$C_{12}$ cycloalkyl, 6- to 18-membered aryl, 5- to 10-membered heteroaryl,
and the substituents $R^a$ and $R^b$ or $R^b$ and $R^c$ or $R^c$ and $R^d$ as well as $R^h$ and $R^g$ or $R^g$ and $R^f$ or $R^f$ and $R^e$ may also be connected to one another while forming a saturated, unsaturated or aromatic ring,
and/or the residues $R^d$ and $R^e$ may be connected to one another while forming a 5-to 8-membered saturated, unsaturated or aromatic ring,
the substituents $R^9$, $R^{10}$, $R^{11}$ may be the same or may be different from one another, and may be selected independently from one another from the following groups: $C_1$-$C_{12}$-alkyl, $C_3$-$C_{12}$-cycloalkyl, 6- to 18-membered aryl, 5- to 10-membered heteroaryl, under the proviso that at least one of these substituents is a 6- to 18-membered aryl residue or 5- to 10-membered heteroaryl residue, wherein said aryl residue or said heteroaryl residue may have for its part at the silicon up to five identical or different substituents selected from the list: H, $C_1$-$C_{12}$-alkyl, $C_1$-$C_{12}$-alkoxy, di-$C_1$-$C_4$-alkylamino, $C_3$-$C_{12}$-cycloalkyl, $C_3$-$C_{12}$-cycloalkyloxy, 6- to 18-membered aryl, 5- to 10-membered heteroaryl, halogen, and wherein X may be N, $CR^{13}$, wherein $R^{13}$ may be $C_1$-$C_{12}$-alkyl, $C_3$-$C_{12}$-cycloalkyl, 6- to 18-membered aryl, 5- to 10-membered heteroaryl.

2. Organometallic compound according to claim 1, **characterized in that** the residues $R^d$ and $R^e$ are connected to one another while forming a 5- to 8-membered saturated, unsaturated or aromatic ring and thus form a bridge, which may be $CR^{41}R^{42}$, $CR^{43}=CR^{44}$, $CR^{45}R^{46}$-$CR^{47}R^{48}$ $CR^{49}R^{50}$-$CR^{51}R^{52}$-$CR^{53}R^{54}$, $CR^{55}R^{56}=C^{57}R^{58}$-$CR^{59}R^{60}$, $CR^{61}R^{62}$-$CR^{63}R^{65}$-$CR^{65}R^{66}$-$CR^{67}CR^{68}$, $CR^{69}=CR^{70}$-$CR^{71}R^{72}$-$CR^{73}CR^{74}$ or $CR^{75}R^{76}$-$CR^{77}=CR^{78}$-$CR^{79}CR^{80}$, in which $R^{41}$ $R^{42}$, $R^{43}$ $R^{44}$ $R^{45}$ $R^{46}$ $R^{47}$ $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$, $R^{75}$, $R^{76}$, $R^{77}$, $R^{78}$, $R^{79}$, and $R^{80}$ may be selected independently from one another, respectively, and may have the same meaning as $R^a$ in claim 1.

3. Organometallic compound according to claims 1 or 2, which are represented by the general Formula **11**

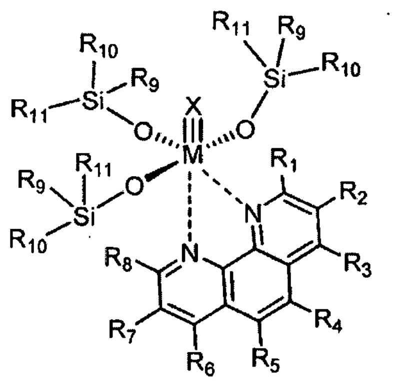

**11**

wherein

the substituents $R^1$, $R^2$, $R^3$, $R^4$, $R^5$, $R^6$, $R^7$, $R^8$ may be the same or may be different from one another, and may be selected independently from one another from: H, $C_1$-$C_{12}$-alkyl, $C_3$-$C_{12}$-cycloalkyl, 6- to 18-membered aryl, 5- to 10-membered heteroaryl, $C_1$-$C_{12}$-alkyloxy, $C_3$-$C_{12}$-cycloalkyloxy, $C_6$-$C_{20}$-aryloxy, di-$C_1$-$C_4$-alkylamino, halogen, nitro, and
M, X, $R^9$, $R^{10}$ and $R^{11}$ are defined as in claim 1.

**4.** Organometallic compounds according to claims 2 or 3, **characterized in that** the phenanthroline ligand in Formula **11** is selected from: 1,10-phenanthroline, 4-methyl-1,10-phenanthroline, 5-methyl-1,10-phenanthroline, 2,9-di-methyl[1,10]phenanthroline, 5,6-dimethyl-1,10-phenanthroline, 5-chloro[1,10]phenanthroline, 4,7-dichloro-1,10-phenanthroline, 4,7-dichloro-1,10-phenanthroline 3,4,7,8-tetramethyl-1,10-phenanthroline, 4,7-di-phenyl[1,10]phenanthroline, 2,9-dimethyl-4,7-diphenyl[1,10]phenanthroline, 5-nitro-1,10-phenanthroline, 4,7-dimethoxy-1,10-phenanthroline.

**5.** Organometallic compounds according to claim 1, which are represented by the general Formula **11a**

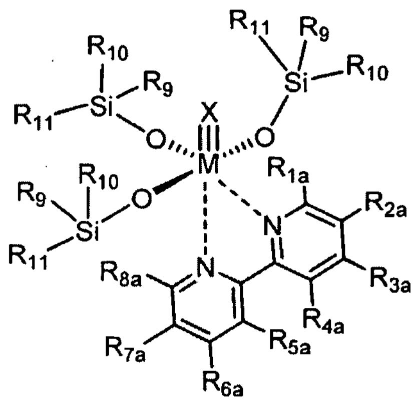

**11a**

wherein

$R^{1a}$, $R^{2a}$, $R^{3a}$, $R^{4a}$, $R^{5a}$, $R^{6a}$, $R^{7a}$, $R^{8a}$ may be the same or may be different from one another, and may be selected independently from one another from: H, $C_1$-$C_{12}$-alkyl, $C_3$-$C_{12}$-cycloalkyl, 6- to 18-membered aryl, 5- to 10-membered heteroaryl, $C_1$-$C_{12}$-alkyloxy, $C_3$-$C_{12}$-cycloalkyloxy, $C_6$-$C_{20}$-aryloxy, di-$C_1$-$C_4$-alkylamino, halogen, nitro, and
M, X, $R^9$, $R^{10}$, and $R^{11}$ are defined as in claim 1.

**6.** Organometallic compounds according to claims 1 or 5, **characterized in that** the bipyridyl ligand is selected from: 2,2'-bipyridine, 5,5'-dimethyl-2,2'-dipyridyl, 4,4'-dimethyl-2,2'-dipyridyl, 6,6'-dimethyl-2,2'-dipyridyl, 4-4'-dimethoxy-2-2'-bipyridine, 2,2'-biquinoline, 4,4'-di-tert-butyl-2,2'-dipyridyl, 2,2'-bipyridinyl-4,4'-dicarboxylic acid dimethylester, 4,4'-diphenyl-2,2'-dipyridyl, 6,6'-dibromo-2,2'-dipyridyl, 4,4'-dinonyl-2,2'-dipyridyl, 2,2'-biquinolinyl-4,4'-dicarboxylic acid dibutylester, 2,2'-biquinolinyl-4,4'-dicarboxylic acid diheptylester, 6-methyl-2,2'-dipyridyl, 2-(2-pyridinyl)quinoline, 2-pyridin-2-yl-4-pyrrolidin-1-yl-quinoline, 4-piperidin-1-yl-2-pyridin-2-yl-quinoline, 4-morpholin-4-yl-2-pyridin-2-yl-quinoline.

**7.** Organometallic compounds according to any one of claims 1 to 6, **characterized in that** $R^9$, $R^{10}$, and $R^{11}$ are a phenyl residue, respectively.

**8.** Organometallic compounds according to claims 1, 2, 3, 4, or 7, which are represented by the general Formula 12

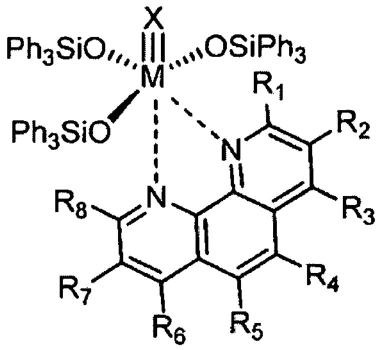

**12**

wherein

M = Mo, W
X = N, $CR^{13}$, wherein
$R^{13}$ may be methyl, ethyl, propyl, butyl, iso-propyl, tert-butyl, trimethylsilylmethyl, benzyl, furyl, phenyl, wherein phenyl may bear for its part up to five identical or different residues from the list: H, methyl, ethyl, propyl, butyl, tert-butyl, iso-propyl, phenyl, fluoro, chloro, trifluoromethyl, methoxy, ethoxy, dimethylamino, diethylamino, trimethylsilyl,
$R^1$, $R^2$, $R^3$, $R^4$, $R^5$, $R^6$, $R^7$, $R^8$ may be selected freely and independently from one another from: H, $C_1$-$C_4$ alkyl, $C_3$-$C_6$ cycloalkyl, phenyl, naphthyl, methylphenyl, dimethylphenyl, trimethylphenyl, methoxyphenyl, dimethoxyphenyl, fluorophenyl, pentafluorophenyl, trifluoromethylphenyl, dimethylaminophenyl, furyl, halogen, nitro, cyano, trifluoromethyl, $-COOR^{12}$, wherein $R^{12}$ = $C_1$-$C_{12}$ alkyl, $C_3$-$C_{12}$ cycloalkyl, 6- to 18-membered aryl, 5- to 10-membered heteroaryl.

**9.** Organometallic compounds according to claims 1, 5, 6, or 7, which are represented by the general Formula **12a**

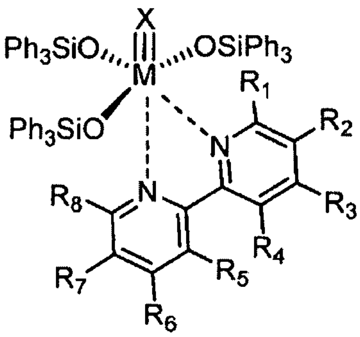

**12a**

wherein

M = Mo, W
X = N, $CR^{13}$, wherein
$R^{13}$ may be methyl, ethyl, propyl, butyl, iso-propyl, tert-butyl, trimethylsilylmethyl, benzyl, furyl, phenyl, wherein phenyl may bear for its part up to five identical or different residues from the list: H, methyl, ethyl, propyl, butyl, tert-butyl, iso-propyl, phenyl, fluoro, chloro, trifluoromethyl, methoxy, ethoxy, dimethylamino, diethylamino, trimethylsilyl,
$R_1$, $R_2$, $R_3$, $R_4$, $R_5$, $R_6$, $R_7$, $R_8$ may be selected freely and independently from one another from: H, $C_1$-$C_4$ alkyl,

$C_3$-$C_6$ cycloalkyl, phenyl, naphthyl, methylphenyl, dimethylphenyl, trimethylphenyl, methoxyphenyl, dimethoxyphenyl, fluorophenyl, pentafluorophenyl, trifluoromethylphenyl, dimethylaminophenyl, furyl, halogen, nitro, -COOR$^{12}$, wherein $R^{12}$ = $C_1$-$C_{12}$ alkyl, $C_3$-$C_{12}$ cycloalkyl, 6- to 18-membered aryl, 5- to 10-membered heteroaryl.

**10.** Organometallic compounds according to claims 1 to 9, which are represented by the Formulas **13a, 13b, 13c, 13d, 13e**

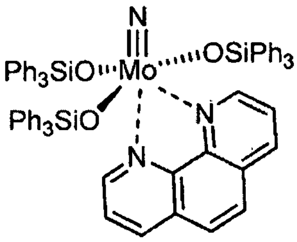

**13a**

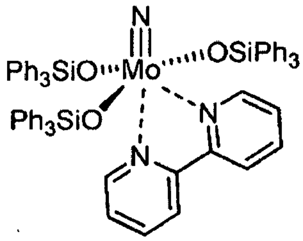

**13b**

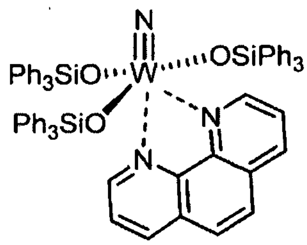

**13c**

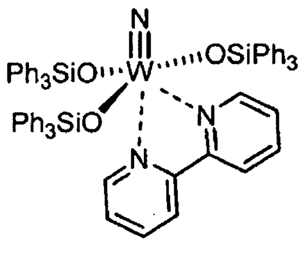

**13d**

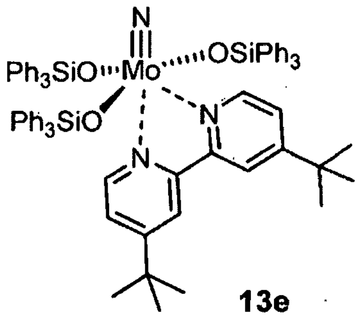

**13e**

**11.** Organometallic compounds according to claims 1 to 9, which are represented by the Formulas **14a, 14b, 15a, 15b, 16a, 16b**

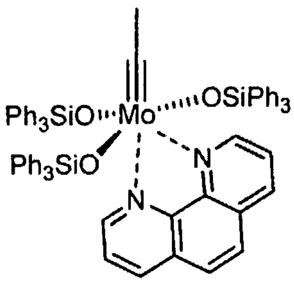

**14a**

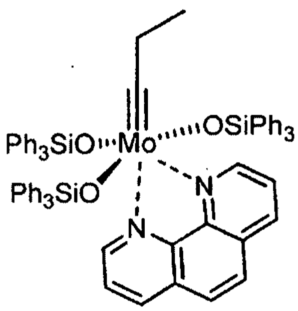

**15a**

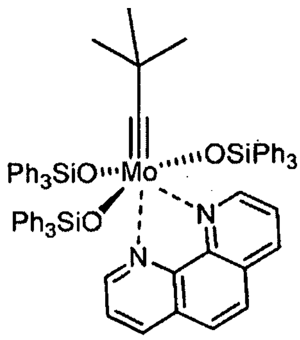

**16a**

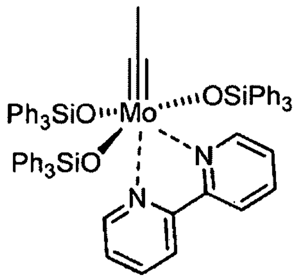

14b

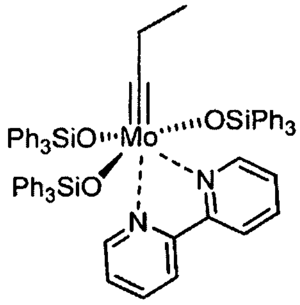

15b

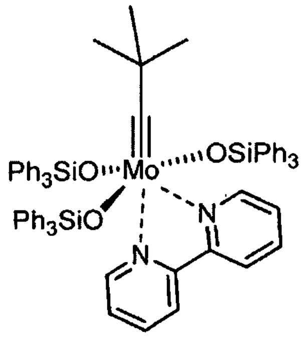

16b

**12.** Organometallic compounds according to claims 1 to 9, which are represented by the Formulas 17a or 17b

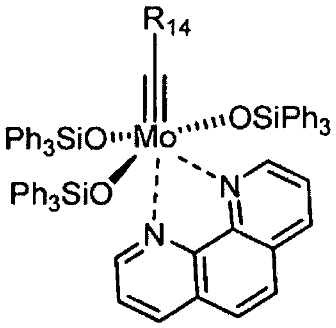

17a

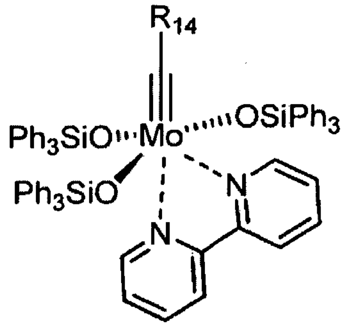

17b

wherein
$R^{14}$ is a phenyl ring, which bears from one to five identical or different substituents selected from the list: H, methyl, ethyl, propyl, butyl, tert-butyl, iso-propyl, phenyl, fluoro, chloro, trifluoromethyl, methoxy, ethoxy, dimethylamino, diethylamino, trimethylsilyl.

**13.** Organometallic compounds according to claim 12, which are represented by the Formulas **24a, 24b, 24c, 24d, 24e, 34a**

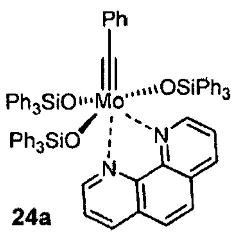

24a

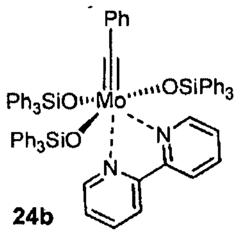

24b

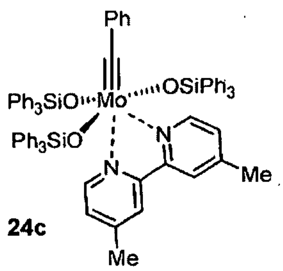

24c

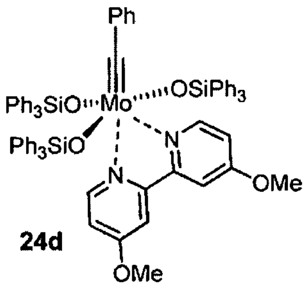

24d

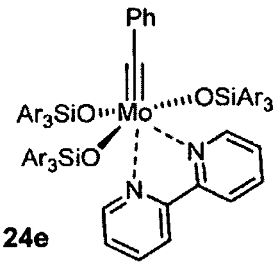

24e

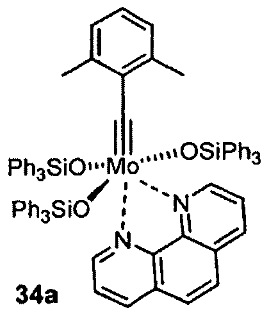

34a

Ar = 4-methoxyphenyl

**14.** Alkylidine complexes of the general Formula 25

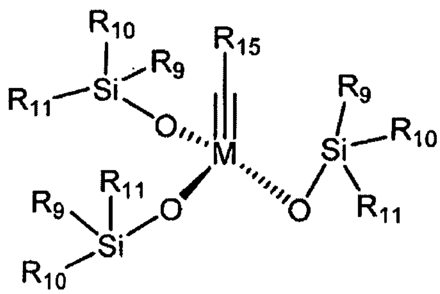

25

wherein

M = Mo, W,
the substituents $R^9$, $R^{10}$, $R^{11}$ may be the same or may be different from one another and have the meaning as defined in claim 1, and
$R^{15}$ may be $C_1$-$C_{12}$ alkyl, $C_3$-$C_{12}$ cycloalkyl, 6- to 18-membered aryl, 5- to 10-membered heteroaryl.

**15.** Adducts of alkylidine complexes of the general Formula **25** according to claim 14 with solvents, which may be selected from the list: acetonitrile, benzonitrile, pivalonitrile, diethylether, di-isopropylether, tert-butylmethylether diphenylether, methoxybenzene, tetrahydrofurane, dioxane, dimethoxyethane, as well as the use as catalysts for the alkyne metathesis.

**16.** Alkylidine complexes according to claims 14 and 15, **characterized in that** $R^9$, $R^{10}$, and $R^{11}$ have the meaning of a phenyl residue, respectively.

**17.** Alkylidine complexes according to claims 14 to 16, **characterized in that** $R^{15}$ may be methyl, ethyl, propyl, butyl, iso-propyl, tert-butyl, trimethylsilylmethyl, benzyl, (dimethyl)(phenyl)methyl, phenyl, methylphenyl, dimethylphenyl, trimethylphenyl, methoxyphenyl, dimethoxyphenyl, fluorophenyl, pentafluorophenyl, trifluoromethylphenyl, or furyl.

**18.** Use of the compounds of Formula 25 and the adducts with solvents thereof

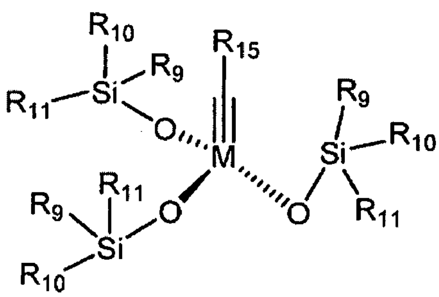

25

wherein

M = Mo, W,
the substituents $R^9$, $R^{10}$, $R^{11}$ may be the same or may be different from one another, and have the meaning as defined in claim 1, and
$R^{15}$ may be $C_1$-$C_{12}$ alkyl, $C_3$-$C_{12}$ cycloalkyl, 6- to 18-membered aryl, 5- to 10-membered heteroaryl, as catalysts for the alkyne metathesis.

**19.** Method of making the compounds of Formula I

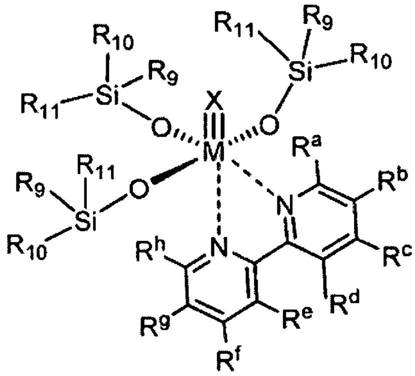

**(I)**

wherein
M, $R^a$, $R^b$, $R^c$, $R^d$, $R^e$, $R^f$, $R^9$, $R^h$, $R^9$, $R^{10}$ $R^{11}$, and X are defined as in claim 1, in which a compound of the general Formula **18**

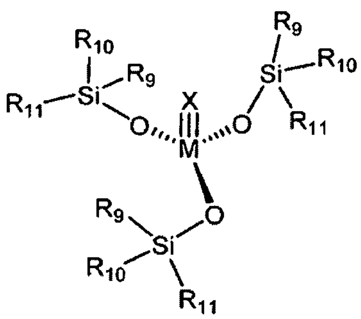

**18**

wherein

M, $R_9$, $R_{10}$, $R_{11}$, and X are defined as in claim 1,
is reacted with a compound of the general Formula **19**

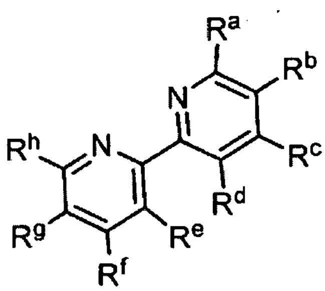

**19**

wherein
$R^a$, $R^b$, $R^c$, $R^d$, $R^e$, $R^f$, $R^g$, and $R^h$ are defined as in claim 1.

**20.** Method according to claim 19, **characterized in that** the compound of Formula 19 is selected from derivatives of 1,10-phenanthroline or 2,2'-bipyridine as defined in claims 4 and 6.

**21.** Use of the compounds according to claims 1 to 13 as pre-catalysts for conducting alkyne metathesis reactions.

**22.** Method of conducting alkyne metathesis reactions, in which a compound of the general Formula I

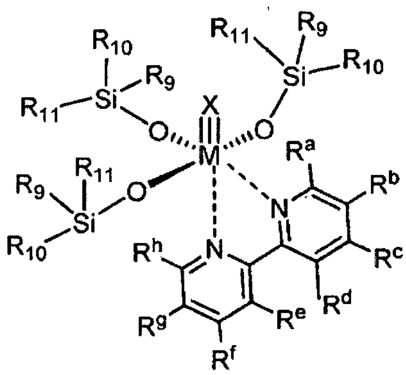

(I)

wherein
M, $R^a$, $R^b$, $R^c$, $R^d$, $R^e$, $R^f$, $R^g$, $R^h$, $R^9$, $R^{10}$, $R^{11}$, and X are defined as in claim 1, is reacted in presence of a metal salt.

**23.** Method according to claim 22, **characterized in that** the metal salt is selected from: $MnY_2$, $FeY_2$, $FeY_3$, $CoY_2$, $CuY_2$, $ZnY_2$, $MgY_2$, $NiY_2$, $PdY_2$, $PtY_2$, $RuY_2$, $RuY_3$, $EuY_3$, wherein Y may be F, Cl, Br, I, acetylacetonate, sulfate, sulfonate, nitrate, acetate, trifluoroacetate.

**24.** Method of conducting alkyne metathesis reactions according to claim 22 in the presence of molecular sieves, **characterized in that** the use of molecular sieves results in the increase of the reaction rate and/or the yield of reaction product compared to the same alkyne metathesis reaction which is conducted in absence of molecular sieves.

**25.** Method according to claims 21 to 23, **characterized in that** the alkyne metathesis reaction is conducted in presence of a molecular sieve.

**26.** Method according to claims 21 to 25, **characterized in that** the alkyne metathesis reaction is conducted in presence of a molecular sieve 4 Angström (MS 4A) or molecular sieve 5 Angström (MS 5A).

## Revendications

**1.** Composés organométalliques de la formule générale **I**,

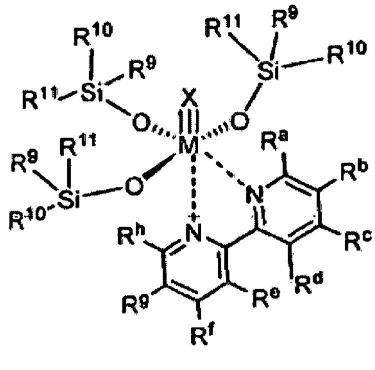

(I)

où

M = Mo, W,
les substituants $R^a$, $R^b$, $R^c$, $R^d$, $R^e$, $R^f$, $R^g$, $R^h$ peuvent être identiques ou différents et peuvent être choisis indépendamment les uns des autres parmi : H, alkyle en $C_1$-$C_{12}$, cycloalkyle en $C_3$-$C_{12}$, aryle à de 6 à 18 éléments, hétéroaryle à de 5 à 10 éléments, alkyloxy en $C_1$-$C_{12}$, cycloalkyloxy en $C_3$-$C_{12}$, aryloxy en $C_6$-$C_{20}$, di-(alkyle en $C_1$-$C_4$)-amino, halogène, nitro, cyano, trifluorométhyle, -$COOR^{12}$, où $R^{12}$ = alkyle en $C_1$-$C_{12}$, cycloalkyle en $C_3$-$C_{12}$, aryle à de 6 à 18 éléments, hétéroaryle à de 5 à 10 éléments,
et les substituants $R^a$ et $R^b$ ou $R^b$ et $R^c$ ou $R^c$ et $R^d$ ainsi que $R^h$ et $R^g$ ou $R^g$ et $R^f$ ou $R^f$ et $R^e$ peuvent également être liés ensemble avec la formation d'un cycle saturé, insaturé ou aromatique,
et/ou les restes $R^d$ et $R^e$ peuvent être liés ensemble avec la formation d'un cycle à de 5 à 8 éléments saturé, insaturé ou aromatique,
les substituants $R^9$, $R^{10}$, $R^{11}$ peuvent être identiques ou différents et peuvent être choisis indépendamment les uns des autres parmi les groupes suivants : alkyle en $C_1$-$C_{12}$, cycloalkyle en $C_3$-$C_{12}$, aryle à de 6 à 18 éléments, hétéroaryle à de 5 à 10 éléments, à condition qu'au moins un de ces substituants soit un reste aryle à de 6 à 18 éléments ou hétéroaryle à de 5 à 10 éléments, où ce reste aryle ou hétéroaryle peut présenter sur l'atome de silicium pour sa part jusqu'à cinq substituants identiques ou différents, parmi la liste : H, alkyle en $C_1$-$C_{12}$, alcoxy en $C_1$-$C_{12}$, di-(alkyle en $C_1$-$C_4$)amino, cycloalkyle en $C_3$-$C_{12}$, cycloalkyloxy en $C_3$-$C_{12}$, aryle à de 6 à 18 éléments, hétéroaryle à de 5 à 10 éléments, halogène, et X = N, $CR^{13}$, où $R^{13}$ = alkyle en $C_1$-$C_{12}$, cycloalkyle en $C_3$-$C_{12}$, aryle à de 6 à 18 éléments, hétéroaryle à de 5 à 10 éléments.

**2.** Composés organométalliques selon la revendication 1, **caractérisés en ce que** les restes $R^d$ et $R^e$ sont liés ensemble avec la formation d'un cycle à de 5 à 8 éléments saturé, insaturé ou aromatique, et forment ainsi un pont, lesquel peut être $CR^{41}R^{42}$, $CR^{43}=CR^{44}$, $CR^{45}R^{46}$-$CR^{47}R^{48}$, $CR^{49}R^{50}$-$CR^{51}R^{52}$-$CR^{53}R^{54}$, $CR^{55}R^{56}=C^{57}R^{58}$-$CR^{59}R^{60}$, $CR^{61}R^{62}$-$CR^{63}R^{65}$-$CR^{65}R^{66}$-$CR^{67}CR^{68}$, $CR^{69}$-$CR^{70}$-$CR^{71}R^{72}$-$CR^{73}CR^{74}$ ou $CR^{75}R^{76}$-$CR^{77}=CR^{78}$-$CR^{79}CR^{80}$ dans lesquels $R^{41}$, $R^{42}$, $R^{43}$, $R^{44}$, $R^{45}$, $R^{46}$, $R^{47}$, $R^{48}$, $R^{49}$, $R^{50}$, $R^{51}$, $R^{52}$, $R^{53}$, $R^{54}$, $R^{55}$, $R^{56}$, $R^{57}$, $R^{58}$, $R^{59}$, $R^{60}$, $R^{61}$, $R^{62}$, $R^{63}$, $R^{64}$, $R^{65}$, $R^{66}$, $R^{67}$, $R^{68}$, $R^{69}$, $R^{70}$, $R^{71}$, $R^{72}$, $R^{73}$, $R^{74}$, $R^{75}$, $R^{76}$, $R^{77}$, $R^{78}$, $R^{79}$, et $R^{80}$, peuvent être choisis à chaque fois indépendamment les uns des autres et peuvent avoir la même signification que $R^a$ dans la revendication 1.

**3.** Composés organométalliques selon la revendication 1 ou 2, lesquels sont représentés par la formule 11 générale

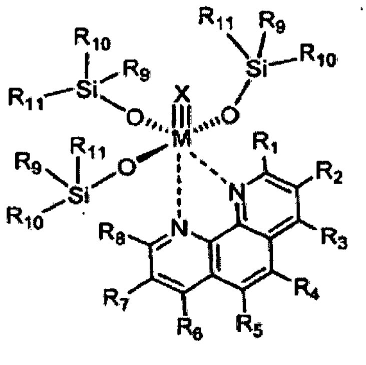

**11**

où
les substituants $R^1$, $R^2$, $R^3$, $R^4$, $R^5$, $R^6$, $R^7$, $R^8$ peuvent être identiques ou différents et peuvent être choisis indépendamment les uns des autres parmi : H, alkyle en $C_1$-$C_{12}$, cycloalkyle en $C_3$-$C_{12}$, aryle à de 6 à 18 éléments, hétéroaryle à de 5 à 10 éléments, alkyloxy en $C_1$-$C_{12}$, cycloalkyloxy en $C_3$-$C_{12}$, aryloxy en $C_6$-$C_{20}$, di-(alkyle en $C_1$-$C_4$)amino, halogène, un groupe nitro, et M, X, $R^9$, $R^{10}$ et $R^{11}$ sont définis comme dans la revendication 1.

**4.** Composés organométalliques selon la revendication 2 ou 3, **caractérisés en ce que** le ligand phénanthroline dans la formule **11** est choisi parmi : 1,10-phénanthroline, 4-méthyl-1,10-phénanthroline, 5-méthyl-1,10-phénantroline, 2,9-diméthyl[1,10]phénanthroline, 5,6-diméthyl-1,10-phénanthroline, 5-chloro[1,10]phénanthroline, 4,7-dichloro-1,10-phénanthroline, 4,7-dichloro-1,10-phénanthroline, 3,4,7,8-tétraméthyl-1,10-phénanthroline, 4,7-diphényl[1,10]-phénanthroline, 2,9-diméthyl-4,7-diphényl[1,10]phénanthroline, 5-nitro-1,10-phénanthroline, 4,7-diméthoxy-1,10-phénanthroline.

**5.** Composés organométalliques selon la revendication 1, lesquels sont représentés par la formule générale **11a**

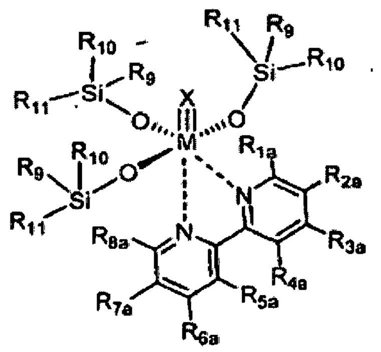

**11a**

où

$R^{1a}$, $R^{2a}$, $R^{3a}$, $R^{4a}$, $R^{5a}$, $R^{6a}$, $R^{7a}$, $R^{8a}$ peuvent être identiques ou différents et peuvent être choisis indépendamment les uns des autres parmi : H, alkyle en $C_1$-$C_{12}$, cycloalkyle en $C_3$-$C_{12}$, aryle à de 6 à 18 éléments, hétéroaryle à de 5 à 10 éléments, alkyloxy en $C_1$-$C_{12}$, cycloalkyloxy en $C_3$-$C_{12}$, aryloxy en $C_6$-$C_{20}$, di-(alkyle en $C_1$-$C_4$)-amino, halogène, nitro, et M, X, $R^9$, $R^{10}$ et $R^{11}$ sont définis comme dans la revendication 1.

6. Composés organométalliques selon la revendication 1 ou 5, **caractérisés en ce que** le ligand bipyridiyle est choisi parmi : 2,2'-bipyridine, 5,5'-diméthyl-2,2'-dipyridyle, 4,4'-diméthyl-2,2'-dipyridyle, 6,6'-diméthyl-2,2'-dipyridiyle, 4,4'-diméthoxy-2,2'-bipyridine, 2,2'-bichinoline, 4,4'-di-tert-butyl-2,2'-dipyridyle, 2,2'-bipyridinyl-4,4'-dicarboxylate de diméthyle, 4,4'-diphényl-2,2'-dipyridyle, 6,6'-dibromo-2,2'-dipyridyle, 4,4'-dinonyl-2,2-dipyridyle, 2,2'-bichinolinyl-4,4'-dicarboxylate de dibutyle, 2,2'-bichinolinyl-4,4'-dicarboxylate de diheptyle, 6-méthyl-2,2'-dipyridyle, 2-(2-pyridinyl)chinoline, 2-pyridin-2-yl-4-pyrrolin-1-yl-chinoline, 4-pipéridin-1-yl-2-pyridin-2-yl-chinoline, 4-morpholin-4-yl-2-pyridin-2-yl-chinoline.

7. Composés organométalliques selon l'une quelconque des revendications 1 à 6, **caractérisés en ce que** $R^9$, $R^{10}$ et $R^{11}$ représentent à chaque fois un reste phényle.

8. Composés organométalliques selon les revendications 1, 2, 3, 4 ou 7, lesquels sont représentés par la formule générale **12**

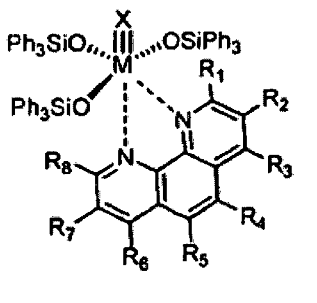

**12**

où

M = Mo, W,
X = N, $CR^{13}$, où
$R^{13}$ peut être un groupe méthyle, éthyle, propyle, butyle, iso-propyle, tert-butyle, triméthylsilylméthyle, benzyle, furyle, phényle, le groupe phényle pouvant porter pour sa part jusqu'à cinq restes identiques ou différents de la liste : H, méthyle, éthyle, propyle, butyle, tert-butyle, iso-propyle, phényle, fluor, chlore, trifluorométhyle, méthoxy, éthoxy, diméthylamino, diéthylamino, triméthylsilyle,
$R^1$, $R^2$, $R^3$, $R^4$, $R^5$, $R^6$, $R^7$, $R^8$ pouvant être choisis librement et indépendamment les uns des autres parmi : H, alkyle en $C_1$-$C_4$, cycloalkyle en $C_3$-$C_6$, phényle, naphtyle, méthylphényle, diméthylphényle, triméthylphényle, méthoxyphényle, diméthoxyphényle, fluorophényle, pentafluorophényle, trifluorométhylphényle, diméthylaminophényle, furyle, halogène, nitro, cyano, trifluorométhyle, $-COOR^{12}$, avec $R^{12}$ = alkyle en $C_1$-$C_{12}$, cycloalkyle en $C_3$-$C_{12}$, aryle à de 6 à 18 éléments, hétéroaryle à de 5 à 10 éléments.

9. Composés organométalliques selon les revendications 1, 5, 6, ou 7, lesquels sont représentés par la formule

générale **12a**

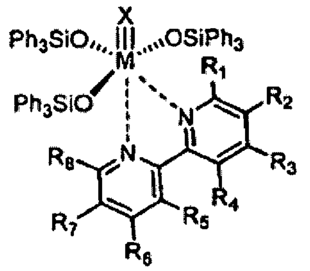

**12a**

où

M = Mo, W
X = N, $CR^{13}$, où
$R^{13}$ peut être un groupe méthyle, éthyle propyle, butyle, iso-propyle, tert-butyle, triméthylsilylméthyle, benzyle, furyle, phényle, le groupe phényle pouvant porter pour sa part jusqu'à cinq restes identiques ou différents de la liste : H, méthyle, éthyle, propyle, butyle, tert-butyle, iso-propyle, phényle, fluor, chlore, trifluorométhyle, méthoxy, éthoxy, diméthylamino, diéthylamino, triméthylsilyle,
$R_1$, $R_2$, $R_3$, $R_4$, $R_5$, $R_6$, $R_7$, $R_8$ peuvent être choisis librement et indépendamment les uns des autres parmi : H, alkyle en $C_1$-$C_4$, cycloalkyle en $C_3$-$C_6$, phényle, naphtyle, méthylphényle, diméthylphényle, triméthylphényle, méthoxyphényle, diméthoxyphényle, fluorophényle, pentafluorophényle, trifluorométhylphényle, diméthylaminophényle, furyle, halogène, nitro, $-COOR^{12}$, avec $R^{12}$ = alkyle en $C_1$-$C_{12}$, cycloalkyle en $C_3$-$C_{12}$, aryle à de 6 à 18 éléments, hétéroaryle à de 5 à 10 éléments.

**10.** Composés organométalliques selon les revendications 1 à 9, lesquels sont représentés par les formules **13a, 13b, 13c, 13d, 13e**

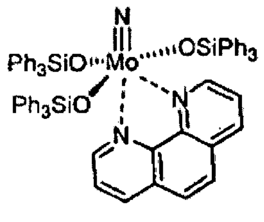

**13a**

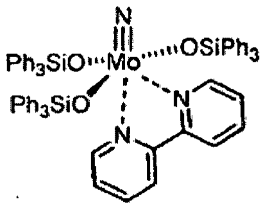

**13b**

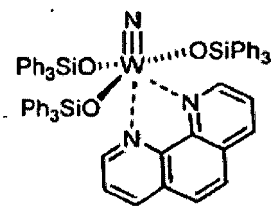

**13c**

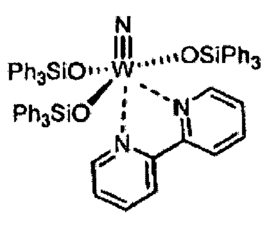

**13d**

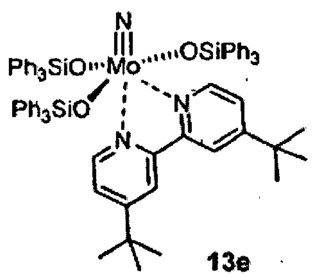

**13e**

**11.** Composés organométalliques selon les revendications 1 à 9, lesquels sont représentés par les formules **14a, 14b, 15a, 15b, 16a, 16b**

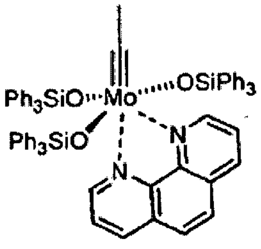
14a
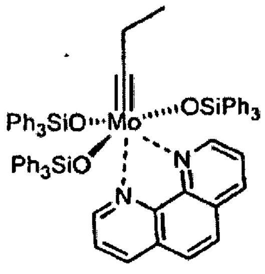
15a
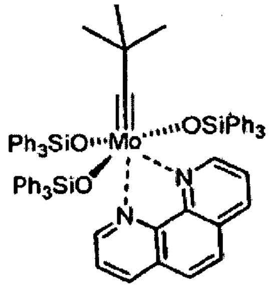
16a

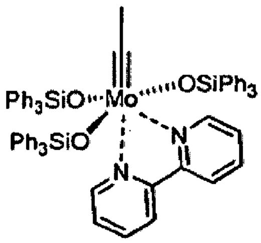
14b
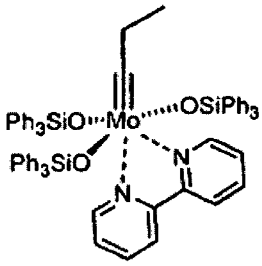
15b
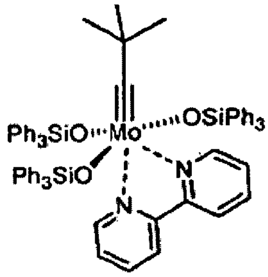
16b

**12.** Composés organométalliques selon les revendications 1 à 9, lesquels sont représentés par les formules **17a** ou **17b**

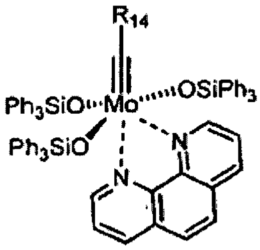
**17a**
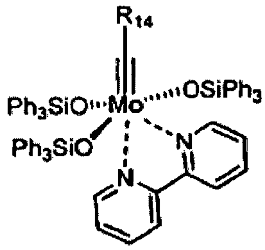
**17b**

où
$R^{14}$ est un cycle phényle, lequel porte de 1 à 5 substituants identiques ou différents choisis dans la liste : H, méthyle, éthyle, propyle, butyle, tert-butyle, iso-propyle, phényle, fluor, chlore, trifluorométhyle, méthoxy, éthoxy, diméthylamino, diéthylamino, triméthylsilyle.

**13.** Composés organométalliques selon la revendication 12, lesquels sont représentés par les formules **24a, 24b, 24c, 24d, 24e, 34a**

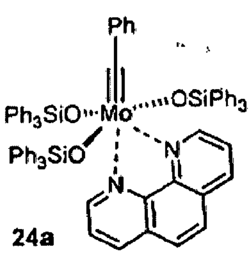
24a
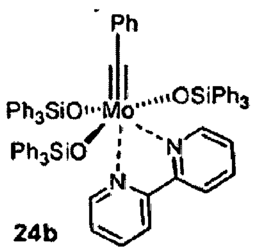
24b
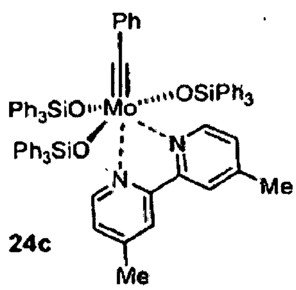
24c

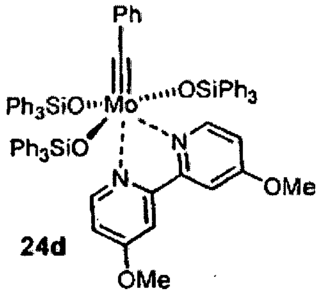

24d

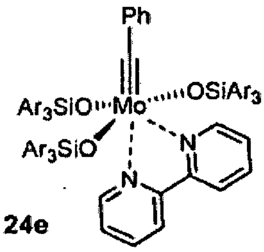

24e

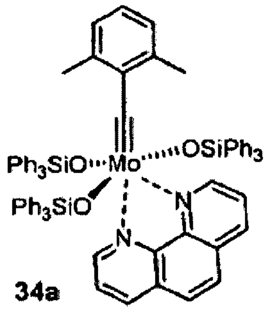

34a

Ar = 4-méthoxyphényle

**14.** Complexes d'alkylidynes de la formule générale **25**

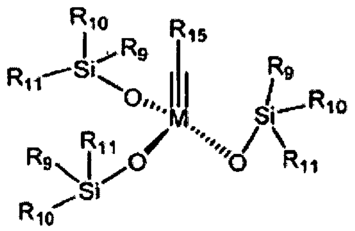

25

où

M = Mo, W,

les substituants $R^9$, $R^{10}$, $R^{11}$ peuvent être identiques ou différents et ont la signification indiquée dans la revendication 1, et

$R^{15}$ est un groupe alkyle en $C_1$-$C_{12}$, cycloalkyle en $C_3$-$C_{12}$, aryle à de 6 à 18 éléments, hétéroaryle à de 5 à 10 éléments.

**15.** Produit d'addition de complexes d'alkylidynes de la formule générale **25** selon la revendication 14 avec des solvants, lesquels peuvent être choisis dans la liste : acétonitrile, benzonitrile, pivalonitrile, diéthyléther, di-isopropyléther, tert-butylméthyléther, diphényléther, méthoxybenzène, tétrahydrofurane, dioxane, diméthoxyéthane, ainsi que leur utilisation comme catalyseurs pour la métathèse d'alcynes.

**16.** Complexes d'alkylidynes selon les revendications 14 et 15, **caractérisés en ce que** $R^9$, $R^{10}$ et $R^{11}$ représentent à chaque fois un reste phényle.

**17.** Complexes d'alkylidynes selon les revendications 14 à 16, **caractérisés en ce que** $R^{15}$ représente un groupe méthyle, éthyle, propyle, butyle, iso-propyle, tert-butyle, triméthylsilylméthyle, benzyle, (diméthyl)(phényle)méthyle, phényle, méthylphényle, diméthylphényle, triméthylphényle, méthoxyphényle, diméthoxyphényle, fluorophényle, pentafluorophényle, trifluorométhylphényle ou furyle.

**18.** Utilisation des composés avec la formule **25** et de leurs produits d'addition avec un solvant

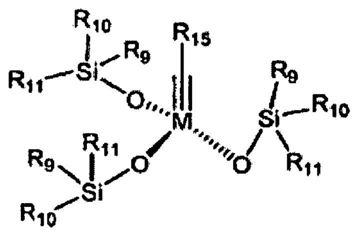

25

où

M = Mo, W,
les substituants $R^9$, $R^{10}$, $R^{11}$ peuvent être identiques ou différents et ont la signification indiquée dans la revendication 1, et
$R^{15}$ = alkyle en $C_1$-$C_{12}$, cycloalkyle en $C_3$-$C_{12}$, aryle à de 6 à 18 éléments, hétéroaryle à de 5 à 10 éléments, comme catalyseurs pour la métathèse d'alcynes.

**19.** Procédé pour la préparation des composés avec la formule I

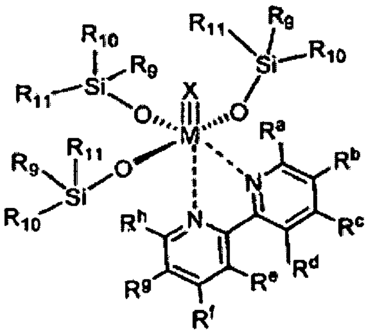

(I)

où

M, $R^a$, $R^b$, $R^c$, $R^d$, $R^e$, $R^f$, $R^g$, $R^h$, $R^9$, $R^{10}$, $R^{11}$ et X sont définis comme dans la revendication 1, dans lequel un composé de la formule générale **18**

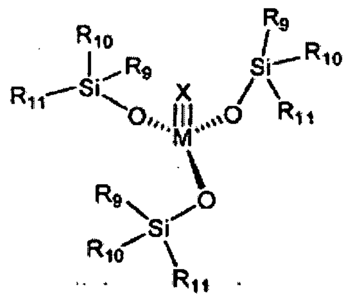

**18**

où
M, $R_9$, $R_{10}$, $R_{11}$ et X sont définis comme dans la revendication 1, réagit avec un composé de la formule générale **19**

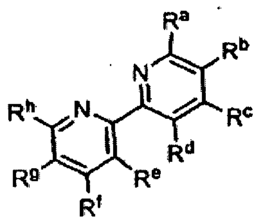

**19**

où
$R^a$, $R^b$, $R^c$, $R^d$, $R^e$, $R^f$, $R^g$ et $R^h$ sont définis comme dans la revendication 1.

**20.** Procédé selon la revendication 19, **caractérisé en ce que** le composé de la formule **19** est choisi parmi des dérivés de la 1,10-phénanthroline ou de la 2,2'-bipyridine comme ils sont définis dans les revendications 4 et 6.

**21.** Utilisation des composés selon les revendications 1 à 13, comme pré-catalyseurs pour la réalisation de réactions

de métathèse d'alcynes.

**22.** Procédé pour la réalisation de réactions de métathèse d'alcynes, dans lequel un composé de la formule générale **I**

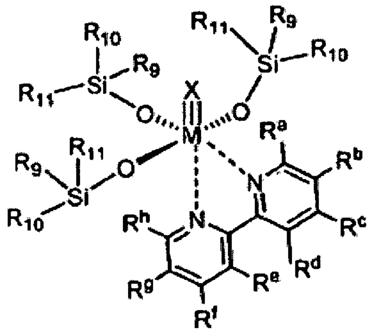

**(I)**

où
M, $R^a$, $R^b$, $R^c$, $R^d$, $R^e$, $R^f$, $R^g$, $R^h$, $R^9$, $R^{10}$ $R^{11}$ et X sont comme définis dans la revendication 1, réagit en présence d'un sel métallique.

**23.** Procédé selon la revendication 22, **caractérisé en ce que** le sel métallique est choisi parmi : $MnY_2$, $FeY_2$, $FeY_3$, $CoY_2$, $CuY_2$, $ZnY_2$, $MgY_2$, $NiY_2$, $PdY_2$, $PtY_2$, $RuY_2$, $RuY_3$, $EuY_3$,
où
Y = F, Cl, Br, I, acétylacétonate, sulfate, sulfonate, nitrate, acétate, trifluoroacétate.

**24.** Procédé pour la réalisation de réactions de métathèse d'alcynes selon la revendication 22 en présence de tamis moléculaires, **caractérisé en ce que** l'utilisation de tamis moléculaires mène à une élévation de la vitesse de réaction et/ou du rendement en produit de réaction en comparaison avec la même réaction de métathèse d'alcynes qui est réalisée en l'absence de tamis moléculaires.

**25.** Procédé selon les revendications 21 à 23, **caractérisé en ce que** la réaction de métathèse d'alcynes est réalisée en présence d'un tamis moléculaire.

**26.** Procédé selon les revendications 21 à 25, **caractérisé en ce que** la réaction de métathèse d'alcynes est réalisée en présence de tamis moléculaires 4 Angströms (MS 4Å) ou de tamis moléculaire 5 Angströms (MS 5Å).

Figur 1

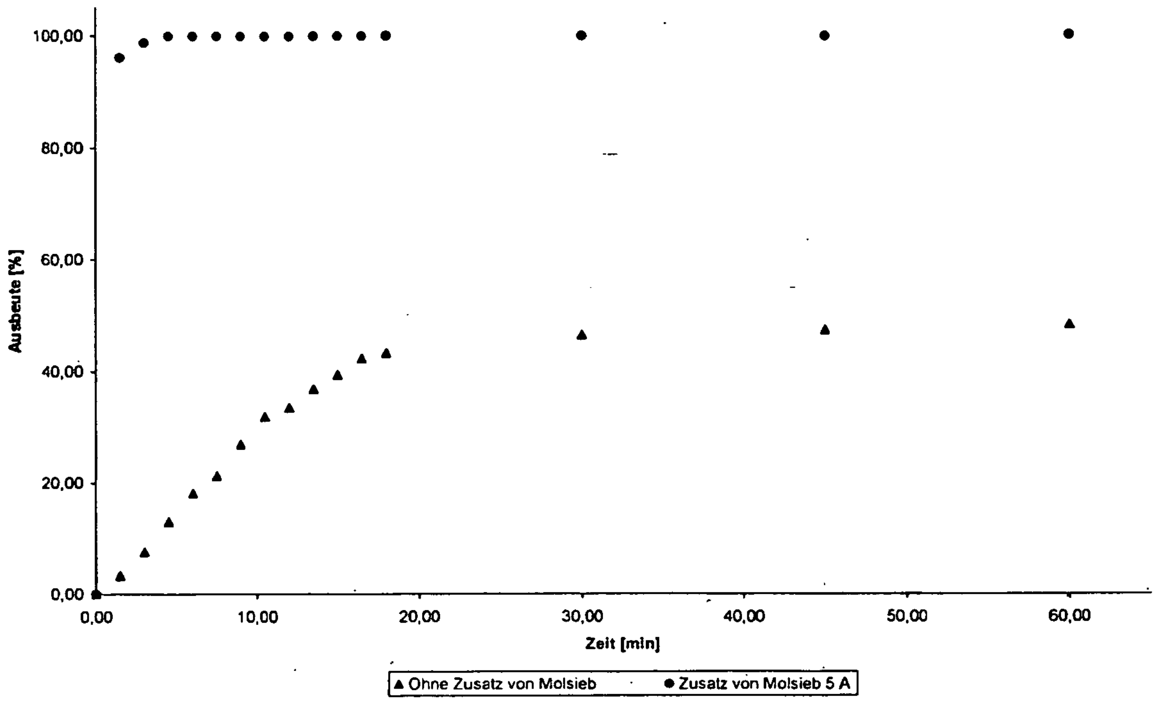
100,00
80,00
60,00
40,00
20,00
0,00
Ausbeute [%]
0,00
10,00
20,00
30,00
40,00
50,00
60,00
Zeit [min]
▲ Ohne Zusatz von Molsieb
● Zusatz von Molsieb 5 A

Figur 2

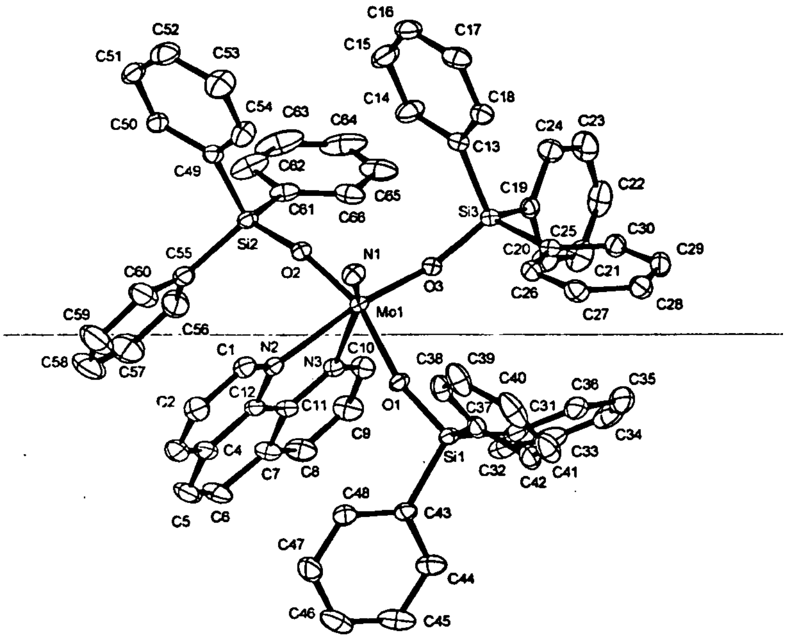

Struktur von $Mo(\equiv N)(OSiPh_3)_3(phen)$ im Festkörper.

Figur 3

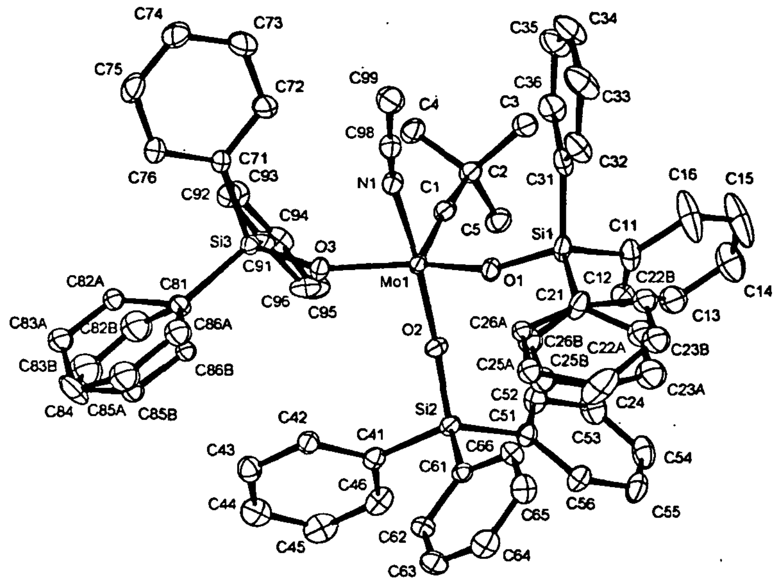

Struktur von Mo(≡CtBu)(OSiPh$_3$)$_3$(MeCN) im Festkörper.

Figur 4

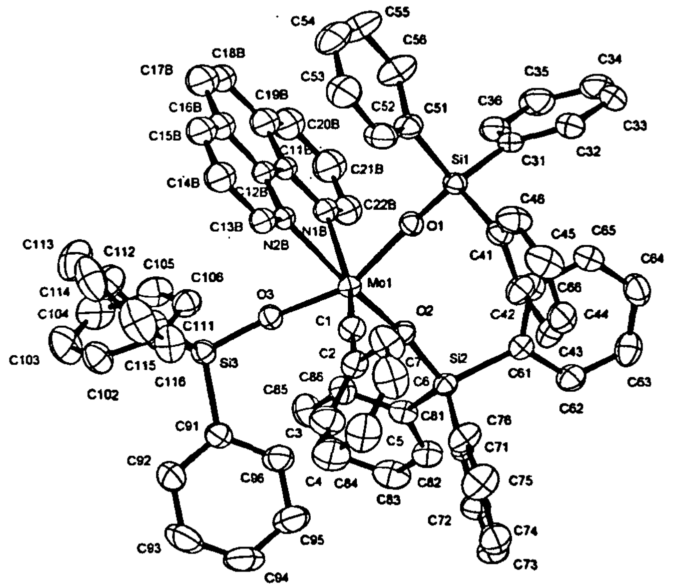

Struktur von Mo(≡CPh)(OSiPh$_3$)$_3$(phen) im Festkörper.

Figur 5

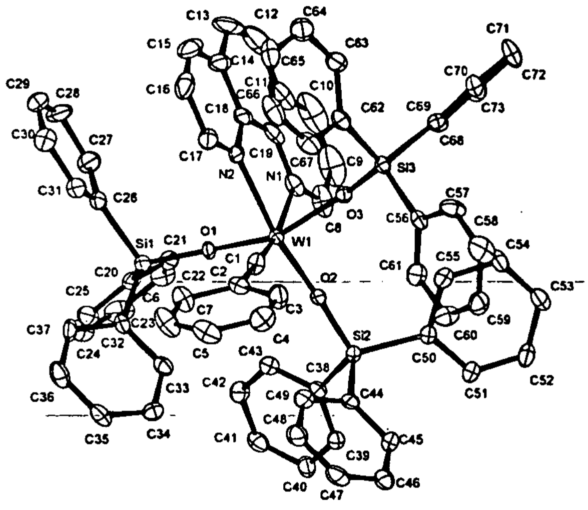

Struktur von [W(≡CAr)(OSiPh$_3$)$_3$(phen)] im Festkörper.

Figur 6

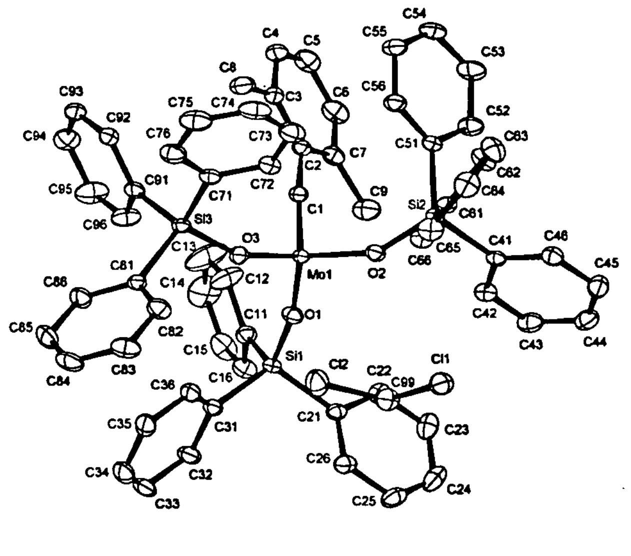

Struktur von Mo(≡CAr)(OSiPh$_3$)$_3$ mit Ar = 2,6-Dimethylphenyl im Festkörper.

Figur 7

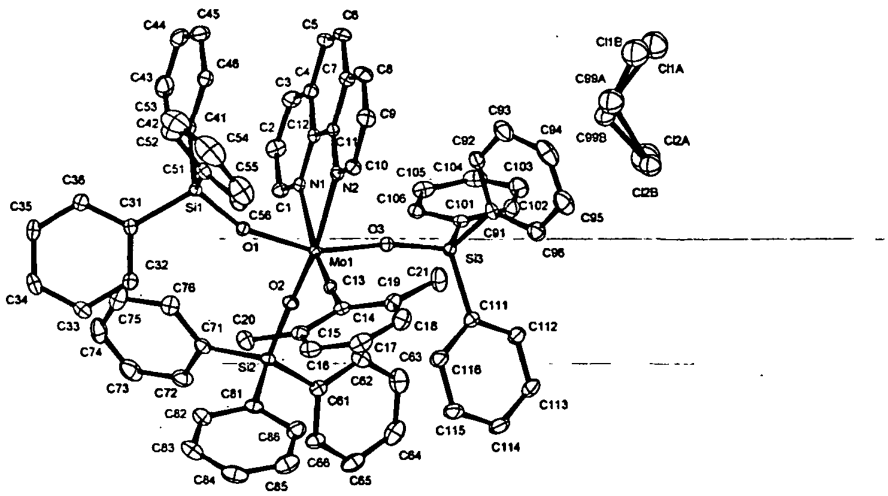

Struktur von [Mo(≡CAr)(OSiPh$_3$)$_3$·(phen)] mit Ar = 2,6-Dimethylphenyl im Festkörper. Der Kristall enthält co-kristallisiertes $CH_2Cl_2$.

Figur 8

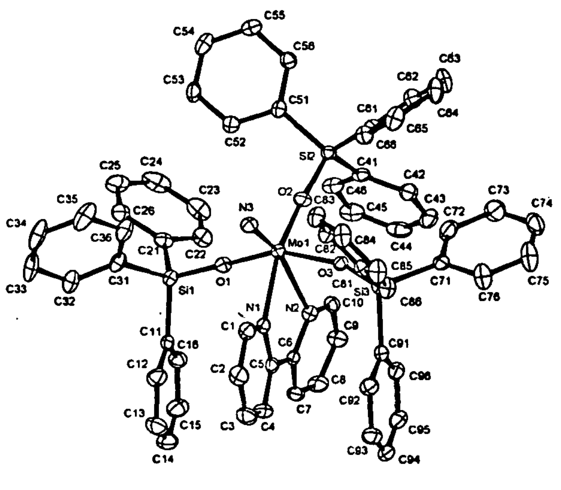

Struktur von [Mo(≡N)(OSiPh$_3$)$_3$(bipy)] im Festkörper.

Figur 9

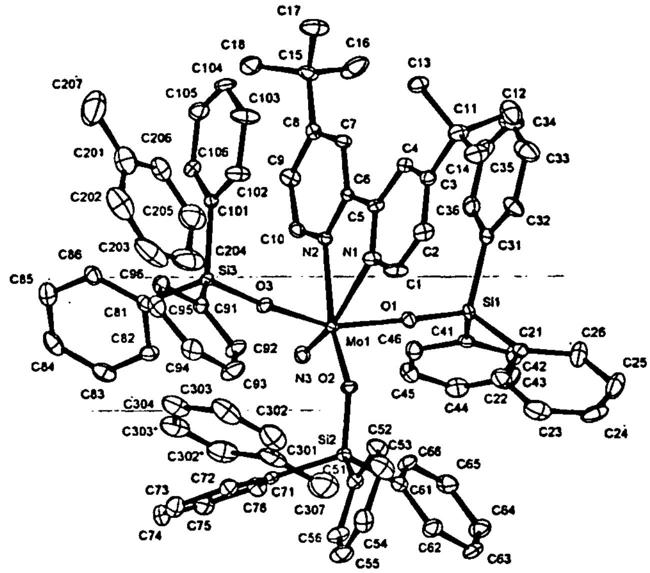

Struktur von [Mo(≡N)($OSiPh_3$)$_3$ (4,4'-di-tert-butyl-2,2'-dipyridyl)] (**24**) im Festkörper.

Figur 10

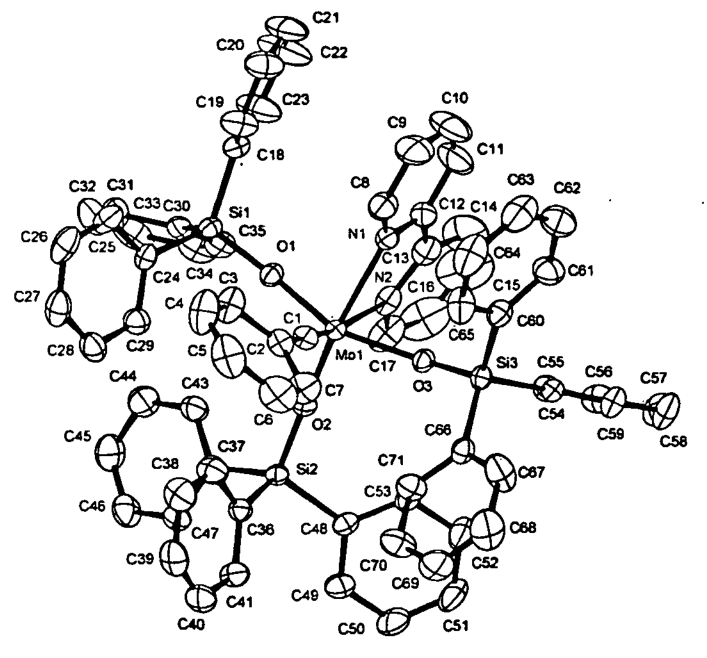

Struktur von [Mo(≡CPh)($OSiPh_3$)$_3$(bipy)] im Festkörper.

Figur 11

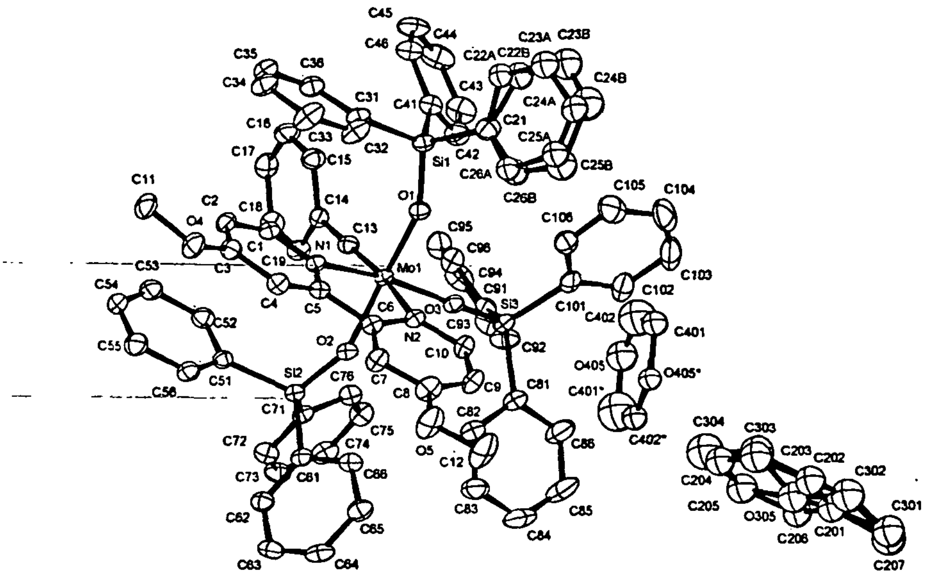

Struktur von [Mo(≡CPh)(OSiPh$_3$)$_3$(4,4'-dimethoxy-2,2'-dipyridyl)] im Festkörper.

Figur 12

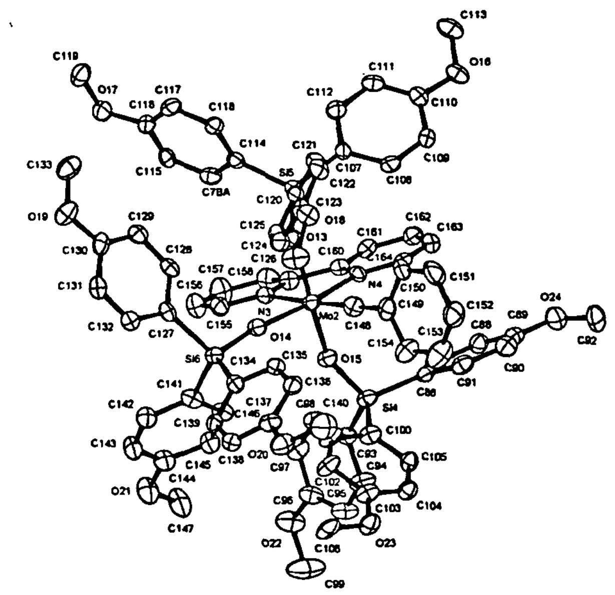

Struktur von [Mo(≡CPh)(OSiAr$_3$)$_3$·(bipy)] mit Ar = 4-Methoxyphenyl im Festkörper.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- **FÜRSTNER, A. ; DAVIES, P. W.** *Chem. Commun.,* 2005, 2307
- **ZHANG, W. ; MOORE, J. S.** *Adv. Synth. Catal.,* 2007, vol. 349, 93
- **SCHROCK, R. R. ; CZEKELIUS, C.** *Adv. Synth. Catal.,* 2007, vol. 349, 55
- **SCHROCK, R. R.** *Chem. Rev.,* 2002, vol. 102, 145
- **SCHROCK, R. R.** *Angew. Chem., Int. Ed.,* 2006, vol. 45, 3748
- **FISCHER, E. O.** *Adv. Organomet. Chem.,* 1976, vol. 14, 1
- **MAYR, A. et al.** *Organometallics,* 1985, vol. 4, 608
- **HAZRA, D. et al.** *J. Organomet. Chem.,* 2003, vol. 671, 52
- **FÜRSTNER, A. et al.** *J. Am. Chem. Soc.,* 1999, vol. 121, 9453
- **ZHANG, W. et al.** *J. Am. Chem. Soc.,* 2004, vol. 126, 329
- **FÜRSTNER, A. et al.** *Chem. Eur. J.,* 2001, vol. 7, 5299
- **FÜRSTNER, A. et al.** *W. Chem. Commun.,* 2005, 2307
- **MORTREUX, A. et al.** *J. Mol. Catal.,* 2006, vol. 254, 96
- **GEYER, A. M. et al.** *J. Am. Chem. Soc.,* 2008, vol. 130, 8984
- **GDULA, R. L. et al.** *J. Am. Chem. Soc.,* 2006, vol. 128, 9614
- **FÜRSTNER A. et al.** *J. Am. Chem. Soc.,* 2009, vol. 131, 9468
- **HEILMANN, E.** *Dissertation,* 2008
- **BINDL, M.** *Technische Universität Dortmund,* 2009
- **CHIU, H.-T. et al.** *Adv. Mater.,* 1998, vol. 10, 1475
- **CLOSE M. R. et al.** *Inorg. Chem.,* 1994, vol. 33, 4198
- **MAYR, A. et al.** *J. Am. Chem. Soc.,* 1986, vol. 108, 548
- **MCCULLOUGH, L. G. et al.** *J. Am. Chem. Soc.,* 1985, vol. 107, 5987
- **STEVENSON, M. A. et al.** *Organometallics,* 1997, vol. 16, 3572
- **HUC, V. et al.** *New J. Chem.,* 2003, vol. 27, 1412
- **MARAVAL, V. et al.** *Tetrahedron Lett.,* 2006, vol. 47, 2155
- **FÜRSTNER, A. et al.** *Angew Chem.,* 1998, vol. 110, 1758
- **BEER, S. et al.** *Organometallics,* 2009, vol. 28, 1534
- **ZHANG, W.** *Org. Synth.,* 2007, vol. 84, 163
- **ZHANG, W. et al.** *J. Am. Chem. Soc.,* 2004, vol. 126, 12796
- **LISTEMANN, M. L. et al.** *Organometallics,* 1985, vol. 4, 74
- **ZHANG, W.** *Org, Synth.,* 2007, vol. 84, 163
- **PSCHIRER, N. G. ; BUNZ, U. H. F.** *Tetrahedron Lett.,* 1999, vol. 40, 2481
- **AKIYAMA, S. ; TAJIMA, K. ; NAKATSUJI, S. ; NAKASHIMA, K. ; ABIRU, K. ; WATANABE, M.** *Bull. Chem. Soc. Jpn.,* 1995, vol. 68, 2043